# EUROPEAN PATENT APPLICATION

(11) **EP 1 335 024 A1**
(43) Date of publication of application: **13.08.2003**
(21) Application number: 01974890.4
(22) Date of filing: 19.10.2001
(51) Int. Cl.: C12N 15/53, C12N 5/10, C12N 1/15, C12N 1/19, C12N 1/21, C12P 21/02, C12P 21/08, C07K 16/24, A61K 39/395, A61P 9/10, G01N 33/53

(54) **ANTIBODY INHIBITING VPLF ACTIVITY**

(30) Priority: 19.10.2000 JP 2000319985
(71) Applicant: KYOWA HAKKO KOGYO CO., LTD., Chiyoda-ku, Tokyo 100-8185 (JP)
(72) Inventor: SHITARA, Kenya, Machida-shi, Tokyo 194-8533 (JP); FURUYA, Akiko, Machida-shi, Tokyo 194-8533 (JP)
(74) Representative: Holliday, Louise Caroline
(86) International application number: JP0109218
(87) International publication number: WO02033094

(57) **Abstract**

There is provided an antibody which specifically recognizes protein comprising an amino acid sequence represented by SEQ ID NO: 1 and inhibits the growth factor activity of the said protein. In accordance with the present invention, it is now possible to prepare a monoclonal antibody which specifically reacts with human VPLF and inhibits its activity and to utilize as a therapeutic agent or a diagnostic agent for the diseases in which VPLF is involved such as diseases associated with abnormal stimulation of angiogenesis, eye diseases based on abnormal angiogenesis, arthritis based on abnormal angiogenesis, skin diseases associated with abnormal angiogenesis, diseases associated with abnormal stimulation of vascular permeability, diseases associated with abnormal differentiation and proliferation of smooth muscle cells, diseases associated with abnormal differentiation and proliferation of kidney mesangial cells, diseases associated with abnormal differentiation and proliferation of blood stem cells, diseases based on abnormality in osteoblasts, diseases based on abnormality in pancreatic β cells, ischemic diseases and diseases associated with the delay of wound healing.

## Description

### Technical Field

The present invention relates to a novel antibody and also to a diagnostic agent, a pharmaceutical agent and a therapeutic agent using the said antibody.

### Background Art

Vascular endothelial growth factor (hereinafter, referred to as "VEGF") having blood-vessel forming and angiogenic activity and platelet-derived growth factor (hereinafter, referred to as "PDGF") having differentiation and proliferation activity for smooth muscle cells are similar in their structures and, including PlGF, VEGF-B, VEGF-C, VEGF-D and VEGF-E which are the factors related thereto, they are classified under growth factors of a VEGF/PDGF superfamily.

Angiogenesis plays an important role in the formation of circulation system and in the construction of many tissues in embryonic stage of vertebrates and is also closely related to luteinization in sexual cycle, transient proliferation of endometrium, placentation, etc. even in mature individuals (female). Moreover, in view of diseased state, angiogenesis is closely related to growth of a solid tumor, development of metastasis, development and progression of pathologic conditions of diabetic retinopathy and rheumatoid arthritis (J. Folkman, et al.; J. Biol. Chem., 267, 10931, 1992).

Angiogenesis comprises a step where an angiogenic factor is secreted, a step where a protease is secreted as a result of the above step from endothelial cells of already-existing blood vessel in the neighborhood thereof, a step where basement membrane and stroma are destroyed by the said protease, a step where migration and proliferation of vascular endothelial cells start and a step where tubes are formed whereby blood vessels are newly generated (J. Folkman, et al.; J. Biol. Chem., 267, 10931, 1992). With regard to the factors for the induction of angiogenesis, involvement of many factors has been reported and, among them, vascular permeability factor (hereinafter, referred to as "VPF")/VEGF is known as the most important factor in angiogenesis during the above-mentioned developmental stage and also in angiogenesis in a diseased state (M. Shibuya; Advances in Cancer Research, 67, 281, 1995). The VPF/VEGF is a protein having a molecular weight of about 40,000 existing as a homodimer and, although it was reported as VPF in 1983 (D. R. Senger, et al.; Science, 219, 983, 1983) and as VEGF in 1989 (N. Ferrara, et al.; Biochem. Biophys. Res. Commun., 161, 851, 1989) as independent molecules, both were found to be the same substance as a result of cDNA cloning (D. W. Leung, et al.; Science, 246, 1306, 1989; P. J. Keck, et al.; Science, 246, 1309, 1989) (hereinafter, referred to as "VEGF"). With regard to the activity of VEGF, there have been reported up to now, for vascular endothelial cells, growth-promoting activity (ED₅₀ = 2-3 pM) (N. Ferrara, et al.; Biochem. Biophys. Res. Commun., 161, 851, 1989), migration-promoting activity (A. E. Koch, et al.; J. Immunol., 152, 4149, 1994), metalloprotease secretion promoting activity (E. N. Unemori, et al.; J. Cell Physiol., 153, 557, 1992), urokinase and tPA secretion promoting activity (M. S. Pepper, et al.; Biochem. Biophys. Res. Commun., 181, 902, 1991), promotion of expression of transcription factor ETS-1 (C. Iwasaka, et al.; J. Cell. Physiol., 169, 522, 1996), increase in express ion of integrin αvβ3 (D. R. Senger, et al.; Am. J. Pathol., 149, 293, 1996), etc. while, in vivo, there have been reported angiogenesis-stimulating activity (T. Asahara, et al., Circulation, 92 suppl II, 365, 1995), vascular permeability-stimulating activity (D. R. Senger, et al.; Science, 219, 983, 1983), etc. It has been reported that VEGF is a growth factor which is highly specific for vascular endothelial cells (N. Ferrara, et al.; Biochem. Biophys. Res. Commun., 161, 851, 1989).

It has been reported that, as a result of a selective splicing, there are four kinds of protein in human VEGF (VEGF₁₂₁, VEGF₁₆₅, VEGF₁₈₉ andVEGF₂₀₆, comprising 121, 165, 189 and 206 amino acid residues, respectively) (K. A. Houck, et al.; J. Biol. Chew., 267, 26031, 1991). It has been reported that even VEGF₁₂₁ having the shortest length has angiogenesis- and vascular permeability-stimulating activity (S. Kondo, et al.; Biochimica et BiophysicaActa, 1243, 195-202, 1995). It has been reported that, although VEGF partial fragment comprising from the 1st to the 110th N-terminal amino acids obtained by the degradation of VEGF₁₆₅ with plasmin has the similar receptor binding activity as VEGF₁₆₅, its growth-promoting activity for vascular endothelial cells decreases to an extent of 1/100 (B. A. Keyt, et al.; J. Biol. Chem., 271, 7788-7795, 1996). Those results show that the 1st to the 110th amino acids are involved in the receptor binding activity while, for a sufficient activation of endothelial cells, the 111th to the 165th amino acids are further necessary.

With regard to VEGF, there have been reported the presence of eight cysteine residues which are important for the formation of a disulfide bond in a dimer, the formation of a disulfide bond in a protein molecule and expression of activity (J. Biol. Chem., 269, 32879-32885, 1994). Those eight cysteine residues are conserved among the factors belonging to a VEGF/PDGF superfamily (C. Betsholds, et al.; Nature, 320, 695-699, 1986). Preparation of an inhibitor for a VEGF binding by modification of amino acid residues of VEGF was attempted and it has been reported that a heterodimer of a VEGF variant in loop II and a VEGF variant in loop III shows inhibitory activity of the VEGF binding and inhibitory activity of the promotion of vascular epithelial cell proliferation (G. Siemeister, etal. ; Proc. Natl. Acad. Sci. USA, 95, 4625-4629, 1998).

With regard to human VEGF receptors, there have been reported two kinds of them, i.e. the first receptor, Flt-1 (fms-like tyrosine kinase) which belongs to a family of receptor type tyrosine kinases (M. Shibuya, et al.; Oncogene, 5, 519, 1990; C. Vries, et al.; Science, 255, 989, 1992) and the second receptor, KDR (kinase insert domain-containing receptor) (B. I. Terman, et al.; WO Publication No. 92/14728; B. I. Terman, et al.; Biochem. Biophys. Res. Commun., 187, 1579, 1992). A mouse homolog of human VEGF receptor KDR has been named Flk-1 (W. Matthews, et al.; Proc. Natl. Acad. Sci. USA, 88, 9026, 1991; A. Ullich, et al.; WO Publication No. 94/11499; B. Millauer, et al.; Cell, 72, 835, 1993). Flt-1 was originally found as a novel gene with unknown function showing a homology to oncogene fms (M. Shibuya, et al.; Oncogene, 5, 519, 1990) and, as a result of attempt of cloning of VEGF receptor gene by means of an expression cloning using VEGF protein, it has been found to be identical with the obtained gene and Flt-1 has been shown to be a VEGF receptor (C. Vries, et al.; Science, 255, 989, 1992).

Extracellular domains of Fil-1 and KDR/Flk-1 comprise seven immunoglobulin-like domains while intracellular domains are membrane proteins of a molecular weight of 180 to 200 kilodaltons having a tyrosine kinase domain. VEGF specifically binds to Flt-1 and KDR/Flk-1 at the K_{d} values of 20 pmol/l and 75 pmol/l, respectively. It has been also reported that Flt-1 and KDR/Flk-1 are specifically expressed in vascular endothelial cells (T. P. Quinn, et al.; Proc. Natl. Acad. Sci. USA, 90, 7533, 1993; R. L. Kendall, et al.; Proc. Natl. Acad. Sci. USA, 90, 8915, 1993).

VEGF has been reported to be deeply involved in growth of a solid tumor, development of metastasis and development of pathologic conditions of diabetic retinopathy and rheumatoid arthritis. With regard to the solid tumor, the production of VEGF has been reported in many human tumor tissues such as renal cancer (A. Takahashi, et al.; Cancer Research, 54, 4233, 1994), breast cancer (L. F. Brown, et al.; Human Pathology, 26, 86, 1995), brain tumor (R. A. Berkman, et al.; J. Clin. Invest., 91, 153, 1993), gastrointestinal cancer (L. F. Brown, et al.; Cancer Research, 53, 4727, 1993) and ovarian cancer (T. A. Olson, et al.; Cancer Research, 54, 276, 1994). With regard to the breast cancer, relation between VEGF and prognosis of the patient was investigated and it is now clarified that, in a tumor expressing VEGF highly, tumor angiogenesis is vigorous and survival rate is low as compared with the lowly-expressing one (M. Toi, et al.; Jpn. J. Cancer Res., 85, 1045, 1994).

Antibody which recognizes VEGF is considered to be important for diagnosis such as tissue diagnosis since it can be used for analysis of VEGF expressed in tumor tissues by immunohistological staining (T. Shibuya, et al.; Clinical Cancer Research, 4, 1483-1487, 1998; Y. Kitadai, et al.; Clinical Cancer Research, 4, 2195-2200, 1998), measurement of VEGF in tumor tissues or serum by ELISA which is an immunoassay (G. Gasparini, et al.; Journal of the National Cancer Institute, 89, 139-147, 1997; S. Kondo, et al.; Biochimica et Biophysica Acta, 1221, 211-214, 1994), etc.

In an experiment with the xenograft model where a human tumor is subcutaneously transplanted to a nude mouse, it has been reported that anti-VEGF monoclonal antibody shows an inhibitory effect for tumor growth(J. K. Kim, et al.; Nature, 362, 841, 1993). It has been also reported that, in metastatic cancer model of a human tumor in a nude mouse, anti-VEGFmonoclonal antibody is able to inhibit the metastasis of cancer (O. Melnyk, et al.; Cancer Research, 56, 921, 1996).

It has been further reported that growth of tumor in nude mouse transplanted tumor model can be inhibited by antisense DNA of DNA encoding VEGF (M. Saleh, et al.; Cancer Research, 56, 393-401, 1996). Accordingly, it is expected that tumor growth or metastasis development in patients suffering from cancer is able to be inhibited if VEGF activity can be inhibited.

Since high concentration of VEGF is detected from human cancerous pleural effusion and ascites, there is a possibility that VEGF is an important factor for accumulation of pleural effusion and ascites (S. Kondo, et al.; Biochimica et Biophysica Acta, 1221, 211, 1994). In mouse models, it has been shown that accumulation of cancerous ascites can be prevented by blocking of VEGF using an anti-VEGF antibody (J. C. Luo, et al.; Cancer Research, 58, 2594-2600, 1998).

In diabetic retinopathy, abnormal angiogenesis causes retinal detachment or vitreous hemorrhage resulting in blindness and it has been reported that there is a positive correlation between angiogenesis in diabetic retinopathy and VEGF level in eye balls of the patients (L. P. Aiello, et al.; N. Engl. J. Med., 331, 1480, 1994). It has been also reported that, in a retinopathy model of monkey, angiogenesis is inhibited when VEGF activity is inhibited by intraocular administration of anti-VEGF neutralizing monoclonal antibody A4.6.1 (A. P. Adamis, et al.; Arch. Ophthalmol., 114, 66, 1996). Accordingly, it is expected that angiogenesis in diabetic retinopathy can be inhibited when an activity of excessively produced VEGF is inhibited.

Progress of pathologic conditions of rheumatoid arthritis rheumatoid arthritis(destruction of bone and cartilage) is accompanied with angiogenesis and it has been reported that VEGF is contained in high concentrations in synovial fluid of patients suffering from rheumatoid arthritisrheumatoid arthritis and that macrophages in joints produce VEGF (A. E. Koch, et al.; J. Immunol., 152, 4149, 1994; R. A. Fava, et al.; J. Exp. Med., 180, 341, 1994). It is expected that angiogenesis in arthritis can be inhibited when the activity of VEGF produced excessively is inhibited.

An increase of the VEGF level in eyes of the patients has been also reported in retinopathy of prematurity (K. Lashkari, et al.; Am. J. Pathol., 156, 1337-1344), age-related macular degeneration (Nobuya Asayama, et al.; Nippon Ganka Gakkaishi, 104, 390-395, 2000) and neovascular glaucoma (R. C. Tripathi, et al.; Ophthalmology, 105, 232-237, 1998) which are eye diseases accompanied with abnormal angiogenesis as the same as in the case of diabetic retinopathy and it is expected that angiogenesis can be inhibited when VEGF activity is inhibited by an antibody.

It has been also reported that VEGF is involved in onset and progress of Crow-Fukase syndrome (O. Watanabe, et al. ; Lancet, 347, 702, 1996), ovarian hyper stimulation syndrome (E. R. Levin, J. Clin. Invest., 102, 1978-1985, 1998), skin diseases such as psoriasis (J. Exp. Med., 180, 1141-1146, 1994; J. Immunol., 154, 2801-2807, 1995) and arterosclerosis (Inoue, M., et al, Circulation, 98, 2108-2116, 1998).

In ischemic diseases such as cerebral infarction, acute myocardial infarction and peripheral artery occlusion, there has been attempted an angiogenic therapy to treat the disease in such a manner that development of collateral blood vessels is promoted to release the ischemia. It has been reported that, when VEGF protein is administered to rabbit chronic ischemic hind limb model in femoral artery, collateral blood vessels are developed in the ischemic site and therapeutic effects such as blood pressure rise in hind limb and increase of blood flow are noted (S. Takeshita, J. Clin. Invest., 93, 662-670, 1994). It has been reported that regeneration of collateral blood vessels is confirmed by intraarterial administration of VEGF cDNA using a catheter or intramuscular injection thereof to lower limbs to patients suffering from arterial occlusion in lower limbs (I. Baumgartner, et al., Circulation, 97, 1114, 1998).

It is shown that vascular endothelial cells and blood cells are differentiated separately from hemoangiogenic stem cells which are the common progenitor cells. Since a VEGF receptor KDR/Flk-1 is expressed in hemoangiogenic stem cells, there has been pointed out a possibility that VEGF is an essential factor for differentiation from hemoangiogenic stem cells into vascular epithelial progenitor cells and blood progenitor cells (S. Nishikawa, et al., Development, 125, 1747-1757, 1998). It has been reported that, when hemoangiogenic stem cells are administered to ischemic model animals, the said cells are utilized for angiogenesis of ischemic sites (T. Asahara, et al., Science, 275, 964-967, 1997).

Although VEGF shows a very high specificity to vascular endothelial cells, its action to certain blood cells, osteoblasts and pancreatic β cells has been reported as well. It has been reported that, in human monocytes, a VEGF receptor Flt-1 is expressed and VEGF has a migration-promoting activity on monocytes (B. Barleon, et al., Blood, 87, 3336-3343, 1996). It has been reported that, in human dendritic cells which are important as antigen-presenting cells, a VEGF receptor Flt-1 is expressed and VEGF has an activity of inhibiting the maturation of dendritic cells. Since activation of dendritic cells is an important step in destruction of tumor by immunity, it has been presumed that VEGF produced from tumor cells promotes the tumor growth by suppression of tumor immunity (D. M. Garbilovich, et al., Nature Medicine, 2, 1096-1103, 1996). It has been reported that VEGF acts on osteoblasts and promotes their migration and differentiation (V. Midy, et al., Biochem. Biophys. Res. Commun., 199, 380-386, 1994). It has been also shown the possibility that VEGF receptor KDR/Flk-1 is expressed in pancreatic β cells and VEGF is involved in differentiation of β cells (C. Oberg, et al., Growth Factors, 10, 115-126, 1994).

With regard to an analogous factor for VEGF, there have been isolated up to now PlGF (placental growth factor) (D. Maglione, Proc. Natl. Acad. Sci. USA, 88, 9267-9271, 1991), VEGF-B (B. Olofsson, et al., Proc. Natl. Acad. Sci. USA, 93, 2576-2581, 1996), VEGF-C (J. Lee, et al., Proc. Natl. Acad. Sci. USA, 93, 1988-1992, 1996), VEGF-D (M. G. Achen, Proc. Natl. Acad. Sci. USA, 95, 548-553, 1998), VEGF homologs of the NZ2 strain and the NZ7 strain of orf virus (hereinafter, referred to as NZ2-VEGF and NZ7-VEGF, respectively; D. J. Lyttle, Journal of Virology, 68, 84-92, 1994), PDGF-A (C. Betsholtz, et al., Nature, 320, 695-699, 1986) and PDGF-B (T. Collins, et al., Nature, 316, 748-750, 1985).

PDGF is present in platelets and was purified in 1979 as a factor having a migration- and growth- stimulating activity mainly on mesenchymal cells (Heldin, C. H., et al., Proc. Natl. Acad. Sci. USA, 76, 3722-3726, 1979). PDGF has a structure where two kinds of polypeptide chains (each having a molecular weight of about 30,000) called A chain (hereinafter, referred to as PDGF-A) and B chain (hereinafter, referred to as PDGF-B) are dimerized by a disulfide bond and three kinds of isoforms PDGF-AA, AB and BB have been reported. There have been isolated cDNAs of A chain (Betshotzs, C., et al., Nature, 320, 695-699, 1986) and B chain (Collins, T., et al., Nature, 316, 748-750, 1985) and, their mature proteinshave a 60% amino acid homology and positions of eight cysteine residues necessary for maintenance of structure and activity are conserved in them (Claesson-Welsh, L., J. Biol. Chem., 269, 32023-32026, 1994). During the biosynthesis, the precursor protein of PDGF receives cleavage of the N-terminal moiety of A chain and cleavage of the N-terminal and C-terminal moieties of B chain to give its mature protein (Claesson-Welsh, L., J. Biol. Chem., 269, 32023-32026, 1994). It has been attempted to prepare a PDGF inhibitor by fragmentation of PDGF, modification of amino acid residues, etc. and, with regard to PDGF-B, it has been reported that a peptide comprising 13 residues corresponding to 116 to 121 and 157 to 163 has an activity of inhibiting the binding of PDGF-B to PDGF receptor (Engstrom, U., et al., J. Biol. Chew., 267, 16581-16587, 1992).

It has been considered that PDGF plays an important role for wound healing since it is secreted from a group of cells related to wound healing, i.e. not only from platelets but also from macrophages, smooth muscle cells, endothelial cells, fibroblasts, etc. and that, in addition to cell migration- and growth-stimulating activity , it promotes production of extracellular matrix such as collagen and production of enzymes involved in remodeling (Ross, R., et al., Cell, 46, 155-169, 1985). It has been reported that, in a rabbit model, repair of damage of the skin is promoted by administration of PDGF (Pierce, et al., Journal of Cellular Biochemistry, 45, 319-326, 1991). At present, PDGF is receiving public attention as a therapeutic agent for pathologic conditions caused by the delay of wound healing such as neurogenic ulcer of lower limb, diabetic ulcer of lower limb, etc.

In the initial stage of development of a lesion of arterosclerosis, it has been considered that PDGF secreted from platelets and macrophages which accumulated to the damaged artery intima causes migration of medial smooth muscle cells to the intima and their proliferation in the intima whereupon the lesion is advanced (Ross, R., et al., Science, 248, 1009-1012, 1990). It is further reported that, as a result of administration of PDGF antibody, pathological conditions can be inhibited in a rat model of arterosclerosis(Ferns, G. A., et al., Science, 253, 1129-1132, 1991).

In onset and progress of glomerulonephritis, mesangial cells play a central role. It has been reported that, in human glomerulonephritis, expression of PDGF increases (Matsuda, M., et al., American Journal of Nephrology, 17, 25-31, 1997) and that, when a PDGF neutralizing antibody is administered to a rat model of nephritis, a therapeutic effect is noted (Johnson, R. J., et al., J. Exp. Med., 175, 1413-1416, 1992).

It has been found that PDGF-B chain is a protooncogene of v-sis which is an oncogene of simian sarcoma virus (Waterfield, M. D., et al., Nature, 304, 35-39, 1983) and PDGF-B chain is receiving public attention from the viewpoint of oncogenic study. It has been reported that 168 kinds of cell lines derived from 26 kinds of different human tumors express the PDGF and that there is a possibility that PDGF is a autocrine and paracrine growth factor (Potapova, O., et al., International Journal of Cancer, 66, 669-677, 1996).

As mentioned hereinabove, the growth factor belonging to a VEGF/PDGF superfamily has been shown to be involved in diseases associated with abnormal stimulation of angiogenesis such as solid tumor and tumor metastasis, eye diseases based on abnormal angiogenesis such as diabetic retinopathy, retinopathy of prematurity, age-related macular degeneration and neovascular glaucoma, arthritis based on abnormal angiogenesis such as rheumatoid arthritis, skin diseases associated with abnormal angiogenesis such as psoriasis, diseases associated with abnormal vascular permeability such as ascites cancer, cancer with pleural effusion, Crow-Fukase syndrome and ovarian hyperstimulation syndrome, diseases associated with abnormal differentiation and proliferation of smooth muscle cells such as arterosclerosis, diseases associated with abnormal differentiation and proliferation of kidney mesangial cells such as glomerulonephritis, diseases associated with abnormal differentiation and proliferation of blood stem cells such as anemia, diseases based on abnormality in osteoblasts such as osteoporosis, diseases based on abnormality in pancreatic β-cells such as diabetes mellitus, ischemic diseases such as cerebral infarction, acute myocardial infarction and peripheral artery occlusion and diseases associated with the delay of wound healing such as neurogenic ulcer of lower limb and diabetic ulcer of lower limb. Further, inhibitors such as antisense DNA and antibody being able to inhibit the activity of a growth factor belonging to a VEGF/PDGF superfamily have been shown to have a therapeutic activity for diseases associated with abnormal stimulation of angiogenesis such as solid tumor and tumor metastasis, eye diseases based on abnormal angiogenesis such as diabetic retinopathy, retinopathy of prematurity, age-related macular degeneration and neovascular glaucoma, arthritis based on abnormal angiogenesis such as rheumatoid arthritis, skin diseases associated with abnormal angiogenesis such as psoriasis, diseases associated with abnormal vascular permeability such as ascites cancer, cancer with pleural effusion, Crow-Fukase syndrome and ovarian hyperstimulation syndrome, diseases associated with abnormal differentiation and proliferation of smooth muscle cells such as arterosclerosis and diseases associated with abnormal differentiation and proliferation of kidney mesangial cells such as glomerulonephritis. Still further, growth factors belonging to a VEGF/PDGF superfamily has been shown to be effective in an angiogenic therapy for ischemic diseases such as cerebral infarction, acute myocardial infarction and peripheral artery occlusion and a wound healing acceleration therapy for diseases such as neurogenic ulcer of lower limb and diabetic ulcer of lower limb by administration of the protein or the gene encoding it. Furthermore, a VEGF receptor has been reported to express in blood stem cells, osteocytes and pancreatic β cells and to possibly be involved in proliferation and differentiation of such cells and has been suggested to be able to be a therapeutic agent for diseases associated with abnormal differentiation and proliferation of blood stem cells such as anemia, diseases based on abnormal osteocytes such as osteoporosis and diseases based on abnormal pancreatic β cells such as diabetes mellitus. Accordingly, the factor belonging to a VEGF/PDGF superfamily has been receiving a big attention as a target for developing useful and new medicines. It has been also presumed of a possibility that novel factors belonging to a VEGF/PDGF superfamily are present and, up to now, VEGF-related gene (WO Publication No. 99/37671) and VEGF-E (WO Publication No. 99/47677) have been reported. Such novel factors may be targets for the development of new medicines and, particularly, antibodies which inhibit the activity of the novel factors will be useful as diagnostic agents, therapeutic agents, etc. for the diseases in which the said factors are involved.

### Disclosure of the Invention

An object of the present invention is to provide an antibody which specifically reacts with a VEGF/PDGF-like factor (hereinafter, referred to as "VPLF") and inhibits the activity of VPLF and also to provide a method for treating or diagnosing the diseases in which VPLF is involved, such as diseases associated with abnormal stimulation of angiogenesis, eye diseases based on abnormal angiogenesis, arthritis based on abnormal angiogenesis, skin diseases associated with abnormal angiogenesis, diseases associated with abnormal stimulation of vascular permeability, diseases associated with abnormal differentiation and proliferation of smooth muscle cells, diseases associated with abnormal differentiation and proliferation of kidney mesangial cells, diseases associated with abnormal differentiation and proliferation of blood stem cells, diseases based on abnormality in osteoblasts, diseases based on abnormality in pancreatic β cells, ischemic diseases , diseases associated with the delay of wound healing, etc using the antibody of the present invention.

The present inventors have carried out intensive investigations for solving the above-mentioned problems and succeeded in preparing a monoclonal antibody which specifically reacts with VPLF and inhibits the activity of the VPLF whereupon the present invention has been achieved.

Thus, the present invention includes the followings.
(1) An antibody which specifically recognizes a protein comprising an amino acid sequence represented by SEQ ID NO: 1 and inhibits a growth factor activity of the said protein.
(2) The antibody according to the above (1), wherein it recognizes an epitope existing in the 227th amino acid to the 345th amino acid in SEQ ID NO: 1.
(3) An antibody which specifically recognizes the protein comprising an amino acid sequence where one or more amino acid(s) is/are deleted, substituted or added in the amino acid sequence represented by SEQ ID NO: 1 and having a growth factor activity of the protein comprising the amino acid sequence represented by SEQ ID NO: 1, and which inhibits the growth factor activity of the said protein.
(4) An antibody which specifically recognizes the protein comprising an amino acid sequence having homology of 60% or more to the amino acid sequence represented by SEQ ID NO: 1 and having a growth factor activity of the protein comprising the amino acid sequence represented by SEQ ID NO: 1, and which inhibits the growth factor activity of the said protein.
(5) An antibody which specifically recognizes the protein comprising a partial sequence of the amino acid sequence represented by SEQ ID NO: 1, containing eight cysteine residues conserved among the factors belonging to a VEGF/PDGF superfamily and having a growth factor activity of the protein comprising the amino acid sequence represented by SEQ ID NO: 1, and which inhibits the growth factor activity of the said protein.
(6) An antibody which specifically recognizes the protein comprising an amino acid sequence where one or more amino acid(s) is/are deleted, substituted or added in a partial sequence of the amino acid sequence represented by SEQ ID NO: 1, containing eight cysteine residues conserved among the factors belonging to a VEGF/PDGF superfamily, and having a growth factor activity of the protein comprising the amino acid sequence represented by SEQ ID NO: 1, and which inhibits the growth factor activity of the said protein.
(7) An antibody which specifically recognizes the protein comprising an amino acid sequence where the amino acids from N-terminal to at least the 226th amino acid are deleted in the amino acid sequence represented by SEQ ID NO: 1 and having a growth factor activity of the protein comprising the amino acid sequence represented by SEQ ID NO: 1, and which inhibits the growth factor activity of the said protein.
(8) An antibody which specifically recognizes a protein comprising the amino acid sequence represented by SEQ ID NO: 32 and inhibits the growth factor activity of the said protein.
(9) An antibody which specifically recognizes a protein comprising the amino acid sequence represented by SEQ ID NO: 33 and inhibits the growth factor activity of the said protein.
(10) The antibody according to any one of (1) to (9), wherein the growth factor activity of the protein is a growth-promoting activity for smooth muscle cells.
(11) The antibody according to (10), wherein the smooth muscle cells are derived from rat.
(12) The antibody according to any one of (1) to (11), wherein the antibody is a monoclonal antibody.
(13) The monoclonal antibody according to (12), wherein the monoclonal antibody is a mouse monoclonal antibody.
(14) The antibody according to (12), wherein the monoclonal antibody is an IgG1 subclass.
(15) The antibody according to (13), wherein the mouse monoclonal antibody is an IgG1 subclass.
(16) A monoclonal antibody which is produced by hybridoma cell line KM 2764 (FERM BP-7293).
(17) A monoclonal antibody which is produced by hybridoma cell line KM 2767 (FERM BP-7294).
(18) An antibody fragment comprising a partial fragment of the monoclonal antibody according to (12).
(19) An antibody derivative where an antibody according to any one of (1) to (17) or an antibody fragment according to (18) is bound to a radioactive isotope, protein or low-molecular-weight agent.
(20) A hybridoma which produces the antibody according to any one of (1) to (17).
(21) The hybridoma according to (20), wherein it is a hybridoma cell line KM 2764 (FERM BP-7293).
(22) The hybridoma according to (20), wherein it is a hybridoma cell line KM 2767 (FERM BP-7294).
(23) DNA encoding the antibody according to any one of
(1) to (17), the antibody fragment according to (18) or the antibody derivative according to (19).
(24) A recombinant vector containing the DNA according to (23).
(25) A transformant which is prepared by introduction of the recombinant vector according to (24) into a host cell.
(26) A process for producing antibody, antibody fragment or derivative, which comprises culturing the transformant in a medium, so as to produce and accumulate the antibody according to any one of (1) to (17), the antibody fragment according to (18) or the derivative according to (19) in a culture and recovering the said antibody, antibody fragment or derivative.
(27) A pharmaceutical agent comprising the antibody according to any one of (1) to (17), the antibody fragment according to (18) or the antibody derivative according to (19).
(28) A therapeutic agent comprising the antibody according to any one of (1) to (17), the antibody fragment according to (18) or the antibody derivative according to (19) for at least one disease selected from a group consisting of diseases associated with abnormal stimulation of angiogenesis, eye diseases based on abnormal angiogenesis, arthritis based on abnormal angiogenesis, skin diseases associated with abnormal angiogenesis, diseases associated with abnormal stimulation of vascular permeability, diseases associated with abnormal differentiation and proliferation of smooth muscle cells and diseases associated with abnormal differentiation and proliferation of kidney mesangial cells.
(29) The therapeutic agent according to (28), wherein the disease associated with abnormal stimulation of angiogenesis is selected from solid tumor and tumor metastasis, the eye disease based on abnormal angiogenesis is selected from a group consisting of diabetic retinopathy, retinopathy of prematurity, age-related macular degeneration and neovascular glaucoma, the arthritis based on abnormal angiogenesis is rheumatoid arthritis, the skin disease associated with abnormal angiogenesis is psoriasis, the disease associated with abnormal stimulation of vascular permeability is selected from a group consisting of ascites cancer, cancer with pleural effusion, Crow-Fukase syndrome and ovarian hyperstimulation syndrome, the disease associated with abnormal differentiation and proliferation of smooth muscle cells is arterosclerosis and the disease associated with abnormal differentiation and proliferation of kidney mesangial cells is glomerulonephritis.
(30) A diagnostic agent comprising the antibody according to any one of (1) to (17), the antibody fragment according to (18) or the antibody derivative according to (19) for at least one disease selected from a group consisting of diseases associated with abnormal stimulation of angiogenesis, eye diseases based on abnormal angiogenesis, arthritis based on abnormal angiogenesis, skin diseases associated with abnormal angiogenesis, diseases associated with abnormal stimulation of vascular permeability, diseases associated with abnormal differentiation and proliferation of smooth muscle cells, diseases associated with abnormal differentiation and proliferation of kidney mesangial cells, diseases associated with abnormal differentiation and proliferation of blood stem cells, diseases based on abnormality in osteoblasts, diseases based on abnormality in pancreatic β cells, ischemic diseases and diseases associated with the delay of wound healing.
(31) The diagnostic agent according to (30), wherein the disease associated with abnormal stimulation of angiogenesis is selected from solid tumor and tumormetastasis, the eye disease based on abnormal angiogenesis is selected from a group consisting of diabetic retinopathy, retinopathy of prematurity, age-related macular degeneration and neovascular glaucoma, the arthritis based on abnormal angiogenesis is rheumatoid arthritis, the skin disease associated with abnormal angiogenesis is psoriasis, the disease associated with abnormal stimulation of vascular permeability is selected from a group consisting of ascites cancer, cancer with pleural effusion, Crow-Fukase syndrome and ovarian hyperstimulation syndrome, the disease associated with abnormal differentiation and proliferation of smooth muscle cells is arterosclerosis, the disease associated with abnormal differentiation and proliferation of kidney mesangial cells is glomerulonephritis, the disease associated with abnormal differentiation and proliferation of blood stem cells is anemia, the disease based on abnormality in osteoblasts is osteoporosis, the disease based on abnormality in pancreatic β cells is diabetes mellitus, the ischemic disease is selected from a group consisting of cerebral infarction, acute myocardial infarction and peripheral artery occlusion and the disease associated with the delay of wound healing is selected from a group consisting of neurogenic ulcer of lower limb and diabetic ulcer of lower limb.
(32) A method for an immunological quantitative determination of at least one protein selected from a group consisting of the following (a) to (j) which comprises using the antibody according to any one of (1) to (17), the antibody fragment according to (18) or the antibody derivative according to (19):
   (a) a protein comprising the amino acid sequence represented by SEQ ID NO: 1;
   (b) a protein comprising an amino acid sequence where one or more amino acid(s) is/are deleted, substituted or added in the amino acid sequence represented by SEQ ID NO: 1 and having the growth factor activity of the protein comprising the amino acid sequence represented by SEQ ID NO: 1;
   (c) a protein comprising an amino acid sequence having 60% or more homology to the amino acid sequence represented by SEQ ID NO: 1 and having the growth factor activity of the protein comprising the amino acid sequence represented by SEQ ID NO: 1;
   (d) a protein comprising a partial sequence of the amino acid sequence represented by SEQ ID NO: 1, containing eight cysteine residues conserved among the factors belonging to a VEGF/PDGF superfamily and having the growth factor activity of the protein comprising the amino acid sequence represented by SEQ ID NO: 1;
   (e) a protein comprising a partial sequence of the amino acid sequence where one or more amino acid(s) is/are deleted, substituted or added in the amino acid sequence represented by SEQ ID NO: 1, containing eight cysteine residues conserved among the factors belonging to a VEGF/PDGF superfamily and having the growth factor activity of the protein comprising the amino acid sequence represented by SEQ ID NO: 1;
   (f) a protein comprising an amino acid sequence where amino acids of from N-terminal to at least the 226th amino acid are deleted from the amino acid sequence represented by SEQ ID NO: 1 and having the growth factor activity of the protein comprising the amino acid sequence represented by SEQ ID NO: 1;
   (g) a protein comprising the amino acid sequence represented by SEQ ID NO: 32;
   (h) a protein comprising the amino acid sequence represented by SEQ ID NO: 33;
   (i) a protein according to any one of the above (a) to (h) where the growth factor activity of the protein is a growth-promoting activity for smooth muscle cells; and
   (j) the protein according to the above (i) where the smooth muscle cells are derived from rat.
(33) A method for the immunological detection of at least one protein selected from a group consisting of the following (a) to (j) which comprises using the antibody according to any one of (1) to (17), the antibody fragment according to (18) or the antibody derivative according to (19):
   (a) a protein comprising the amino acid sequence represented by SEQ ID NO: 1;
   (b) a protein comprising an amino acid sequence where one or more amino acid(s) is/are deleted, substituted or added in the amino acid sequence represented by SEQ ID NO: 1 and having the growth factor activity of the protein comprising the amino acid sequence represented by SEQ ID NO: 1;
   (c) a protein comprising an amino acid sequence having 60% or more homology to the amino acid sequence represented by SEQ ID NO: 1 and having the growth factor activity of the protein comprising the amino acid sequence represented by SEQ ID NO: 1;
   (d) a protein comprising a partial sequence of the amino acid sequence represented by SEQ ID NO: 1, containing eight cysteine residues conserved among the factors belonging to a VEGF/PDGF superfamily and having the growth factor activity of the protein comprising the amino acid sequence represented by SEQ ID NO: 1;
   (e) a protein comprising a partial sequence of the amino acid sequence where one or more amino acid(s) is/are deleted, substituted or added in the amino acid sequence represented by SEQ ID NO: 1, containing eight cysteine residues conserved among the factors belonging to a VEGF/PDGF superfamily and having the growth factor activity of the protein comprising the amino acid sequence represented by SEQ ID NO: 1;
   (f) a protein comprising an amino acid sequence where amino acids of from N-terminal to at least the 226th amino acid are deleted from the amino acid sequence represented by SEQ ID NO: 1 and having the growth factor activity of the protein comprising the amino acid sequence represented by SEQ ID NO: 1;
   (g) a protein comprising the amino acid sequence represented by SEQ ID NO: 32;
   (h) a protein comprising the amino acid sequence represented by SEQ ID NO: 33;
   (i) the protein according to any one of the above (a) to (h) where the growth factor activity of the protein is a growth-promoting activity for smooth muscle cells; and
   (j) the protein according to the above (i) where the smooth muscle cells are derived from rat.
(34) A method for detecting at least one disease selected from a group consisting of diseases associated with abnormal stimulation of angiogenesis, eye diseases based on abnormal angiogenesis, arthritis based on abnormal angiogenesis, skin diseases associated with abnormal angiogenesis, diseases associated with abnormal stimulation of vascular permeability, diseases associated with abnormal differentiation and proliferation of smooth muscle cells, diseases associated with abnormal differentiation and proliferation of kidney mesangial cells, diseases associated with abnormal differentiation and proliferation of blood stem cells, diseases based on abnormality in osteoblasts, diseases based on abnormality in pancreatic β cells, ischemic diseases and diseases associated with the delay of wound healing, which comprises using the antibody according to any one of (1) to (17), the antibody fragment according to (18) or the antibody derivative according to (19).
(35) The method according to (34), wherein the disease associated with abnormal stimulation of angiogenesis is selected from solid tumor and tumor metastasis, the eye disease based on abnormal angiogenesis is selected from a group consisting of diabetic retinopathy, retinopathy of prematurity, age-related macular degeneration and neovascular glaucoma, the arthritis based on abnormal angiogenesis is rheumatoid arthritis, the skin disease associated with abnormal angiogenesis is psoriasis, the disease associated with abnormal acceleration of vascular permeability is selected from a group consisting of ascites cancer, cancer with pleural effusion, Crow-Fukase syndrome and ovarian hyperstimulation syndrome, the disease associated with abnormal differentiation and proliferation of smooth muscle cells is arterosclerosis, the disease associated with abnormal differentiation and proliferation of kidney mesangial cells is glomerulonephritis, the disease associated with abnormal differentiation and proliferation of blood stem cells is anemia, the disease based on abnormality in osteoblasts is osteoporosis, the disease based on abnormality in pancreatic β cells is diabetes mellitus, the ischemic disease is selected from a group consisting of cerebral infarction, acute myocardial infarction and peripheral artery occlusion and the disease associated with the delay of wound healing is selected from a group consisting of neurogenic ulcer of lower limb and diabetic ulcer of lower limb.

The present invention relates to an antibody which specifically recognizes VPLF and inhibits the activity of VPLF.

VPLF is a novel growth factor belonging to a VEGF/PDGF superfamily cloned from cDNA libraries derived from human neural precursor cell NT-2 and human ovarian cancer tissue.

With regard to VPLF in the present invention, there may be given a protein which comprises the amino acid sequence represented by SEQ ID NO: 1, a protein which comprises an amino acid sequence where one or more amino acid(s) is/are deleted, substituted or added in the amino acid sequence represented by SEQ ID NO: 1 and a protein which comprises an amino acid sequence having 60% or more homology to the amino acid sequence represented by SEQ ID NO: 1 and has a growth factor activity of the protein.

With regard to VPLF in the present invention, there may be further given protein which comprises a partial sequence of the amino acid sequence represented by SEQ ID NO: 1, contains eight cysteine residues conserved among the factors belonging to a VEGF/PDGF superfamily and has a growth factor activity of the above-mentioned VPLF and a protein which comprises an amino acid sequence where one or more amino acid(s) is/are deleted, substituted or added in the amino acid sequence represented by SEQ ID NO: 1, contains eight cysteine residues conserved among the factors belonging to a VEGF/PDGF superfamily and has a growth factor activity of the above-mentioned VPLF. With regard to the above-mentioned partial sequence, there may be exemplified an amino acid sequence where N-terminal sequence is deleted from the amino acid sequence represented by SEQ ID NO: 1 and, to be more specific, there may be given an amino acid sequence comprising from the 227th phenylalanine to the 345th glycine in the amino acid sequence represented by SEQ ID NO: 1. With regard to such a protein, there may be an exemplified protein which contains an amino acid sequence comprising from the 227th phenylalanine to the 345th glycine in the amino acid sequence represented by SEQ ID NO: 1 and has an amino acid sequence (SEQ ID NO: 32) where four amino acids (Asp-Pro-Ser-Pro: SEQ ID NO: 34) are added to the N terminal and a protein which has an amino acid sequence (SEQ ID NO: 33) where two amino acids (Ser-Pro) are added.

VPLF in the present invention is a protein which is characterized in having a growth factor activity. VPLF in the present invention has a growth factor activity such as growth-promoting activity for vascular endothelial cells, migration-promoting activity, tube formation promoting activity, protease production promoting activity, angiogenesis stimulating activity, vascular permeability stimulating activity, activity to promote differentiation and proliferation of hemoangiogenic stem cells, monocyte migration promoting activity, maturation inhibiting activity for dendritic cells, activity to promote migration and proliferation of mesenchymal cells including smooth muscle cells, etc. and, particularly preferably, it has a growth-promoting activity for smooth muscle cells. Up to now, growth-promoting activity has been investigated for a human undifferentiated hematopoietic cell (CD 34 positive human bone marrow cells; manufactured by Biowhittaker), HMVEC which is a microvascular endothelial cell derived from human skin (manufactured by Kurabo) and RSMC which is a smooth muscle cell derived from rat (FEBS Letters, 425, 123, 1998) using VPLFΔN (an N-terminal deleted mutant where from the 1st to the 226th amino acids are deleted in SEQ ID NO: 1) expressed in insect cells whereupon a concentration-depending growth-promoting activity has been noted for RSMC.

The protein which comprises an amino acid sequence where one or more amino acid(s) is/are deleted, substituted or added in the amino acid sequence represented by SEQ ID NO: 1 and has a growth factor activity of the protein can be prepared by, for example, introduction of a site-direcited mutation to a DNA which encodes the protein having the amino acid sequence represented by SEQ ID NO: 1 (hereinafter, referred to as "VPLF of SEQ ID NO: 1") using a method for site-direted mutagenesis described, for example, in Molecular Cloning, A Laboratory Manual, Second Edition, Cold Spring Harbor Laboratory Press (1989) (hereinafter, referred to as "Molecular Cloning, Second Edition"), Current Protocols in Molecular Biology, John Wiley & Sons (1987-1997) (hereinafter, referred to as "Current Protocols in Molecular Biology"), Nucleic Acids Research, 10, 6487 (1982), Proc. Natl. Acad. Sci. USA, 79, 6409 (1982), Gene, 34, 315 (1985), Nucleic Acids Research, 13, 4431 (1985) and Proc. Natl. Acad. Sci. USA, 82, 488 (1985). Although there is no particular limitation for the numbers of amino acid to be deleted, substituted or added, that is preferably one to dozens such as 1 to 20 or, more preferably, one to several such as 1 to 5 amino acid(s). For the object that the VPLF in the present invention has a function as a growth factor, it preferably has at least 60% or more homology, more preferably 80% or more or, still more preferably, 95% or more homology to the amino acid sequence represented by SEQ ID NO: 1. In addition, even in the case where deletion, substation or addition of amino acid(s) is introduced as such, it is still preferred that the product contains eight cysteine residues conserved among the factors belonging to aVEGF/PDGF superfamily.

The protein which comprises a partial sequence of the amino acid sequence represented by SEQ ID NO: 1 can be prepared by a method known among persons skilled in the art and, for example, it can be prepared by deleting a part of DNA encoding the amino acid sequence represented by SEQ ID NO: 1 and culturing a transformant transfected with an expression vector containing the above deleted DNA. It is also possible to prepare a protein where one or more amino acid(s) is/are deleted, substituted or added in a partial sequence of the amino acid sequence represented by SEQ ID NO: 1 by the same method as mentioned above based on the DNA or protein prepared as such.

With regard to the DNA encoding VPLF in the present invention, there may be exemplified a DNA which has a nucleotide sequence reperesented by SEQ ID NO: 2 as a DNA encoding the VPLF of SEQ ID NO: 1 although that is not limited to it. Usually, there are plural genetic codes for one amino acid and, therefore, a DNA having a nucleotide sequence which is different from SEQ ID NO: 2 may be used in the present invention so far as it encodes the amino acid sequence represented by SEQ ID NO: 1. Further, since the amino acid sequence of VPLF in the present invention may be other than SEQ ID NO: 1 as described above, DNA which encodes such a protein may be also used in the present invention. Examples of the DNA encoding VPLF in the present invention are a DNA which has a nucleotide sequence represented by SEQ ID NO: 2 and DNA which hybridizes to the above DNA under stringent conditions.

The DNA which hybridizes under stringent conditions means a DNA which is prepared by colony hybridization, plaque hybridization, Southern blot hybridization, etc. using the DNA having a nucleotide sequence represented by SEQ ID NO: 2 and, to be more specific, there may be exemplified DNA which is able to be identified by carrying out a hybridization at 65°C in the presence of 0.7 to 1.0 mol/l of sodium chloride using a filter on which a DNA derived from colony or plaque is immobilized and then washing the filter under the condition of 65°C using 0.1 to 2-fold concentration of SSC solution (1-fold concentration of SSC solution comprises 150 mmol/l of sodium chloride and 15 mmol/l of sodium citrate). Hybridization may be carried out by a method according to that described in "Molecular Cloning, Second Edition", "Current Protocols in Molecular Biology" and "DNA Cloning I: Core Techniques, A Practical Approach, Second Edition, Oxford University (1995)", etc. With regard to the DNA which is able to hybridize, more specific examples are a DNA having at least not less than 60%, preferably not less than 80% and, more preferably, not less than 95% of homology to the nucleotide sequence represented by SEQ ID NO: 2.

Abbreviations for amino acids and protective groups therefor used in the present specification are in accordance with the recommendations of the IUPAC-IUB Joint Commission on Biochemical Nomenclature [European Journal of Biochemistry, 138, p. 9, 1984].

The abbreviations shown below represent the corresponding following amino acids unless otherwise stipulated. Ala: L-alanine; Arg: L-arginine; Asn: L-asparagine; Asp: L-aspartic acid; Asx: L-aspartic acid or L-asparagine; Cys: L-cysteine; Gln: L-glutamine, Glu: L-glutamic acid; Glx: L-glutamic acid orL-glutamine; Gly:Glycine; Ile:L-isoleucine; Leu: L-leucine; Lys: L-lysine; Phe: L-phenylalanine; Pro: L-proline; Ser: L-serine; Thr: L-threonine; Trp: L-tryptophane.

The abbreviations shown below represent protective groups and side chain protective groups for the corresponding following amino acids. Fmoc: 9-fluorenylmethyloxycarbonyl; tBt: tert-butyl; Trt: trityl; Pmc: 2,2,5,7,8-pentamethylchroman-6-sulfonyl; Boc: tert-butyloxycarbonyl; Fmox-Arg(Pmc)-OH: N^{α}-9-fluorenylmethyl-oxycarbonyl-N^{ε}-trityl-L-glutamine; Fmoc-Glu(ItBu)-OH: N^{α}-9-fluorenylmethyloxycarbonyl-N^{g}-2,2,5,7,8-pentamethylchroman-6-sulfonyl-L-arginine; Fmoc-Asn(Trt)-OH: N^{α}-9-fluorenylmethyloxycarbonyl-N^{γ}-trityl-L-asparagine; Fmoc-Asp(OtBu)-OH: N^{α}-9-fluorenylmethyloxycarbonyl-L-aspartic acid β-tert-butyl ester; Fmoc-Cys(Trt)-OH: N^{α}-9-fluorenylmethyloxycarbonyl-S-trityl-L-cysteine; Fmoc-Gln(Trt)-OH: N^{α}-9-fluorenylmethyloxycarbonyl-L-glutamic acid γ-tert-butyl ester; Fmoc-Lys(Boc)-OH: N^{α}-9-fluorenylmethyloxycarbonyl-N^{ε}-tert-butyloxycarbonyl-L-lysine; Fmoc-ser(tBu)-OH: N^{α}-9-fluorenylmethyloxycarbonyl-O-tert-butyl-L-serine; Fmoc-Thr(tBu)-OH: N^{α}-9-fluorenylmethyloxycarbonyl-O-tert-butyl-L-threonine; and Fmoc-Trp(Boc)-OH: N^{α}-9-fluorenylmethyloxycarbonyl-N^{ind}-tert-butyloxycarbonyl-L-tryptophane.

Further, the following abbreviations mean the corresponding following reaction solvents, reaction reagents, etc. HBTU: 2-(1H-benzotriazol-1-yl)-1,1,3,3-tetramethyluronium hexafluorophosphate; DIPC: N,N'-diisopropylcarbodiimide; HOBt: N-hydroxybenzotriazole; DMF: N,N-dimethylformamide; NMP: N-methylpyrrolidone; TFA: trifluoroacetic acid; and DIEA: diisopropylethylamine.

Unless otherwise mentioned, homology values mentioned in the present specification may be a value which is calculated using a homology search program which has been known for persons skilled in the art, and it is preferably calculated using parameter of default (initially-set one) in BLAST [J. Mol. Biol., 215, 403 (1990)] in the case of a nucleotide sequence while, in the case of an amino acid sequence, it is preferably calculated using parameter of default (initially-set one) in BLAST 2 [Nucleic Acids Res., 25, 3389 (1997); Genome Res., 7, 649 (1997); http://www.ncbi.nlm.nih.gov/Education/BLASTinfo/information 3.html].

As to the antibody of the present invention, a polyclonal antibody and a monoclonal antibody are given and, preferably, a monoclonal antibody such as an antibody produced by hybridoma, a humanized antibody and a human antibody are given.

In the present specification, "hybridoma" means a cell which produces a monoclonal antibody having the desired antigen specificity and is prepared by a cell fusion of a B cell prepared by immunization of antigen to non-human mammals with a myeloma cell derived from mouse, etc.

As to the humanized antibody, there are given humanized chimeric antibody, humanized complementarity determining region (hereinafter, referred to as "CDR")-grafted antibody, etc.

In the present specification, "humanized chimeric antibody" means an antibody comprising antibody heavy chain variable region of non-human animals (it will be hereinafter referred to "HV" or "VH" since heavy chain is called "H chain" and variable region is called "V region") and antibody light chain variable region thereof (it will be hereinafter referred to "LV" or "VL" since light chain is called "L chain" and variable region is called "V region") and also a heavy chain constant region of human antibody (it will be hereinafter referred to "CH" since heavy chain is called "H chain" and constant region is called "C region") and light chain constant region of human antibody (it will be hereinafter referred to "CL" since light chain is called "L chain" and constant region is called "C region") With regard to the above-mentioned non-human animals, any animal may be used so far as it is able to produce hybridoma such as mouse, rat, hamster and rabbit.

The humanized chimeric antibody of the present invention can be produced in such a manner that cDNAs encoding VH and for VL are obtained from hybridoma which specifically reacts with VPLF and produces a monoclonal antibody inhibiting the activity of VPLF, then each of them is inserted into expression vector for animal cells having genes which encode human antibody CH and human antibody CL to construct a humanized chimeric antibody expressing vector is constructed and then it is introduced into animal cells to be expressed therein.

With regard to CH of humanized chimeric antibody, although any CH may be used so far as it belongs to human immunoglobulin (hereinafter, referred to as "hIg"), that of an hIgG class is preferred and, further, any of subclasses such as hIgG1, hIgG2, hIgG3, hIgG4, etc. belonging to an hIgG class may be used. With regard to CL of humanized chimeric antibody, any CL may be used so far as it belongs to hIg and that of κ class or λ class may be used.

In the present specification, "humanized CDR-grafted antibody" means an antibody where an amino acid sequence of CDR of VH and VL of antibody of non-human animals is grafted to an appropriate position of VH and VL of a human antibody.

The humanized CDR-grafted antibody can be produced in such a manner that there is constructed cDNA encoding V region where CDR sequence of VH and VL of antibody of non-human animals which specifically reacts with VPLF and inhibits the activity of VPLF is grafted to CDR region of VH and VL of any human antibody, each of them is inserted into expression vector for animal cells having genes which encode CH of a human antibody and for CL of a human antibody to construct a humanized CDR-grafted antibody expression vector and the said expression vector is introduced into animal cells for expression thereof.

With regard to CH of the humanized CDR-grafted antigen, although CH may be used so far as it belongs to hIg, the preferred one is that of an hIgG class and, further, any of subclasses such as hIgG1, hIgG2, hIgG3 and hIgG4 belonging to an hIgG class may be used. With regard to CL of the humanized CDR-grafted antibody, any CL may be used so far as it belongs to hIg and that of κ class or λ class may be used.

Originally, human antibody means an antibody which is naturally present in a human body. However, a human antibody phage library prepared as a result of recent progress in technology of genetic engineering, cell technology and developmental technology, antibody which is obtained froma human antibody-producing transgenic animals, etc. are also included therein.

In order to obtain an antibody existing in a human body, it is possible, for example, that human peripheral lymphocytes are isolated, immortalized by infection with EB virus or the like and cloned whereupon lymphocytes producing the said antibody can be cultured and, thereafter, the said antibody is purified from the culture.

The humanized antibody phage library is a library where antibody gene prepared from human B cell is inserted into phage gene whereupon antibody fragments such as Fab and single-stranded antibody are expressed on the surface of the phage. From the said library, it is possible to recover the phage which expresses antibody fragments having the desired antigen binding activity using a binding activity to the base where the antigen is immobilized as an index. The said antigen fragments may also be converted by a genetic engineering technique to a human antibody molecule comprising two complete H chains and two complete L chains.

The humanized antibody producing transgenic animal means an animal where human antibody gene is integrated into a cell. To be more specific, human antibody gene is introduced into mouse ES cell and the said ES cell is transplanted to primary embryo of another mouse to be developed and a human antibody producing transgenic animal can be prepared. With regard to the method for the preparation of human antibody from the human antibody producing transgenic animals, there may be exemplified a method where human antibody producing hybridoma is obtained from the said transgenic animal according to a method of preparation of hybridoma which is carried out for non-human mammals and the said hybridoma is cultured whereupon human antibody is produced and accumulated in the culture.

With regard to the antibody fragment, there may be exemplified Fab (fragment of antigen binding), Fab', F(ab')₂, single-stranded antibody (single chain Fv; hereinafter, referred to as "scFv"), disulfide stabilized antibody (hereinafter, referred to as "dsFv") and peptide containing CDR.

Fab is an antibody fragment of a molecular weight of about 50,000 having an antigen binding activity where, among the fragments obtained by the treatment of IgG with papain which is aprotease (cleaving takes place at the 224th amino acid residue of H chain), about one half of amino acids at the N-terminal side of H chain and a whole L chain are bound by way of a disulfide bond.

Fab of the present invention can be prepared by the treatment of antibody which specifically reacts with VPLF and inhibits the activity of VPLF with papain which is a protease. Alternatively, DNA encoding Fab of the said antibody is inserted into an expression vector for prokaryote or an expression vector for eukaryote and the said vector is introduced into prokaryote or eukaryote to express whereupon Fab can be produced.

F(ab')₂ is an antibody fragment of a molecular weight of about 100,000 having an antigen binding activity and is a bit larger than a product where Fab is bound via a disulfide bond in a hinge region among the fragments obtained by the treatment of IgG with pepsin which is a protease (cleaving takes place at the 234th amino acid residue of H chain).

F(ab')₂ of the present invention can be prepared by the treatment of antibody which specifically reacts with VPLF and inhibits the activity of VPLF with pepsin which is a protease. Alternatively, it can be prepared by subjecting the following Fab' to a thioether bonding or a disulfide bonding.

Fab' is an antibody fragment of a molecular weight of about 50,000 having an antigen binding activity where a disulfide bond of a hinge region of the above F(ab')₂ is cleaved.

Fab' of the present invention can be prepared by the treatment of F(ab')₂ which specifically reacts with VPLF and inhibits the activity of VPLF with dithiothreitol which is a reducing agent. Alternatively, a DNA encoding Fab' fragment of the said antibody is inserted into an expression vector for prokaryote or an expression vector for eukaryote and the said vector is introduced into prokaryote or eukaryote to express whereupon Fab' can be produced.

scFv is a VH-P-VL or VL-P-VH polypeptide where one VH and one VL are linked using an appropriate peptide linker (hereinafter, referred to as "P"). With regard to VH and VL contained in the scFv of the present invention, any of antibody produced by the hybridoma of the present invention, humanized antibody and human antibody may be used.

The scFv of the present invention can be prepared by such a manner that cDNAs encoding VH and VL of an antibody which specifically reacts with VPLF and inhibits the activity of VPLF is prepared, a DNA encoding scFv is constructed, the DNA is inserted into an expression vector for prokaryote or an expression vector for eukaryote and the vector is introduced into prokaryote or eukaryote to express.

dsFv is that where polypeptides in which one amino acid residue in each of VH and VL is substituted with a cysteine residue are linked via a disulfide bond between the said cysteine residues. The amino acid residue substituted with a cysteine residue can be selected based on a steric structure presumption of an antibody according to a method shown by Reiter, et al. [Protein Engineering, 7, 697 (1994)]. With regard to VH and VL contained in the dsFv of the present invention, any of antibody produced by the hybridoma of the present invention, humanized antibody and human antibody may be used.

The dsFv of the present invention can be prepared by such a manner that cDNAs encoding VH and VL of an antibody which specifically reacts with VPLF and inhibits the activity of VPLF is prepared, DNAs encoding dsFv are constructed, the said DNAs are inserted into an expression vector for prokaryote or an expression vector for eukaryote and the said vector is introduced into prokaryote or eukaryote to express.

Peptide containing CDR is constructed by containing at least one region of H-chain or L-chain CDR. A plurality of CDR can be linked either directly or via an appropriate peptide linker.

The peptide containing CDR of the present invention can be prepared by such a manner that cDNAs encoding VH and VL of an antibody which specifically reacts with VPLF and inhibits the activity of VPLF are prepared, a DNA encoding CDR is constructed, the said DNA is inserted into an expression vector for prokaryote or an expression vector for eukaryote and the said vector is introduced into prokaryote or eukaryote to express.

The peptide containing CDR can also be produced by a chemical synthetic method such as Fmoc method (fluorenylmethyloxycarbonyl method) and tBoc method (tert-butyloxycarbonyl method).

Derivative of the antibody of the present invention is an antibody where radioisotope, protein, low-molecular-weight compound or the like is bound to antibody produced by the hybridoma of the present invention, humanized antibody or human antibody or antibody fragment thereof.

The derivative of the antibody of the present invention can be produced by binding of radioisotope, protein, low-molecular-weight compound or the like to N-terminal side or C-terminal side of H chain or L chain, , an appropriate substituent or side chain or sugar chain of the antibody or antibody fragment which specifically reacts with VPLF and inhibits the activity of VPLF by a chemical means [Kotai Kogaku Nyumon (Introduction to Antibody Technology) by Osamu Kanemitsu, 1994, K. K. Chijin Shokan].

Alternatively, the antibody derivative of the present invention may also be produced by a genetic engineering technique where DNA encoding an antibody or an antibody fragment which specifically reacts with VPLF and inhibits the activity of VPLF is bound to a DNA encoding the protein which is to be bound, then inserted into an expression vector and the expression vector is introduced into a host cell.

With regard to the above-mentioned radioisotope, there may be exemplified ¹³¹I and ¹²⁵I and that may be bound to an antibody by a chloramines T method, etc.

With regard to the above-mentioned low-molecular-weight compound (agent), there may be exemplified anti-cancer agents, for example, alkylating agent such as nitrogen mustard and cyclophosphamide, antimetabolite such as 5-fluorouracil and methotrexate, antibiotic substance such as daunomycin, bleomycin, mitomycin C, daunorubicin and doxorubicin plant alkaloid such as vincristine, vinblastine and vindesin and hormone agent tamoxifen and dexamethasone [Rinsho Shuyogaku (Clinical Oncology), edited by Japan Clinical Tumor Study Group, 1996, published by Gan To Kagakuryoho Sha] and anti-inflammatory agents, for example, steroidal agent such as hydrocortisone and prednisone, nonsteroidal agent such as aspirin and indomethacin, immunomodulator such as gold thiomalate and penicillamine, immunosuppressant such as cyclophosphamide and azathioprine and antihistamic agent such as chlorpheniramine maleate and clemastine [Ensho to Koensho Ryoho (Inflammation and Anti-Inflammatory Treatment), 1982, published by Ishiyaku Shuppan K. K.]. With regard to the method for binding daunomycin to antibody, there may be exemplified a method where amino group of daunomycin and that of antibody are crosslinked by glutaraldehyde and a method where amino group of daunomycin and carboxyl group of antibody are crosslinked by a water-soluble carbodiimide.

With regard to the above-mentioned protein, cytokine which activates the cells in charge of immune is suitable and there may be exemplified human interleukin-2 (hereinafter, referred to as "hIL-2"), human granulocyte-macrophage-colony stimulating factor (hereinafter, referred to as "hGM-CSF"), human macrophage colony stimulating factor (hereinafter, referred to as "hM-CSF") and human interleukin 12 (hereinafter, referred to as "hIL-12"). It is also possible to use toxin such as ricin and diphtheria toxin in order to directly hinder the cancer cells. For example, a fused antibody with protein can be produced in such a manner that cDNA encoding protein is linked to cDNA encoding antibody or antibody fragment to construct DNA which encodes fused antibody, the said DNA is inserted into an expression vector for prokaryote or eukaryote and the expression vector is expressed by introducing into prokaryote or eukaryote.

The present invention is now illustrated in detail as hereunder.

### 1. Preparation of DNA which encodes VPLF in the present invention

DNA which encodes VPLF in the present invention can be prepared in such a manner that mRNA derived from human ovary or testis is isolated to prepare its cDNA library and then the said cDNA library is screened whereupon the aimed clone is obtained.

With regard to the human ovary or testis mRNA, commercially available one (such as that manufactured by Clontech) may be used or it may be prepared from human ovary or ovarian cancer tissues (hereinafter, referred to as "ovary-derived tissues") or from human testis or human fetal testis-derived teratocarcinoma (hereinafter, referred to as "testis-derived tissues") as will be mentioned below. In the latter case, total RNA is firstly prepared from ovary-derived tissues or from testis-derived tissues and then mRNA can be isolated from the total RNA.

With regard to the method for the preparation of total RNA from the ovary-derived tissues or from the testis-derived tissues, there are exemplified a guanidine thiocyanate-cesium trifluoroacetate method [Methods in Enzymology, 154, 3 (1987)] and a guanidine acidic thiocyanate-phenol-chloroform (AGPC) method[Analytical Biochemistry, 162, 156 (1987); Jikken Igaku, 9, 1937 (1991)]. With regard to the method for the isolation of mRNA as poly(A)⁺RNA from total RNA, there is exemplified an oligo(dT) immobilized cellulose column method (Molecular Cloning, Second Edition). Alternatively, mRNA can be prepared using a kit such as Fast Track mRNA Isolation Kit (Invitrogen), Quick Prep mRNA Purification Kit (Pharmacia), etc.

The cDNA library is prepared from the human ovary-derived tissue or testis-derived tissue mRNA prepared as such. With regard to the method for the preparation of the cDNA library, there are exemplified a method described in Molecular Cloning, Second Edition, Current Protocols in Molecular Biology, etc. and a method using commercially available kit such as Superscript Plasmid System for cDNA Synthesis and Plasmid Cloning (Life Technologist) and ZAP-cDNA Synthesis Kit (Stratagene).

With regard to the cloning vector for the preparation of the cDNA library, any vector including phage vector and plasmid vector may be used so far as it is able to autonomously replicate in *Escherichia coli* K12 strain. To be more specific, there may be exemplified ZAP Express [Stratagene; Strategies, 5, 58 (1992); pBluescript II SK(+) [Nucleic Acids Research, 17, 9494 (1989)], Lambda ZAP II (Stratagene), λgt 10, λgt 11 [DNA Cloning, A Practical Approach, 1, 49(1985)], λTriplEx (Clontech), λExCell (Pharmacia), pT7T 318U (Pharmacia), pcD2 [Mol. Cell. Biol., 3, 280 (1983)] and pUC 18 [Gene, 33, 103 (1985)].

With regard to the host microorganism, any microorganism may be used so far as it is a microorganism belonging to the genus *Escherichia* or particularly to *Escherichia coli* (hereinafter, referred to as "*E. Coil*"). To be more specific, there may be used *E. coli* XL1-Blue MRF' [Stratagene; Strategies, 5, 81 (1992)], *E. coli* C600 [Genetics, 39, 440 (1954)], *E. coli* Y1088 [Science, 222, 778 (1983)], *E. coli* Y1090 [Science, 222, 778 (1983)], *E. coli* NM522 [J. Mol. Biol., 166, 1 (1983)], *E. coil* k802 [J. Mol. Biol., 16, 118 (1966)], *E. coli* JM105 [Gene, 38, 275 (1985)], etc.

Although the cDNA library may be used as it is for a screening thereafter, in order to lower the ratio of the non-full-length cDNA and to obtain the full-length cDNA at a high efficiency as much as possible, it is also possible to use a cDNA library which is prepared using an oligo cap method developed by Sugano [Gene, 138, 171 (1994); Gene, 200, 149 (1997); Tampakushitsu Kakusan Koso, 41, 603 (1996); Jikken Igaku, 11, 2491 (1993); cDNA Cloning, published by Yodosha (1996); Idenshi Library no Sakuseiho (Method for Preparing Genetic Library), published by Yodosha (1994)] for the following screening.

Screening of the cDNA library is carried out by firstly determining the nucleotide sequence of total clone contained in the library and then by comparing each nucleotide sequence with the known sequence. The above-mentioned determination of the nucleotide sequence of the total clone is carried out by such a manner that each clone is isolated from the cDNA library which is prepared as mentioned above and then the nucleotide sequence of cDNA for each clone is determined from the terminal. Isolation of each clone from the CDNA library can be carried out by a method known by persons skilled in the art such as a single colony isolation method (Molecular Cloning, Second Edition). Determination of nucleotide sequence for each clone may be carried out by a commonly used nucleotide sequence analyzing method such as a dideoxymethod of Sanger, et al. [Proc. Nat1. Acad. Sci. USA, 74, 5463 (1977)] or by means of analysis using a nucleotide sequence analyzing apparatus such as ABI PRISM 377 DNA Sequencer (manufactured by PE Biosystems).

Thereafter, a nucleotide sequence of each clone is compared with the known sequences. The fact that the nucleotide sequence of each cDNA is novel can be confirmed by the following step that nucleotide sequence databases such as GenBank, EMBL and DDBJ are searched using a homology search program such as BLAST to confirm there is no nucleotide sequence showing a clear homology which is seemingly identical with the nucleotide sequence of the known gene in the database. With regard to the novel nucleotide sequence of the DNA obtained by such a method, there is exemplified a nucleotide sequence represented by SEQ ID NO: 2.

The amino acid sequence (SEQ ID NO: 1) of VPLF obtained by translation of DNA comprising a nucleotide sequence represented by SEQ ID NO: 2 has a homology of 29%, 29%, 25%, 29%, 26%, 36% and 28% each to each of amino acid sequences of human VEGF, human VEGF-B, human VEGF-C, human VEGF-D and human PlGF belonging to a VEGF family and each of amino acid sequences of human PDGF-A and human PDGF-B belonging to a PDGF family, respectively according to a homology analysis using BLAST 2. Further, there is a homology of 29% and 30% each to the amino acid sequences of NZ2-VEGF and NZ7-VEGF, respectively, belonging to the same VEGF/PDGF superfamily.

It has been known in a VEGF/PDGF superfamily that eight cysteine residues which are important for a disulfide bond formation between dimers, a disulfide bond formation in a protein molecule and expression of activity are present (J. Biol. Chem., 269, 32879-32885, 1994) and that those eight cysteine residues are conserved among the factors belonging to a VEGF/PDGF superfamily. In the amino acid sequence represented by SEQ ID NO: 1, positions and numbers of the cysteine residues which are essential for the formation of the said motif are completely conserved as well. Accordingly, it is apparent that VPLF of SEQ ID NO: 1 has an activity as a growth factor belonging to a VEGF/PDGF superfamily.

The growth factor belonging to a VEGF/PDGF superfamily has been shown to be involved in diseases associated with abnormal stimulation of angiogenesis such as solid tumor and tumor metastasis, diseases of eye based on abnormal angiogenesis such as diabetic retinopathy, retinopathy of prematurity, age-related macular degeneration and neovascular glaucoma, arthritis based on abnormal angiogenesis such as rheumatoid arthritis, skin diseases associated with abnormal angiogenesis such as psoriasis, diseases associated with abnormal vascular permeability such as ascites cancer, cancer with pleural effusion, Crow-Fukase syndrome and ovarian hyperstimulation syndrome, diseases associated with abnormal differentiation and proliferation of smooth muscle cells such as arterosclerosis, diseases associated with abnormal differentiation and proliferation of kidney mesangial cells such as glomerulonephritis, diseases associated with abnormal differentiation and proliferation of blood stem cells such as anemia, diseases based on abnormal osteoblasts such as osteoporosis, diseases based on abnormal pancreatic β-cells such as diabetes mellitus, ischemic diseases such as cerebral infarction, acute myocardial infarction and peripheral artery occlusion and diseases associated with the delay of healing of wound such as neurogenic ulcer of lower limb and diabetic ulcer of lower limb. Since an antibody which is able to inhibit the activity of proliferation of VPLF is able to detect and quantify the VPLF, it can be a diagnostic for diseases associated with abnormal stimulation of angiogenesis such as solid tumor and tumor metastasis, diseases of eye based on abnormal angiogenesis such as diabetic retinopathy, retinopathy of prematurity, age-related macular degeneration and neovascular glaucoma, arthritis based on abnormal angiogenesis such as rheumatoid arthritis, skin diseases associated with abnormal angiogenesis such as psoriasis, diseases associated with abnormal vascular permeability such as ascites cancer, cancer with pleural effusion, Crow-Fukase syndrome and ovarian hyperstimulation syndrome, diseases associated with abnormal differentiation and proliferation of smooth muscle cells such as arterosclerosis, diseases associated with abnormal differentiation and proliferation of kidney mesangial cells such as glomerulonephritis, diseases associated with abnormal differentiation and proliferation of blood stem cells such as anemia, diseases based on abnormal osteoblasts such as osteoporosis, diseases based on abnormal pancreatic β-cells such as diabetes mellitus, ischemic diseases such as cerebral infarction, acute myocardial infarction and peripheral artery occlusion and diseases associated with the delay of wound healing such as neurogenic ulcer of lower limb and diabetic ulcer of lower limb. Further, an antibody being able to inhibit the activity of growth factor belonging to a VEGF/PDGF superfamily has been shown to have an activity which is able to treat diseases associated with abnormal stimulation of angiogenesis such as solid tumor and tumor metastasis, diseases of eye based on abnormal angiogenesis such as diabetic retinopathy, retinopathy of prematurity, age-related macular degeneration and neovascular glaucoma, arthritis based on abnormal angiogenesis such as rheumatoid arthritis, skin diseases associated with abnormal angiogenesis such as psoriasis, diseases associated with abnormal vascular permeability such as ascites cancer, Crow-Fukase syndrome and ovarian hyperstimulation syndrome, diseases associated with abnormal differentiation and proliferation of smooth muscle cells such as arterosclerosis and diseases associated with abnormal differentiation and proliferation of kidney mesangial cells such as glomerulonephritis. Accordingly, an antibody which is able to inhibit the activity of growth factor of VPLF can be a therapeutic agent for diseases associated with abnormal stimulation of angiogenesis such as solid tumor and tumor metastasis, diseases of eye based on abnormal angiogenesis such as diabetic retinopathy, retinopathy of prematurity, age-related macular degeneration and neovascular glaucoma, arthritis based on abnormal angiogenesis such as rheumatoid arthritis, skin diseases associated with abnormal angiogenesis such as psoriasis, diseases associated with abnormal vascular permeability such as ascites cancer, Crow-Fukase syndrome and ovarian hyperstimulation syndrome, diseases associated with abnormal differentiation and proliferation of smooth muscle cells such as arterosclerosis and diseases associated with abnormal differentiation and proliferation of kidney mesangial cells such as glomerulonephritis.

After DNA comprising the nucleotide sequence represented by SEQ ID NO: 2 is once obtained and its nucleotide sequence is determined, it is now possible that a primer designed on the basis of nucleotide sequences of 5'-terminal and 3'-terminal of the said nucleotide sequence is prepared and that amplification of DNA is carried out by means of a PCR (PCRProtocols, Academic Press (1990)) using a cDNA or cDNA library synthesized from mRNA contained in cells or tissues such as ovary and testis of human being or non-human animals as a template whereupon the DNA encoding VPLF in the present invention is obtained.

It is also possible that a colony hybridization, a plaque hybridization (Molecular Cloning, Second Edition), etc. are carried out for a cDNA or cDNA library synthesized from mRNA contained in cells or tissues such as ovary and testis of human or non-human animals using a full-length DNA represented by SEQ ID NO: 2 or a part thereof as a probe whereupon the DNA encoding VPLF in the present invention is prepared.

Alternatively, it is possible that a chemical synthesis is conducted using a DNA synthesizer such as DNA Synthesizer Model 392 of Perkin-Elmer utilizing a phosphoamidite method on the basis of the determined nucleotide sequence of DNA whereupon the DNA encoding VPLF in the present invention is obtained.

With regard to the obtained DNA, recombination vector containing the said DNA is introduced into a host cell and the resulting transformant is used to express the protein, or homology of an amino acid sequence encoded by the said DNA to an amino acid sequence of VEGF, VEGF-B, VEGF-C, VEGF-D, PDGF-A, PDGF-B, PlGF, NZ2-VEGF or NZ7-VEGF is compared whereupon it can be confirmed that the said DNA is the DNA which encodes protein having the growth factor activity.

### 2. Production of VPLF in the present invention

VPLF in the present invention can be produced by, for example, carrying out the following method according to a method described, for example, in Molecular Cloning, Second Edition or Current Protocols in Molecular Biology whereupon DNA encoding it is expressed in host cell.

Firstly, a full-length cDNA is inserted at the lower stream of promoter of an appropriate expression vector to prepare a recombinant vector. At that time, if necessary, a DNA fragment of an appropriate length containing a part encoding the protein of the present invention is prepared from the full-length cDNA and the said DNA fragment may be used instead of the above-mentioned full-length cDNA. Thereafter, the said recombinant vector is introduced into a host cell suitable for the said expression vector whereupon the transformant producing the VPLF in the present invention can be obtained.

With regard to the host cell, any cell such as bacteria, yeast, an animal cell, an insect cell and a plant cell may used so far as it is able to express the aimed gene.

With regard to the expression vector, any vector in which autonomous replication in the used host cell or integration into chromosome is possible and that which contains a promoter at the position where it can transcribe DNA encoding VPLF in the present invention is able to be may be used.

When prokaryote such as bacterium is used as a host cell, it is preferred that the recombinant vector containing the DNA encoding VPLF in the present invention is able to be autonomously replicated in the prokaryote and also is a vector containing promoter, ribosome binding sequence, DNA of the present invention and transcription termination sequence. The said recombinant vector may also contain a gene which controls promoter.

Examples of the expression vector are pBTrp2, pBTac1, pBTac2 (all commercially available from Boehringer-Mannheim), pKK233-2 (Pharmacia), pSE280 (Invitrogen), pGEMEX-1 (Promega), pQE-8 (Qiagen), pKYP10 (Japanese Published Unexamined Patent Application No. 110600/1983), pKYP200 [Agricultural Biological Chemistry, 48, 669 (1984)], pLSA1 [Agric. Biol. Chem., 53, 277 (1989)], pGEL1 [Proc. Natl. Acad. Sci. USA, 82, 4306 (1985)], pBluescript II SK(-) (Stratagene), pTrs30 [prepared from *E. coli* JM109/pTrS30 (FERM BP-5407)], pTr32 [prepared from *E. coli* JM109/pTrS32 (FERMBP-5408)], pGHA2 [prepared from *E. coil* IGHA1 (FERMBP-400); Japanese Published Unexamined Patent Application No. 221091/1985], pGKA2 [prepared from *E. coli* IGKA2 (FERM BP-6798); Japanese Published Unexamined Patent Application No. 221091/1985], pTerm2 (US 4686191), US 4939094, US 5160735), pSupex, pUB110, pTP5, pC194, pEG400 [J. Bacteriol., 172, 2392 (1990)], pGEX (Pharmacia), pET system (Novagen) and pSupex.

With regard to the promoter, any promoter may be used so far as it is able to achieve the function in the host cell used. Its examples are promoters derived from *E. coli,* phage, etc. such as trp promoter (Pₜᵣₚ), lac promoter, P_{L} promoter, P_{R} promoter and T7 promoter. It is also possible to use promoters where design is artificially modified such as promoter where two Pₜᵣₚ are arranged in series (Pₜᵣₚ × 2), tac promoter, lac T7 promoter and let I promoter.

With regard to the above-mentioned recombinant vector, it is preferred to use a plasmid where the distance between Shine-Dalgarno sequence which is a ribosome binding sequence and initiation codon is adjusted to an appropriate extent (for example, 6 to 18 bases). In a nucleotide sequence of the DNA encoding VPLF in the present invention, it is possible to substitute a nucleotide so as to give the optimum codon for expressing in the host and, as a result thereof, productivity of the aimed protein can be improved. Further, in the above-mentioned recombinant vector, although a transcription termination sequence is not always necessary for expression of DNA encoding VPLF in the present invention, it is preferred to arrange a transcription termination sequence immediately downstream the structural gene.

With regard to the host cell, there may be exemplified a microorganism belonging to the genus *Escherichia,* the genus *Serratia,* the genus *Bacillus*, the genus *Brevibacterium*, the genus *Corynebacterium,* the genus *Microbacterium* and the genus *Pseudomonas* such as *E. coli* XL1-Blue, *E. coli* XL2-Blue, *E. coli* DH1, *E. coli* MC 1000, *E. coli* KY 3276, *E. coli* W 1485, *E. coli* JM 109, *E. coli* HB 101, *E. coli* No. 49, *E. coli* W 3110, *E. coli* NY 49, *Serratia ficaria, S. fonticola, S. liquefaciens, S. marcescens, Bacillus subtilis, B. amyloliquefaciens, Brevibacterium immariophilum* ATCC 14068, *B. saccharolyticum* ATCC 14066, *B. flavum* ATCC 14067, *B. lactofermentum* ATCC 13869, *Corynebacterium glutamicum* ATCC 13032, *C. acetoacidophilum* ATCC 13870, *Microbacterium ammoniaphilum* ATCC 15354 and *Pseudomonas* sp. D-0110.

With regard to the method for the transfection of recombinant vector, any method may be used so far as it is a method where DNA is transfected into the above-mentioned host cell and its examples are a method where calcium ion is used [Proc. Natl. Acad. Sci. USA, 69, 2110 (1972)], a protoplast method (Japanese Published Unexamined Patent Application No. 248394/1988) and a method described in Molecular & General Genetics, 168, 111 (1979).

When yeast is used as a host cell, examples of the expression vector are YEP 13 (ATCC 37115), YEp 24 (ATCC 37051), YCp 50 (ATCC 37419).

With regard to the promoter, any promoter may be used so far as it is able to achieve its function in yeast strain and its examples are promoter of the gene of glycolytic pathway such as hexose kinase, PHO5 promoter, PGK promoter, GAP promoter, ADH promoter, gal 1 promoter, gal 10 promoter, heat shock protein promoter, MFα1 promoter and CUP 1 promoter.

With regard to the host cell, there may be exemplified microorganisms belonging to the genus *Saccharomyces*, the genus *Kluyveromyces*, the genus *Trichosporon* and the genus *Schwanniomyces* such as *Saccharomyces cerevisiae, Schizosaccharomyces pombe, Kluyveromyces lactis, Trichosporon pullulans* and *Schwanniomyces alluvius.*

With regard to the method for the transfection of recombinant vector, any method may be used so far as it is a method for the transfection of DNA into yeast and its examples are an electroporation method [Methods. Enzymol., 194, 182 (1990)], a spheroplast method [Proc. Natl. Acad. Sci. USA, 84, 1929 (1978)], a lithium acetate method [J. Bacteriology, 183, 163 (1983)] and a method described in Proc. Natl. Acad. Sci. USA, 75, 1929 (1978).

Examples of the expression vector when animal cell is used as a host cell are pcDNAI, pcDM8 (commercially available from Funakoshi), pAGE 107 [Japanese Published Unexamined Patent Application No. 22979/1991; Cytotechnology, 3, 133 (1990)], pAS 3-3 (Japanese Published Unexamined Patent Application No. 227075/1990), pCDM 8 [Nature, 329, 840 (1987)], pcDNAI/Amp (Invitrogen), pREP 4 (Invitrogen), pAGE 103 [J. Biochemistry, 101, 1307 (1987)] and pAGE 210.

With regard to the promoter, any promoter may be used so far as it is able to achieve its function in animal cells and its examples are a promoter of IE (immediate early) gene of cytomegalovirus (CMV), an early promoter of SV 40, a promoter of retrovirus, a metallothionein promoter, a heat shock promoter and a SRα promoter. It is also possible to use an enhancer of IE gene of human CMV together with a promoter.

With regard to the host cell, there may be exemplified Namalwa cell which is a human cell, COS cell which is a simian cell, CHO cell which is a cell of Chinese hamster and HBT 5637 (Japanese Published Unexamined Patent Application No. 299/1988). With regard to a method for the transfectin of a recombinant vector, any method may be used so far as it is a method for the transfection of DNA into animal cells and its examples are an electroporation method [Cytotechnology, 3, 133 (1990)], a calcium phosphate method (Japanese Published Unexamined Patent Application No. 227075/1990) and a lipofection method [Proc. Natl. Acad. Sci. USA, 84, 7413 (1987)].

When insect cell is used as a host, it is possible to express the protein by a method which is described, for example, in Current Protocols in Molecular Biology, Baculovirus Expression Vectors, A Laboratory Manual, W. H. Freeman and Company, New York (1992) and Bio/Technology, 6, 47 (1988).

Thus, a recombinant gene transfer vector and a baculovirus are co-transfected into an insect cell to prepare a recombinant virus in a supernatant of the cultured insect cell and then the recombinant virus is infected to insect cell whereupon the protein can be expressed.

With regard to the gene transfer vector used in the said method, there may be exemplified pVL 1392, pVL 1393 and pBlueBacIII (all Invitrogen). With regard to the baculovirus, there may be used, for example, *autographa californica* nuclear polyhedrosis virus which is virus infecting to insects of family of *Noctuidae*.

With regard to the insect cell, there may be used Sf9 and Sf21 which are ovarian cells of *Spodoptera frugiperda* [Baculovirus Expression Vectors, A Laboratory Manual, W. H. Freeman and Company, New York (1992)], High 5 (Invitrogen) which is an ovarian cell of *Trichoplusia ni*, etc.

With regard to the method for the co-transfection of the above-mentioned recombinant gene transfer vector and the above-mentioned baculovirus into insect cells for the preparation of recombinant virus, there may be exemplified a calcium phosphate method (Japanese Published Unexamined Patent Application No. 227075/1990) and a lipofection method [Proc. Natl. Acad. Sci. USA, 84, 7413 (1987)].

When a plant cell is used as a host cell, there may be given, for example, Ti plasmid and tobacco mosaic virus vector as an expression vector.

With regard to the promoter, any promoter may be used so far as it is able to achieve its function in plant cells and its examples are 35S promoter of cauliflower mosaic virus (CaMV) and rice actin 1 promoter.

With regard to the host cell, there may be given, for example, plant cells such as tobacco, potato, tomato, carrot, soybean, rape, alfalfa, rice, wheat and barley.

With regard to the method for the transfection of recombinant vector, any method may be used so far as it is a method for the transfection of DNA into plant cells and its examples are an Agrobacterium method (Japanese Published Unexamined Patent Application No. 140855/1993, Japanese Published Unexamined Patent Application No. 70080/1985, WO Publication No. 94/00977), an electroporation method (Japanese Published Unexamined Patent Application No. 251887/1985) and a method using particle gun (gene gun) (JP Patent No. 2606856, JP Patent No. 2517813).

With regard to the method for the expression of gene, there may be carried out secretion production, fusion protein expression, etc. according to a method described, for example, in Molecular Cloning, Second Edition in addition to direct expression.

When the expression is carried out using yeast, animal cell, insect cell or plant cell, it is possible to prepare protein to which sugar or sugar chain is added.

VPLF in the present invention can be produced when the transformant prepared as mentioned above is cultured in a medium and VPLF in the present invention is produced and accumulated in a culture and recovered from the culture. A method for the cultivation of the said transformant in the medium may be carried out according to a method which is common by used for the cultivation of a host.

With regard to a medium for the cultivation of transformant which is obtained by the use of prokaryote such as *E. coli* or eukaryote such as yeast, any of natural medium and synthetic medium may be used so far as it is a medium which contains carbon source, nitrogen source, inorganic salt, etc. being able to be assimilated by the living organism whereby cultivation of the transformant can be efficiently carried out.

With regard to the carbon source, any carbon source may be used so far as it can be assimilated by the living organism and there may be used, for example, hydrocarbons such as glucose, fructose, sucrose, molasses containing them, starch and hydrolyzed starch; organic acids such as acetic acid and propionic acid; and alcohols such as ethanol and propanol.

With regard to the nitrogen source, any nitrogen source may be used so far as it can be assimilated by the living organism and there may be used, for example, ammonia; ammonium salts of inorganic or organic acids such as ammonium chloride, ammonium sulfate, ammonium acetate and ammonium phosphate; other nitrogen-containing compounds; and, furthermore, peptone, meat extract, yeast extract, corn steep liquor, hydrolyzed casein, soybean cake, hydrolyzed soybean cake, various fermented cell body and digested product thereof.

With regard to the inorganic salt, any inorganic salt may be used so far as it can be assimilated by the living organism and there may be used, for example, potassium dihydrogen phosphate, dipotassium hydrogen phosphate, magnesium phosphate, magnesium sulfate, sodium chloride, ferrous sulfate, manganese sulfate, copper sulfate and calcium carbonate.

The cultivation is usually carried out under an aerobic condition such as shaking culture or deep agitation stirring culture. Culturing temperature may be 15 to 40°C and culturing time is usually from 16 hours to 7 days. The pH during the cultivation is kept at 3.0 to 9.0. Adjustment of the pH is carried out using inorganic or organic salt, alkaline solution, urea, calcium carbonate, ammonia, etc. If necessary, an antibiotic substance such as ampicillin or tetracycline may be added to the medium during the cultivation.

In culturing the microorganism which is transformed with recombinant vector using an inductive promoter as a promoter, an inducer may be added to a medium if necessary. For example, in culturing the microorganism which is transformed with a recombinant vector using lac promoter, isopropyl-β-D-thiogalactopyranoside or the like may be added to the medium while, in cultivation the microorganism transformed with a recombinant vector using trp promoter, indole acrylic acid or the like may be added to the medium.

With regard to the medium for culturing the transformant obtained by the use of animal cell as a host, there may be used commonly-used RPMI 1640 medium [The Journal of the American Medical Association, 199, 519 (1967)], Eagle's MEM [Science, 122, 501 (1952)], Dulbecco-modified MEM [Virology, 8, 396 (1959)]:, 199 medium [Proc. Soc. Exp. Biol. Med. 73, 1 (1950)] or a medium prepared by addition of fetal bovine serum or the like to the above-mentioned medium.

Culturing is usually carried out for 1 to 7 day(s) under the condition of pH 6 to 8 and 30 to 40°C in the presence of 5% CO₂, etc. During the cultivation, an antibiotic substance such as kanamycin or penicillin may be added to the medium if necessary.

With regard to the medium for culturing a transformant prepared by using insect cell as a host, commonly-used TNM-FH medium (Pharmingen), Sf-900 II SFM medium (Life Technologies), ExCell 400 or ExCell 405 (both JRH Bioscience), Grance's Insect Medium (Grace, T. C. C., Nature, 195, 788 (1962)), etc. may be used.

Culturing is usually carried out for 1 to 5 day(s) under the condition of pH 6 to 7 at 25 to 30°C, etc. During the cultivation, an antibiotic substance such as gentamicin may be added to the medium if necessary.

The transformant where plant cell is used as a host cell may be cultured either as a cell or by differentiating into a plant cell or organ. With regard to the medium for culturing the said transformant, there may be used commonly-used Murashige and Scoog (MS) medium, White medium or a medium prepared by addition of auxin, cytokinin or other plant hormone thereto, etc.

Culturing is usually carried out for 3 to 60 days under the condition of pH 5 to 9 at 20 to 40°C. During the cultivation, an antibiotic such as kanamycin and hygromycin may be added to the medium if necessary.

As mentioned above, a transformant derived from microorganisms, animal cells or plant cells transfected with a recombinant vector in which DNA encoding the VPLF in the present invention is cultured by a conventional culturing method, the said VPLF is produced and accumulated and then the said VPLF is recovered from the culture whereupon the said VPLF can be produced.

With regard to the method for the expression of gene, it is also possible to conduct secretion production, fusion protein expression, etc. according to the method described in Molecular Cloning, Second Edition, etc. in addition to the direct expression.

With regard to the method for the production of VPLF in the present invention, there are available a method where it is produced in host cells, a method where it is secreted outside the host cells and a method where it is produced on extracellular membrane of host cells and an appropriate method can be selected by modifying the host cell used and the structure of the protein to be produced.

When the VPLF in the present invention is produced in host cells or on extracellular membrane, the said VPLF can be positively secreted outside the host cells by applying a method of Paulson, et al. [J. Biol. Chem., 264, 17619 (1989)], a method of Row, et al. [Proc. Natl. Acad. Sci. USA, 86, 8227 (1989), Gene Develop., 4, 1288 (1990)] or a method described in Japanese Published Unexamined Patent Application No. 336963/1993, WO Publication No. 94/23021, etc.

Thus, expression is carried out in such a form that signal peptide is added to the N-terminal side of protein containing the active site of VPLF in the present invention using a means of gene recombination whereupon the VPLF in the present invention can be positively secreted outside the host cells.

It is also possible that the productivity is enhanced utilizing a gene amplification system using dihydrofolate reductase gene, etc. according to a method mentioned in Japanese Published Unexamined Patent Application No. 227075/1990.

It is further possible that animal or plant cells transfected with the gene are re-differentiated to prepare an animal individual (transgenic non-human animal) or a plant individual (transgenic plant) transfected with the gene and that the VPLF in the present invention is produced using such an individual.

When the transformant is an animal individual or a plant individual, it is possible to produce the said VPLF by breeding or cultivating according to a common method to produce and accumulate the said VPLF and by recovering the said VPLF from the said animal individual or plant individual.

With regard to the method for the production of VPLF in the present invention using the animal individual, there is exemplified a method where the VPLF in the present invention is produced in the animal prepared by introduction of gene according to a known method [American Journal of Clinical Nutrition, 63, 639S (1996), American Journal of Clinical Nutrition, 63, 627S (1996), Bio/Technology, 9, 830 (1991)].

When the animal individual is used, it is possible to produce the said VPLF by such a manner that, for example, a transgenic non-human animal into which DNA encoding the VPLF in the present invention is raised to produce and accumulate the said protein in the said animal and the said VPLF is recovered from the said animal. With regard to the place for the production and accumulation in the said animal, there may be exemplified milk (Japanese Published Unexamined Patent Application No. 309192/1988) and egg of the said animal. With regard to a promoter used at that instance, any promoter may be used so far as it is able to achieve its function in animal and appropriately used ones are, for example, promoters which are specific to mammary cells such as α casein promoter, β casein promoter, β lactoglobulin promoter and whey acidic protein promoter.

With regard to the method for producing VPLF in the present invention using the plant individual, there is exemplified a method where a transgenic plant into which DNA encoding the VPLF in the present invention is introduced is cultivated according to a known method [Soshiki Baiyo, 20 (1994), Soshiki Baiyo, 21 (1995), Trends in Biotechnology, 15, 45 (1997)] to produce and accumulate the said VPLF in the said plant and the said VPLF is recovered from the said plant.

The VPLF which is produced by the transformant in the present invention can be isolated and purified, for example, as follows. Thus, when the VPLF in the present invention is expressed in a state of being dissolved in cells, the cells are recovered by centrifugal separation after completion of the cultivation, suspended in an aqueous buffer and disintegrated by means of ultrasonic disintegrator, French press, Manton-Gaulin homogenizer, dynomill, etc. to give a cell-free extract. It is possible to prepare a pure sample by subjecting a supernatant liquid obtained by centrifugal separation of the said cell-free extract to a common method for isolation and purification of enzymes such as solvent extraction method, salting-out method by ammonium sulfate, etc., desalting method, precipitation method using organic solvent, anion-exchange chromatographic method using resin such as Diaion HPA-75 (Mitsubishi Chemical), cation-exchange chromatographic method using resin such as S-Sepharose FF (Pharmacia), hydrophobic chromatographic method using resin such as butyl Sepharose or phenyl Sepharose, gel filtration method using molecular sieve, affinity chromatographic method, chromatofocusing method, electrophoretic method such as isoelectric electrophoresis, etc. either solely or in combination.

When the said VPLF is expressed by forming an insoluble matter in the cells, the cells are similarly recovered, disrupeted and centrifuged to recover the insoluble matter of protein as a precipitated fraction. The insoluble matter of the recovered protein is solubilized by a protein denaturant. When the said solubilized solution is diluted or dialyzed, the said protein is refolded to a normal steric structure and, thereafter,a pure sample of the said VPLF can be obtained by the same isolating and purifying method as mentioned above.

When the VPLF in the present invention or its derivative such as glycosylated substance thereof is secreted outside the cells, it is possible to recover the said VPLF or its derivative such as glycosylated substance thereof in the cultured supernatant. Thus, the said cultured product is treated by the same means as mentioned above such as centrifugation to prepare a soluble fraction and the said soluble fraction is subjected to the same isolating and purifying method as mentioned above whereupon a pure sample is obtained.

As to the VPLF obtained as such, there is exemplified VPLF having an amino acid sequence represented by SEQ ID NO: 1.

It is also possible that the VPLF in the present invention is produced by a chemical synthetic method such as Fmoc method (fluorenylmethyloxycarbonyl method) and tBoc method (tert-butyloxycarbonyl method). It is further possible to conduct a chemical synthesis using peptide synthesizers manufactured by Advanced ChemTech, Perkin-Elmer, Pharmacia, Protein Technology Instrument, Synthecell-Vega, PerSeptive, Shimadzu, etc.

### 3. Preparation of anti-VPLF monoclonal antibody

### (1) Preparation of antigen

DNA encoding VPLF is prepared by a method mentioned in 1. and then the expression vector containing the said DNA is introduced by a method mentioned in 2. into *E. coli*, yeast, an insect cell, an animal cell, etc. to prepare a recombinant VPLF protein. Alternatively, VPLF is purified from human established cells, etc. wherein VPLF is expressed. It is also possible to use a synthetic peptide having a partial sequence of VPLF as an antigen.

Such an antigen may be administered as it is or by linked to a carrier protein having a high molecular weight such as keyhole limpet hemocyanin (KLH), bovine serum albumin (BSA), methylated bovine serum albumin (methylated BSA) and bovine thyroglobulin (THY).

### (2) Immunization of animals and preparation of antigen-producing cells

With regard to the animal used for immunization, any animal may be used so far as it is able to prepare a hybridoma, for example, mouse, rat, hamster and rabbit. Hereinafter, examples using mice and rats will be illustrated.

Antigen prepared in the above (1) is immunized to mice or rats of 3 to 20 weeks age and antigen-producing cells are collected from spleen, lymph node and peripheral blood. The immunization is carried out by administration of the antigen for several times together with an appropriate adjuvant to the animal either subcutaneously, intravenously or intraperitoneally. With regard to the adjuvant, there may be exemplified complete Freund's adjuvant, aluminum hydroxide gel and pertussis vaccine. After 3 to 7 days from each administration, blood is collected from venous plexus of fundus oculi or tail vein, then its reactivity to VPLF used as antigen is confirmed by an enzyme immunoassay, etc. [Koso Meneki Sokuteiho (Enzyme Immunoassay) (ELISA method) published by Igaku Shoin (1976)] and mice or rats where their serum show a sufficient antigen value are used as supplying sources for antigen-producing cells. After 3-7 days from the final administration of the antigenic substance, spleen is excised from the immunized mice or rats by a known method [Antibodies - A Laboratory Manual, Cold Spring Harbor Laboratory, 1988; hereinafter, referred to as "Antibodies - A Laboratory Manual"] and used for fusion of spleen cell with myeloma cell which will be conducted later.

### (3) Preparation of myeloma cell

With regard to the myeloma cell, any myeloma cell may be used so far as it is a myeloma cell which is able to be proliferated in vitro such as 8-azaguanine-resistant mouse (derived from BALB/c) myeloma cell line P3-X63Ag8-U1 (P3-U1) which is an established cell obtained from mouse [Euro. J. Immunol., 6, 511 (1976)], SP2/0-Ag14 (SP-2) [Nature, 276, 269 (1978)], Ps-X63-Ag8653(653) [J. Immunol., 123, 1548 (1979)] and P3-X63-Ag8 (X63) [Nature, 256, 495 (1975)]. In cultivation and passage of those cell lines, cell numbers of 2 × 10⁷ or more are ensured until cell fusion according to a known method (Antibodies - A Laboratory Manual).

### (4) Cell fusion

After washing the antibody producing cells and myeloma cells obtained in the above (2) and (3), a cell-aggregating medium such as polyethylene glycol 1000 (PEG-1000) is added so that the cells are fused and suspended in the medium. For washing the cells, there are used MEM medium, PBS (1.83 g of disodium phosphate, 0.21 g of monopotassium phosphate, 7.65 g of salt and 1 liter of distilled water; pH 7.2), etc. With regard to the medium for suspending the fused cells, there is used a HAT medium {a medium where hypoxanthine (10⁻⁴ mol/l), thymidine (1.5 × 10⁻⁵ mol/l) and aminopterin (4 × 10⁻⁷ mol/l) are added to an ordinary medium [a medium where glutamine (1.5 mmol/l), 2-mercaptoethanol (5 × 10⁻⁵ mol/l), gentamicin (10 µg/ml) and fetal bovine serum (FCS) (manufactured by CSL; 10%) are added to an RPMI 1640 medium]} so that only aimed fused cells are selectively obtained.

After the cultivation, a part of the cultured supernatant is taken out and a sample which reacts with antigen protein and does not react with non-antigen protein is selected by way of an enzymatic immunoassay. Thereafter, cloning is carried out by a limiting dilution method and the product where high antibody value is noted in a stable manner by an enzymatic immunoassay is selected as a monoclonal antibody-producing hybridoma strain.

### (5) Selection of hybridoma-producing anti-VPLF monoclonal antibody

Selection of hybridoma which produces anti-VPLF monoclonal antibody is carried out by a measuring method as mentioned below in accordance with a method described in Antibodies - A Laboratory Manual, etc.

### Binding ELISA

Antigen, cells where antigen is expressed, etc. are coated on a 96-well plate and is made to react with the supernatant obtained in cultivation of hybridoma or the purified antibody obtained by the above method (refer to the following (6) for specific procedures) as a first antibody.

After the reaction with the first antibody, the plate is washed and the second antibody is added thereto.

The second antibody is an antibody where an antibody which is able to recognize the immunoglobulin of the first antibody is labeled with biotin, enzyme, chemiluminescent substance, radioactive compound, etc. To be more specific, an antibody which is able to recognize mouse immunoglobulin is used as the second antibody when mouse is used in the preparation of antibody-producing cells in the above (2).

After the reaction, a detection reaction corresponding to the type of the label of the second antibody is carried out and there is selected a hybridoma which produces a monoclonal antibody specifically reacting with antigen.

### (6) Purification of monoclonal antibody

Pristane-treated mouse or nude mouse of 8 to 10 weeks age [0.5 ml of 2,6,10,14-tetramethylpentadecane (pristane) is intraperitoneally administered thereto followed by raising for two weeks] is intraperitoneally administered with 2 × 10⁷ to 5 × 10⁶ cells/mouse of anti-VPLF monoclonal antibody-producing hybridoma cells. The hybridoma becomes ascites cancer within 10 to 21 days. Ascites is collected from the said mouse or nude mouse, centrifuged, salted out with 40 to 50% saturated ammonium sulfate and subjected to a caprylic acid precipitation method and IgG or IgM fraction is recovered using a column such as DEAE-Sepharose column, protein A column or Cellulofine GSL 2000 (Seikagaku Corporation) to give a pure monoclonal antibody.

Determination of a subclass for the pure monoclonal antibody can be carried out using a mouse monoclonal antibody typing kit or a rat monoclonal antibody typing kit. Amount of protein can be calculated by a Lowry method or from the absorbance at 280 nm.

Subclass of antibody is an isotype in a class and, in mouse, it is IgG1, IgG2a, IgG2b and IgG3 and, in human being, it is IgG1, IgG2, IgG3 and IgG4.

### (7) Reaction specificity of anti-VPLF monoclonal antibody

Reaction specificity of the anti-VPLF monoclonal antibody selected in the above (5) is confirmed by a binding ELISA shown in the above (5). In that instance, when VEGF or PDGF is used in addition to VPLF as an antigen for coating the 96-well plate, it is possible to investigate whether the anti-VPLF monoclonal antibody has a specific reactivity to VPLF.

### (8) Measurement of inhibiting activity of anti-VPLF monoclonal antibody to VPLF biological activity

It is possible to check whether the anti-human VPLF monoclonal antibody has a VPLF-inhibiting activity using a proliferation activity of VPLF on rat-derived smooth muscle cells RSMC.

Namely, RSMC is inoculated on a 96-well culture plate in a constant cell concentration, anti-human VPLF monoclonal antibody is added thereto, then VPLF is further added thereto and the mixture is cultured for two days at 37°C in a CO₂ incubator. After completion of the cultivation, a reagent which is colored corresponding to the living cell numbers such as WST-1 reaction reagent (manufactured by Boehringer-Mannheim) is added thereto and absorbance is measured to quantify the living cell numbers. When the fact whether the proliferation of RSMC (increase in living cell numbers) induced by addition of VPLF is inhibited by anti-VPLF monoclonal antibody is checked, it is possible to judge the presence or absence of inhibiting activity of the anti-VPLF monoclonal antibody.

### 4. Method for the immunological detection of VPLF using the antibody of the present invention

When an antigen-antibody reaction is carried out using the antibody of the present invention, antibody fragment thereof or derivatives thereof, it is possible to immunologically detect VPLF or the tissue containing VPLF. The detection method can be utilized for diagnosis of diseases associated with VPLF or diseases caused by mutation of gene encoding VPLF such as diseases associated with abnormal stimulation of angiogenesis, eye diseases based on abnormal angiogenesis, arthritis based on abnormal angiogenesis, skin diseases associated with abnormal angiogenesis, diseases associated with abnormal stimulation of vascular permeability, diseases associated with abnormal differentiation and proliferation of smooth muscle cells, diseases associated with abnormal differentiation and proliferation of kidney mesangial cells, diseases associated with abnormal differentiation and proliferation of blood stem cells, diseases based on abnormality in osteoblasts, diseases based on abnormality in pancreatic β cells, ischemic diseases and diseases associated with the delay of wound healing. The detection is also able to be used for quantitative determination of VPLF.

Examples of the immunological detection method used are fluorescent antibody technique, enzyme-linked immunosorbent assay (ELISA), radio immunoas say (RIA), immunohistochemistry , immunocytochemistry, Western blotting, immunoprecipitation , enzyme immunoassay and sandwich ELISA [Tankuron Kotai Jikken Manyuaru (Manual for Experiments on Monoclonal Antibody) (published by Kodansha Scientific, 1987); Zoku Seikagaku Jikken Koza 5, Meneki Seikagaku Kenkyuho (Biochemical Experiments, Supplementary Issue No. 5, Methods for Immunobiochemical Studies) (published by Tokyo Kagaku Dojin, 1986)].

Fluorescent antibody technique can be carried out using a method described, for example, in the literatures [Monoclonal Antibodies: Principles and Practice, Third edition (Academic Press, 1996); Tankuron Kotai Jikken Manyuaru (Manual for Experiments on Monoclonal Antibody) (published by Kodansha Scientific, 1987)]. To be more specific, it is a method where the antibody of the present invention is made to react with the separate cell or tissue and then made to react with anti-immunoglobulin antibody or bound fragment labeled with a fluorescent substance such as fluorescein isothiocyanate (FITC) or phycoerythrin and the fluorescent dye is measured by a flow cytometer.

Enzyme-linked immunosorbent assay (ELISA) is a method where antigen or cells where antigen is expressed is made to react with the antibody or bound fragment of the present invention and then made to react with anti-immunoglobulin antibody labeled with enzyme such as peroxidase, labeled with biotin, etc. and the colored dye is measured by a spectrophotometer.

Radioimmunoassay (RIA) is a method where antigen or cell where antigen is expressed is made to react with the antibody or bound fragment of the present invention and then made to react with radio-labeled anti-immunoglobulin antibody and measurement is carried out by a scintillation counter or the like.

Immunohistochemistry and immunocytochemistry are the methods where antigen or cell where antigen is expressed is made to react with the antibody of the present invention, then further made to react with an anti-immunoglobulin antibody or bound fragment labeled with a fluorescent substance such as fluorescein isothiocyanate (FITC), enzyme such as peroxidase, biotin, etc. and an observation is conducted under a microscope and it can be carried out using a method described in the literatures [Monoclonal Antibodies: Principles and Practice, Third edition (Academic Press, 1996); Tankuron Kotai Jikken Manyuaru (Manual for Experiments on Monoclonal Antibody) (published by Kodansha Scientific, 1987)], etc.

Western blotting is a method where cell in which antigen or antibody is expressed, etc. is fractionated by an SDS-polyacrylamide gel electrophoresis [Antibodies - A Laboratory Manual, Cold Spring Harbor Laboratory, 1988], the gel is subjected to a blotting to PVDF membrane or nitrocellulose membrane, the monoclonal antibody or the antibody fragment thereof is made to react with the said membrane and further made to react with anti-mouse IgG antibody or bound fragment labeled with a fluorescent substance such as FITC, enzyme such as peroxidase or with biotin and then the said label is made visible whereupon confirmation is done.

Immmunoprecipitation is a method where the antigen or the cell in which the antigen is expressed is made to react with the monoclonal antibody of the present invention or antibody fragment thereof and then a carrier having a specific binding ability to immunoglobulin such as protein G-Sepharose whereupon an antigen-antibody complex is precipitated.

Sandwich ELISA is an ELISA where concentration of antigen is quantified using two kinds of antibodies to antigen. In the ELISA, two kinds of monoclonal antibodies which are the monoclonal antibodies of the present invention or antibody fragments thereof where their antigen-recognizing sites are different are prepared and one of the monoclonal antibodies or antibody fragments is previously adsorbed with a plate (such as a 96-well plate) while another monoclonal antibody or antibody fragment is labeled with fluorescent substance such as FITC, enzyme such as peroxidase, biotin, etc. The above-mentioned antibody-adsorbed plate is made to react with cell or disintegrated liquid thereof, tissue or disintegrated liquid thereof, cultured supernatant of cells, serum, pleural effusion, ascites, eye liquid, etc. and then made to react with labeled monoclonal antibody or antibody fragment thereof and a detection reaction corresponding to the labeled substance is carried out. It is possible to calculate the concentration of the sample to be tested from a calibration curve prepared by a stepwise dilution of the VPLF protein having known concentration.

Diagnosis can be carried out as follows. Thus, quantitative determination of VPLF is carried out for a biological sample such as tissue, blood, serum, pleural effusion, ascites, eye liquid, etc. collected from a plurality of healthy living body by the above-mentioned immunological detection method using the antibody of the present invention, antibody fragment thereof or derivative thereof so that the expression level of VPLF in the biological sample from healthy persons is previously checked. Quantitative determination for VPLF is also carried out for the biological sample of the person to be tested and the expression level thereof is compared with the expression level of healthy persons. In the diseases where an increase in VPLF is noted such as diseases associated with abnormal stimulation of angiogenesis, eye diseases based on abnormal angiogenesis, arthritis based on abnormal angiogenesis, skin diseases associated with abnormal angiogenesis, diseases associated with abnormal acceleration of vascular permeability, diseases associated with abnormal differentiation and proliferation of smooth muscle cells and diseases associated with abnormal differentiation and proliferation of kidney mesangial cells, it is diagnosed to be positive when the expression level of the person to be tested is increased as compared with that of the healthy persons. In the diseases where a decrease in VPLF is noted such as diseases associated with abnormal differentiation and proliferation of blood stem cells, diseases based on abnormality in osteoblasts, diseases based on abnormality in pancreatic β cells, ischemic diseases and diseases associated with the delay of wound healing, it is diagnosed to be positive when the expression level of the person to be tested is decreased as compared with that of the healthy persons. With regard to the biological sample used for the diagnosis, it is preferred to use the tissue associated to the disease or the body fluid, etc. which is derived from the tissue for each of the diseases.

A diagnostic agent comprising the antibody of the present invention, antibody fragment thereof or derivative thereof may further contain a reagent for conducting an antigen-antibody reaction and a reagent for detecting the reaction depending upon the aimed diagnostic method. With regard to the reagent for conducting the antigen-antibody reaction, there may be exemplified buffer and salt. With regard to the reagent for the detection, there may be exemplified the antibody of the present invention, antibody fragment thereof or derivative thereof, labeled secondary antibody recognizing the antibody of the present invention, antibody fragment thereof or derivative thereof and reagent used for usual immunoassay such as substrate corresponding to the label.

### 5. Pharmaceutical agent comprising the antibody of the present invention

VPLF such as the VPLF of SEQ ID NO: 1 shows a high homology to VEGF while VEGF has been reported to progress and worsen diseases associated with abnormal stimulation of angiogenesis such as solid tumor and tumor metastasis, diseases of eye based on abnormal angiogenesis such as diabetic retinopathy, retinopathy of prematurity, age-related macular degeneration and neovascular glaucoma, arthritis based on abnormal angiogenesis such as rheumatoid arthritis, skin diseases associated with abnormal angiogenesis such as psoriasis and diseases associated with abnormal vascular permeability such as ascites cancer, cancer with pleural effusion, Crow-Fukase syndrome and ovarian hyperstimulation syndrome and VEGF antibody has been reported to be useful for the treatment of such diseases whereby the antibody of the present invention, antibody fragment thereof or derivative thereof, particularly, the antibody to VPLF of SEQ ID NO: 1, antibody fragment thereof or derivative thereof is able to be a therapeutic agent for diseases associated with abnormal stimulation of angiogenesis such as solid tumor and tumor metastasis, diseases of eye based on abnormal angiogenesis such as diabetic retinopathy, retinopathy of prematurity, age-related macular degeneration and neovascular glaucoma, arthritis based on abnormal angiogenesis such as rheumatoid arthritis, skin diseases associated with abnormal angiogenesis such as psoriasis and diseases associated with abnormal vascular permeability such as ascites cancer, cancer with pleural effusion, Crow-Fukase syndrome and ovarian hyperstimulation syndrome.

Further, VPLF such as the VPLF of SEQ ID NO: 1 shows a high homology to PDGF while PDGF has been reported to progress and worsen diseases associated with abnormal differentiation and proliferation of smooth muscle cells such as arterosclerosis and diseases associated with abnormal differentiation and proliferation of kidney mesangial cells such as glomerulonephritis and PDGF antibody has been reported to be useful for the treatment of such diseases whereby the antibody of the present invention, antibody fragment thereof or derivative thereof, particularly, the antibody to VPLF of SEQ ID NO: 1, antibody fragment thereof or derivative thereof is able to be a therapeutic agent for diseases associated with abnormal differentiation and proliferation of smooth muscle cells such as arterosclerosis and diseases associated with abnormal differentiation and proliferation of kidney mesangial cells such as glomerulonephritis.

The therapeutic agent comprising the antibody of the present invention, antibody fragment thereof or derivative thereof may be that which contains only the said antibody, antibody fragment thereof or derivative thereof as an effective ingredient but, usually, it is preferred to be provided as a pharmaceutical agent preparation produced according to any method which is well known in the technical field of pharmaceutical preparations by mixing with one or more pharmacologically acceptable carrier(s).

With regard to the administration route, it is preferred to select the most effective one for the treatment and there may be exemplified oral administration and parenteral administration such as intraoral, intra-airway, intrarectal, subcutaneous, intramuscular, intravenous, etc. and, in the case of antibody or a peptide preparation, intravenous administration may be exemplified as a preferred one.

With regard to the dosage form, there may be exemplified air spray, capsule, tablet, granule, syrup, emulsion, suppository, injection, ointment and tape.

Examples of the preparation suitable for oral administration are emulsion, syrup, capsule, tablet, diluted powder and granule.

Liquid preparation such as emulsion and syrup may be produced using water, saccharide such as sucrose, sorbitol and fructose, glycol such as polyethylene glycol and propylene glycol, oil such as sesame oil, olive oil and soybean oil, antiseptic such as p-hydroxybenzoate, flavor such as strawberry flavor and peppermint, etc. as an additive.

Capsule, tablet, diluted powder, granule, etc. may be produced using an excipient such as lactose, glucose, sucrose and mannitol, a disintegrating agent such as starch and sodium alginate, a lubricant such as magnesium stearate and talc, a binder such as polyvinyl alcohol, hydroxypropyl cellulose and gelatin, a surfactant such as fatty acid ester, a plasticizer such as glycerol, etc. as an additive.

Examples of a preparation suitable for parenteral administration are injection, suppository and air spray.

Injection is prepared using a carrier, etc. comprising a salt solution, a glucose solution or a mixture thereof.

Suppository is prepared using a carrier such as cacao butter, hydrogenated fat, carboxylic acid, etc.

Air spray is prepared using the said antibody or peptide itself or a carrier or the like which does not irritate oral cavity and airway of the person to be treated and disperses the said compound into fine particles so as to make absorption easy.

Specific examples of the carrier are lactose and glycerol. Preparations such as aerosol and dry powder are possible depending upon the properties of the said antibody and the carrier used. Such parenteral agent may also be added with the component which is exemplified as an additive for oral preparations.

Dose and administering frequency vary depending upon the aimed therapeutic effect, administering method, period for the therapy, age, body weight, etc. and, usually, it is from 10 µg/kg to 8 mg/kg per day for adults.

### Brief Description of the Drawings

Fig. 1 is a drawing which shows the comparison of amino acid sequences of VPLF and VEGF/PDGF family.
Fig. 2 is a drawing which shows the relation between VPLF and EST clones.
Fig. 3 is a drawing which shows a hydrophobicity plot of VPLF.
Fig. 4 shows the result of analysis of expression of factors belonging to a VEGF/PDGF superfamily in human tissues by an RT-PCR.
Fig. 5 shows the result of analysis of expression of factors belonging to a VEGF/PDGF superfamily in cancer cells by an RT-PCR.
Fig. 6 shows a process of construction of plasmid pVL-VPLF.
Fig. 7 shows a process of construction of plasmid pVL-VPLFΔN.
Fig. 8 shows the result of SDS-PAGE of purified VPLFΔN under reducing and non-reducing conditions.
Fig. 9 shows a process of construction of plasmid pIRES-VPLF.
Fig. 10 shows a process of construction of plasmids pAGE248-VPLF and pAGE210-VPLF.
Fig. 11 shows the result of expression of VPLF using animal cells (PC-9, CHO) as a host.
Fig. 12 shows the result of investigating the action of VPLFΔN to human CD34 positive cells.
Fig. 13 shows the result of investigating the growth-promoting activity of VPLFΔN, VEGF165, PDGF BB and IL-5 for rat smooth muscle cells RSMC.
Fig. 14 shows the result of investigating the growth-promoting activity of VPLFΔN, VEGF165, PDGF BB and IL-5 for human skin-derived microvascular endothelial cells HMVEC.
Fig. 15 shows the result of analysis of the reaction specificity of anti-human VPLF monoclonal antibody KM 2676.
Fig. 16 shows the result of detection of human VPLF by Western blotting.
Fig. 17 is a drawing which shows the reaction specificity of anti-human VPLF monoclonal antibodies.
Fig. 18 is a drawing which shows the cross reactivity of anti-VPLF monoclonal antibodies to VEGF and PDGF.
Fig. 19 is a drawing which shows inhibition of growth-promoting activity of VPLF for rat smooth muscle cells (RSMC) by anti-human VPLF monoclonal antibodies.
Fig. 20 is a drawing which shows quantification of human VPLF by a sandwich ELISA system using anti-human VPLF monoclonal antibody.

### Best Mode for Carrying Out the Invention

As hereunder, the present invention will be specifically illustrated byway of Examples and Reference Examples. However, those Examples and Reference Examples are just for illustration and do not limit the technical scope of the present invention.

### [Reference Example 1] Construction of cDNA libraries from human neural precursor cell NT-2 and human ovarian cancer tissue

There were used NT-2 neural precursor cells (purchased from Stratagene) which were teratocarcinoma cells derived from human fetal testis and able to be differentiated to neural cells by a treatment with retinoic acid. NT-2 cells were cultured according to the manual attached thereto, and retinoic acid was added thereto followed by culturing for two weeks more. The cultured cells were collected and mRNA was extracted therefrom according to the method mentioned in the literature Molecular Cloning, Second Edition. Further, polyA(+) RNA was purified by oligo dT cellulose.

Similarly was extracted mRNA from human ovarian cancer tissue according to the method mentioned in the literature (J. Sambrook, E. F. Fritsch & T. Maniatis, Molecular Cloning, Second Edition, Cold Spring Harbor Laboratory Press, 1989). Further, polyA(+) RNA was purified by oligo dT cellulose.

From each polyA(+) RNA was constructed a CDNA library by an oligo-cap method [M. Maruyama and S. Sugano, Gene, 138, 171-174 (1994)]. According to a method mentioned in the literatures [Suzuki and Sugano, Tanpakushitsu Kakusan Koso, 41, 197-201 (1996); Y. Suzuki, et al., Gene, 200, 149-156 (1997)] and using a oligo-cap linker (SEQ ID NO: 3) and a oligo dT primer (SEQ ID NO: 4), polyA(+) RNA was subjected to BAP (bacterial alkaline phosphatase) treatment, TAP (tobacco acid phosphatase) treatment, RNA ligation, synthesis of the cDNA first strand and RNA removal. Thereafter, conversion to double-stranded cDNA was carried out by PCR (polymerase chain reaction) using two kinds of PCRprimers - a sense primer (SEQ ID NO: 5) for 5'-terminal and an antisense primer (SEQ ID NO: 6) for 3'-terminal - and cleaving by SfiI was conducted. The PCR was carried out by heating at 95°C for 5 minutes and then repeating a reaction cycle of 95°C for 1 minute, 58°C for 1 minute and 72°C for 10 minutes for 12 times followed by keeping at 4°C using a commercially available kit (GeneAmp XL PCR kit manufactured by Perkin-Elmer). Thereafter, the product was cloned to a vector pME18SFL (GenBank AB009864, an expression vector, 3392bp) cleaved with DraIII by determining the direction of cDNA to construct a cDNA library. With regard to the cloned plasmid DNA prepared as such, nucleotide sequences at 5'-terminal and 3'-terminal of cDNAwere determined using a DNA sequencer (ABI PRISM 377; manufactured by PE Biosystems) after performing a sequencing reaction according to themanual using DNA sequencing reagents (Dye Terminator Cycle Sequencing FS Ready Reaction Kit, dRhodamine Terminator Cycle Sequencing FS Ready Reaction Kit or Big Dye Terminator Cycle Sequencing FS Ready Reaction Kit; manufactured by PE Biosystems).

### [Reference Example 2] Identification of a novel growth factor belonging to a VEGF/PDGF superfamily

With regard to an amino acid sequence of each frame which a nucleotide sequence of each clone of the constructed CDNA libraries is able to encode, a homology analysis was carried out to amino acid sequences of 7 molecules of known proteins belonging to a VEGF/PDGF superfamily, which are registered in a protein amino acid sequence database SWISSPROT or a nucleotide sequence database GenBank,- human VEGF (SWISSPROT accession No. P15692), human VEGF-B (SWISSPROT accession No. P49765), human VEGF-C SWISSPROT accession No. P49767), human VEGF-D (GenBank accession No. AJ000185), human PlGF (SWISSPROT accession No. P49763), human PDGF-A (SWISSPROT accession No. P04085) and human PDGF-B (SWISSPROT accession No. P01127), then cDNA clone encoding an amino acid sequence having a homology to the amino acid sequences of those molecules was selected and the protein encoded by such a clone was defined as VPLF. SEQ ID NO: 1 and SEQ ID NO: 2 show an amino acid sequence of VPLF and a nucleotide sequence thereof, respectively.

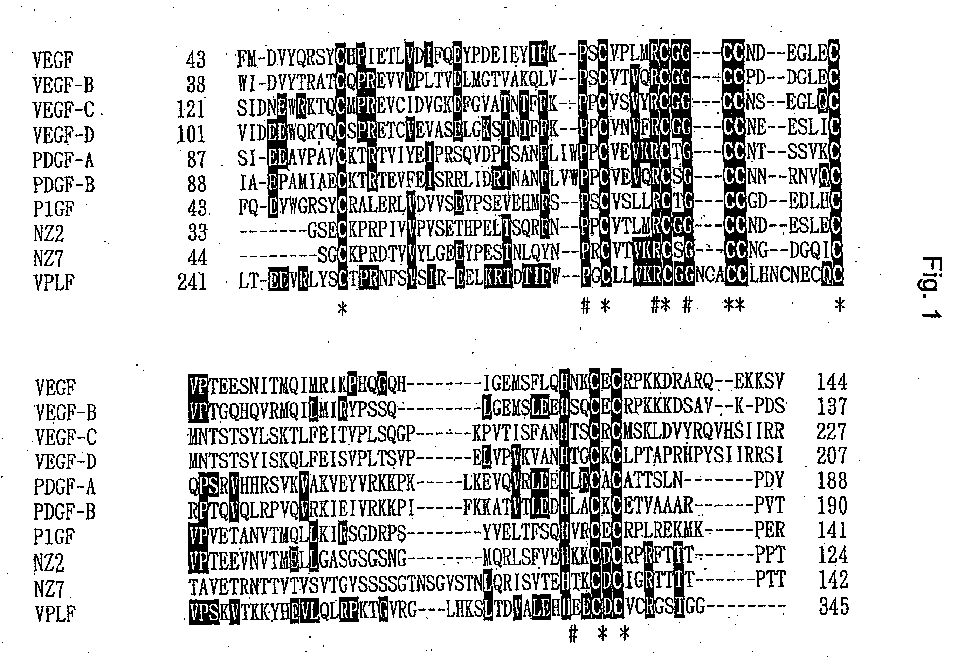

In a homology analysis using BLAST2, VPLF showed significant homologies of 29% at P value of 0.0022, 29% at P value of 0.0014, 25% at P value of 0.00022, 29% at P value of 1.8 × 10⁻⁷, 36% at P value of 0.0016 and 28% at P value of 0.00059 to human VEGF, human VEGF-B, human VEGF-C and human VEGF-D which are proteins belonging to a vascular epithelial growth factor family and human PDGF-A and human PDGF-B which are proteins belonging to a platelet-derived growth factor family, respectively. It also showed significant homologies of 29% at P value of 4.7 × 10⁻⁵ and 30% at P value of 7.7 × 10⁻⁷ to NZ2-VEGF (SWISSPROT accession No. P52584) and NZ7-VEGF (SWISSPROT accession No. P52585) belonging to the same VEGF/PDGF superfamily, respectively. To human PlGF which is a protein belonging to a vascular epithelial growth factor family, it shows a homology of 26% at P value of 0.94. In a VEGF/PDGF superfamily, it has been known that there are eight cysteine residues which are important for disulfide bond formation in a dimer, disulfide bond formation in a protein molecule and expression of activity (J. Biol. Chem., 269, 32879-32885, 1994) and that those eight cysteine residues are conserved among the factors belonging to a VEGF/PDGF superfamily. When an amino acid sequence of VPLF was compared with the known VEGF/PDGF superfamily molecules using an alignment program CLUSTALW (Nucleic Acids Research, 22, 4673-4680, 1994), it was found that positions and numbers essential for the formation of said motif are completely conserved even in the amino acid sequence represented by SEQ ID NO: 1 (Fig. 1). Accordingly, it is apparent that VPLF has an activity as a growth factor belonging to a VEGF/PDGF superfamily. Incidentally, VEGF, VEGF-B, VEGF-C, VEGF-D, PDGF-A, PDGF-B, PlGF, NZ2 and NZ7 in Fig. 1 show human VEGF, human VEGF-B, human VEGF-C, human VEGF-D, human PDGF-A, human PDGF-B, human PlGF, NZ2-VEGF and NZ7-VEGF, respectively. Sequences conserved between VPLF and family molecules are shown with outline characters, cysteine residues conserved in all family molecules are marked with * and other amino acid residues are marked with #.

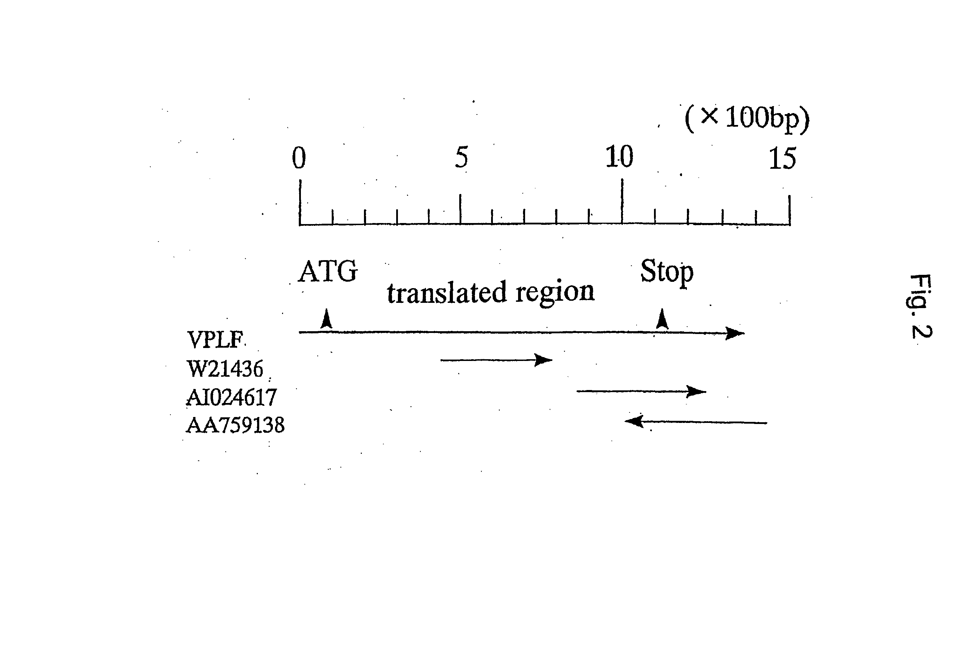

When the nucleotide sequence database GenBank/EMBL/DDBJ was searched using BLAST2 using a nucleotide sequence represented by SEQ ID NO: 2 as a query, it was found to be identical with three nucleotide sequences which are considered to be ESTs derived from the same gene (as of February 5, 1999). GenBank accession Nos. of those ESTs are W21436, AI024617 and AA759138 and the relation with VPLF nucleotide sequence is shown in Fig. 2. Those EST nucleotide sequences do not cover the full length of VPLF. Although a gene which shows a homology to a nucleotide sequence of each EST was searched from each of the databases GenBank, EMBL and DDBJ using BLAST2, a gene showing a significant homology to any EST was not selected. Accordingly, VPLF has been found to be a novel gene which was obtained for the first time by the present invention.

*Escherichia coli* DH10B/NT2RP4000328 which contains plasmid NT2RP4000328 comprising a cDNA encoding VPLF and *Escherichia coli* DH10B/OVARC1001401 which contains plasmid OVARC1001401 comprising its partial sequence CDNA (from the 576th base to the 1328th base in SEQ ID NO: 2) have been deposited as FERM BP-6686 and FERM BP-6687, respectively, on April 1, 1999 at the International Patent Organism Depositary, National Institute of Advanced Industrial Science and Technology (Central 6, 1-1, Higashi1-chome, Tsukuba-shi, Ibaraki, Japan; postoffice code: 305-8566).

### [Reference Example 3] Analysis of nucleotide sequence of VPLF

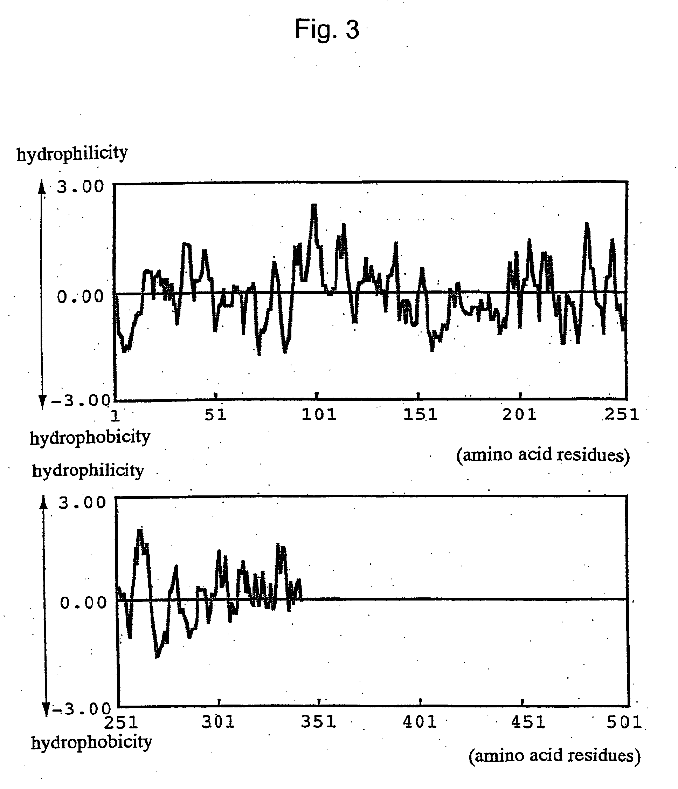

On the basis of the nucleotide sequence of VPLF represented by SEQ ID NO: 2, a sequence around the initiation codon was analyzed using ATGPr which is an initiation codon prediction program for protein (Bioinformatics, 14, 384-390, 1998). ATG at the 94th to the 96th positions from the 5'-terminal and TAG at the 1129th to the 1131st positions were specified as an initiation codon and a termination codon, respectively and the protein encoded by ORF was presumed to be consisting of 345 amino acids. Further, on the basis of an amino acid sequence of VPLF represented by SEQ ID NO: 1, analysis was carried out whether VPLF has the characteristic as secretory proteinusing PSORT which is a program for prediction of localization site of the protein in cells (Genomics, 14, 897-911, 1992). An amino acid sequence of 14 residues at the N-terminal of VPLF had the characteristic of a signal peptide sequence noted in secretory protein and VPLF was classified under secretory protein. Result of preparation of a hydrophobicity plot used GENETYX-MAC7.3 (manufactured by Software Development Co., Ltd.) is shown in Fig. 3. In the N-terminal part of VPLF, a region showing a high hydrophobicity which is characteristic to secretory protein is present.

### [Reference Example 4] Organs in which VPLF is expressed

The three ESTs - W21436, AI024617 and AA759138 - which were found to be identical with a part of the nucleotide sequence of VPLF had been isolated from lung, testis and testis, respectively. When the nucleotide sequence databases - GenBank, EMBL and DDBJ - were searched using BLAST2 on the basis of a nucleotide sequence of 3'-terminal untranslated region of cDNA clone encoding VPLF, they were found to be identical with 16 nucleotide sequences which were considered to be ESTs derived from the same gene (on February 5, 1999). Accession Nos. at GenBank for those ESTs are AA631149, AA039965, AA039880, AI128937, N89807, AA613059, AA868252, C02066, AI051824, AA594888, N66753, AI193332, AI243165, AI262908, AI284795 and N22076 and have been registered as UniGene Hs. 43080. Those 16 ESTs had been isolated from colon, ear, lung, kidney, ovary, thyroid epithelium, prostate gland, testis and uterus. Accordingly, VPLF was found to be expressed in colon, ear, lung, kidney, ovary, thyroid epithelium, prostate gland, testis and uterus.

### [Reference Example 5] Analysis of expression of DNA encoding VPLF using RT-PCR

cDNA was synthesized using 4 µg of human organ polyA⁺ RNAs purchased from Clontech and total RNAs (4 µg) prepared from cancer cell lines by an AGPC method [Analytical Biochemistry, 162, 156 (1987); Jikken Igaku, 9, 1937 (1991)] as templates and commercially-available SUPER SCRIPT Preamplification System for First Strand cDNA Synthesis (manufactured by Gibco BRL) according to the manual attached thereto.

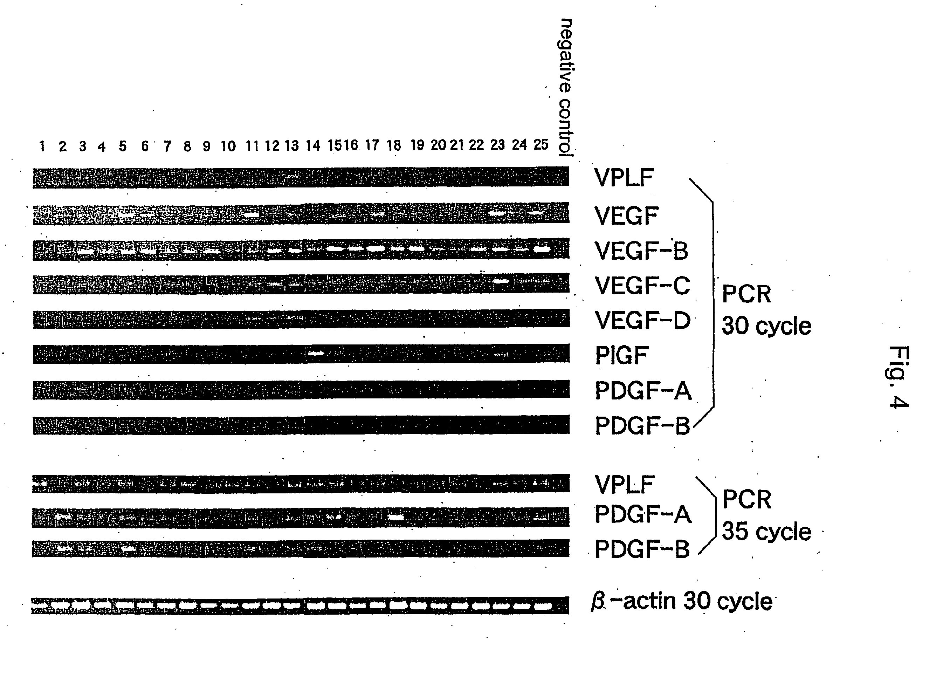

With regard to human organ polyA⁺ RNAs, those derived from adrenal gland, brain, cerebellum, pituitary gland, kidney, small intestine, bone marrow, heart, liver, lung, lymph node, mammary gland, placenta, prostate, salivary gland, skeletal muscle, spinal cord, spleen, stomach, testis, thymus, thyroid, trachea and uterus were used (In Fig 4, they are abbreviated as 1, 2, 3, 4, 5, 6, 7, 8, 9, 10, 11, 12, 13, 14, 15, 16, 17, 18, 19, 20, 21, 22, 23, 24 and 25, respectively).

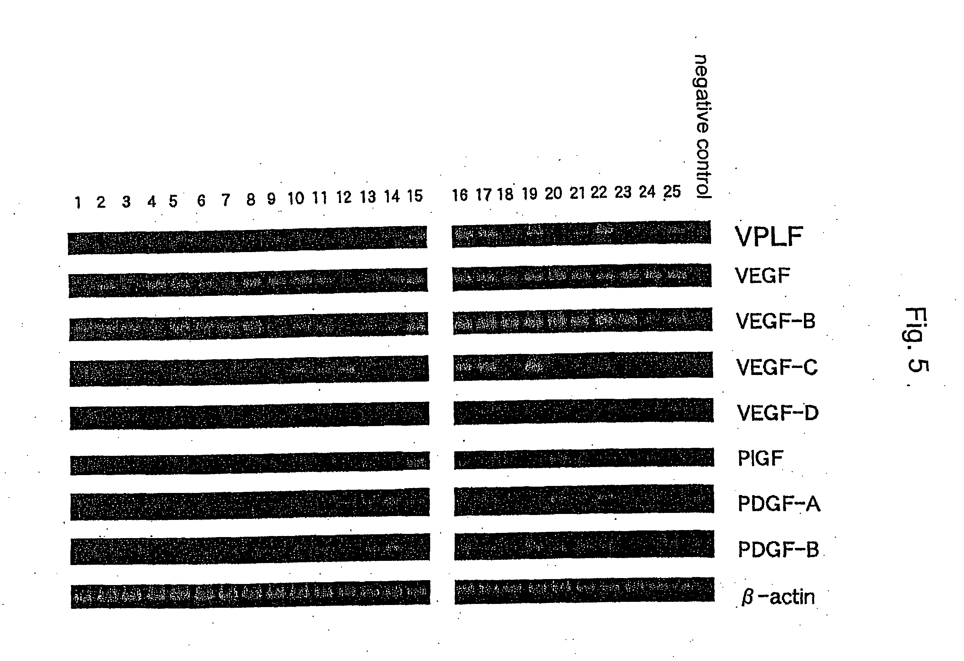

With regard to cancer cell lines, there were used T cell lines (Jurkat, Molt-3, Molt-4 and HUT78; abbreviated as 1, 2, 3 and 4, respectively, in Fig. 5), B cell lines (Namalwa KJM-1, Daudi and Raji; abbreviated as 5, 6 and 7, respectively in Fig. 5), granulocytic/monocytic cell lines (HL-60, U-937 and THP-1; abbreviated as 8, 9 and 10, respectively in Fig. 5), vascular endothelial cell lines (IVEC and HUVEC; abbreviated as 11 and 12, respectively in Fig. 5), melanoma cell lines (WM266-4 and WM115; abbreviated as 13 and 14, respectively in Fig. 5), a neuroblastoma cell line (SK-N-MC; abbreviated as 15 in Fig. 5), lung cancer cell lines (PC-9, HLC-1 and QG90; abbreviated as 16, 17 and 18, respectively in Fig. 5), a prostate cancer cell line (PC-3; abbreviated as 19 in Fig. 5), a gastric cancer cell line (KATO III; abbreviated as 20 in Fig. 5), pancreatic cancer cell lines (Capan-1 and Capan-2; abbreviated as 21 and 22, respectively in Fig. 5) and colorectal cancer cell lines (Colo205, SW1116 and LS180; abbreviated as 23, 24 and 25, respectively in Fig. 5). Jurkat, QG90 and SW1116 were obtained from the Aichi Cancer Center. HLC-1 was obtained from the Cancer Institute, Osaka University. KATO III and PC-9 were obtained from IBL. HUVEC (human umbilical vascular endothelia cell) was obtained from Kurabo. IVEC [J. Cell. Physiol., 157, 41 (1993)] was obtained from N. T. L. France. Molt-4 and Daudi were obtained from a cell bank of the Japanese Collection of Research Bioresources (JCRB) [its internet address is http://cellbank.nihs.go.jp/]. Cells other than the above were obtained from the American Type Culture Collection.

Thereafter, PCR was carried out using the synthesized cDNA as a template. Thus, a reaction solution [10 mmol/l of Tris-HCl (pH 8.3), 50 mmol/l of KCl, 1.5 mmol/l of MgCl₂, 0.2 mmol/l of dNTP, 0.001% (w/v) of gelatin, 0.2 µmol/l of gene-specific primers and 1 unit of recombinant Taq polymerase (manufactured by Takara)] was prepared using a synthesized cDNA solution diluted 50-fold with sterilized water as a component and PCR was carried out using primers having a nucleotide sequence specific to human VPLF, human VEGF, human VEGF-B, human VEGF-C, human VEGF-D, human PDGF-A, human PDGF-B, human PlGF or human β actin under a condition wherein the solution is reacted at 94°C for 3 minutes, then a cycle of 94°C for 30 seconds, 65°C for 1 minute and 72°C for 2 minutes was repeated for 30 or 35 cycles, finally reacted at 72°C for 7 minutes and stored at 4°C for one night.

In the PCR, there were used the oligonucleotides represented by SEQ ID NO: 7 and SEQ ID NO: 8 as primers specific to human VPLF, the oligonucleotides represented by SEQ ID NO: 9 and SEQ ID NO: 10 as primers specific to human VEGF, the oligonucleotides represented by SEQ ID NO: 11 and SEQ ID NO: 12 as primers specific to human VEGF-B, the oligonucleotides represented by SEQ ID NO: 13 and SEQ ID NO: 14 as primers specific to human VEGF-C, the oligonucleotides represented by SEQ ID NO: 15 and SEQ ID NO: 16 as primers specific to human VEGF-D, the oligonucleotides represented by SEQ ID NO: 17 and SEQ ID NO: 18 as primers specific to human PlGF, the oligonucleotides represented by SEQ ID NO: 19 and SEQ ID NO: 20 as primers specific to human PDGF-A, the oligonucleotides represented by SEQ ID NO: 21 and SEQ ID NO: 22 as primers specific to human PDGF-B and the oligonucleotides represented by SEQ ID NO: 23 and SEQ ID NO: 24 as primers specific to human β-actin.

When the PCR solution was analyzed by an agarose gel electrophoresis, bands of DNA specific to each of the primers were observed and their sizes were about 1,000 bp, 350 bp, 300 bp, 520 bp, 500 bp, 420 bp, 430 bp, 360 bp and 800 bp for human VPLF, human VEGF, human VEGF-B, human VEGF-C, human VEGF-D, human PDGF-A, human PDGF-B, human PlGF and human β actin, respectively.

Expression levels of the factors were compared semi-quantitatively by comparison of intensities of those bands. The result when cDNAs prepared from human organs were used as templates is shown in Fig. 4 while the result when cDNAs prepared from cancer cell lines were used as templates is shown in Fig. 5. It was noted from Fig. 4 and Fig. 5 that the expression level of VPLF was higher in cancerous cells than in normal tissues, and that the expression level was high in endothelial cells and low in blood cells among the cancer cell lines .

### [Reference Example 6] Expression of full-length VPLF and VPLFΔN (an N-terminal-deleted mutant wherein the 1st to the 226th amino acids in SEQ ID NO: 1 are deleted) using insect cells as a host

For the production of a recombinant protein by insect cells, it is necessary to prepare a recombinant virus where aimed DNA is integrated and, for the preparation as such, there are included (1) a step where a special vector (transfer vector) comprising cDNA encoding an aimed protein is constructed, (2) a step where baculovirus DNA and transfer vector are co-transfected to insect cells to prepare a recombinant virus by a homologous recombination followed by proliferating the recombinant virus and (3) a step where the recombinant virus is infected to the cells to express the aimed protein. To be more specific, the recombinant virus was prepared to produce the aimed protein as shown below.

### (1) Construction of transfer vector

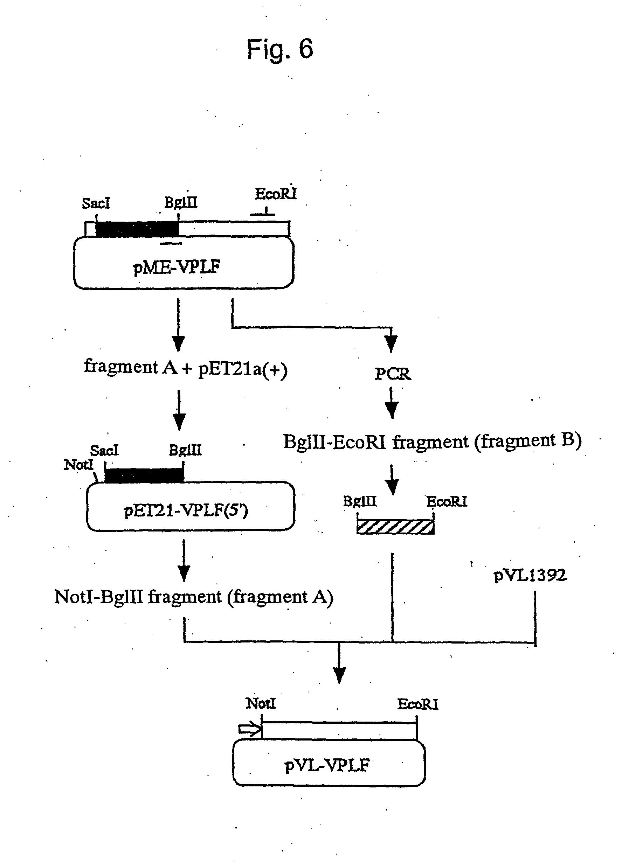

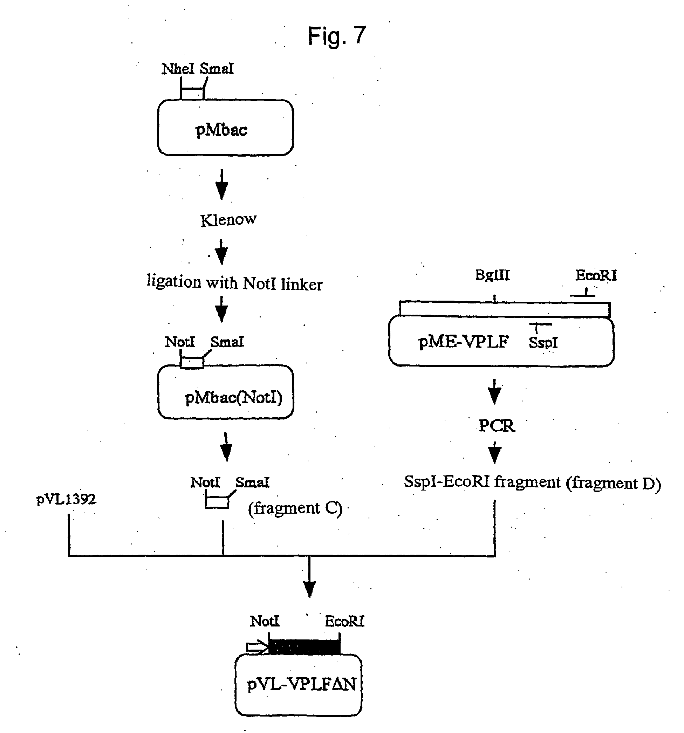

(i) pVL-VPLF: The pVL-VPLF which is a vector carrying the DNA encoding the full length of VPLF (an amino acid sequence from the 1st to the 345th amino acids of SEQ ID NO: 1) was constructed as follows (Fig. 6).
   SacI-BglII fragment (0.66 kb) of the plasmid NT2RP4000328 (hereinafter, referred to as "pME-VPLF") mentioned in Reference Example 2 was inserted into a SacI-BglII site of vector pET21a(+) (manufactured by Novagen) to construct a plasmid pET21-VPLE(5'). This plasmid was cleaved with NotI-BglII to prepare a fragment of 0.68 kb (fragment A).
   PCR was carried out using pME-VPLF as a template and using the DNA represented by SEQ ID NO: 25 and SEQ ID NO: 26 as primers. The amplified DNA fragment was cleaved with BglII and EcoRI to prepare a fragment of 0.4 kb (fragment B).
   The fragment A and the fragment B were inserted into a NotI-EcoRI site of an insect cell transfer vector pVL1392 (manufactured by Pharmingen) to construct pVL-VPLF. This plasmid comprised a DNA encoding from the 1st Met to the 345th Gly of VPLF.
(ii) pVL-VPLFΔN: The pVL-VPLFΔN which is a vector comprising a DNA encoding the partial C-terminal peptide of VPLF (an amino acid sequence from the 227th to the 345th amino acids of SEQ ID NO: 1) was constructed as follows (Fig. 7).

A plasmid pMbac (manufactured by Stratagene) carrying a signal peptide DNA of melittin which is a secretory protein derived from insect was cleaved with NheI, treated with Klenow fragment and linked with NotI linker (5'-GCGGCCGC-3') to construct a plasmid pMbac(Not I). This plasmid was cleaved with NotI and SmaI to prepare a fragment of 85 bp (fragment C).

PCR was carried out using pME-VPLF as a template and using DNAs represented by SEQ ID NO: 26 and NO: 27 as primers. The amplified fragment was cleaved with SspI and ECORI to prepare a fragment of 0.36 kb (fragment D).

The fragment C and the fragment D were inserted into a NotI-EcoRI site of a vector pVL1392 to construct pVL-VPLFAN. This plasmid comprised a DNA encoding a signal peptide derived frommelittin, a peptide (Asp-Pro-Ser-Pro; SEQ ID NO: 34) derived from melittin and from the 227th Phe to the 345th Gly of VPLF.

### (2) Preparation of recombinant virus

Insect cells Sf9 (manufactured by Iwaki Glass) cultured in an ESF 921 medium (manufactured by Protein Expression) were transfected with a linear baculovirus DNA [Baculo Gold Baculovirus DNA; manufactured by Pharmingen] and the transfer vector constructed in the above (1) by a lipofectin method [Tanpakushitsu Kakusan Koso, 37, 2701 (1992)] to prepare a recombinant baculovirus. To be more specific, that was carried out as follows.

Four µg of pVL-VPLF or pVL-VPLFΔN and 15 ng of linear baculovirus DNA were dissolved in 12 ml of distilled water, then a mixture of 6 ml of lipofectin and 6 ml of sterilized distilled water was added thereto and the mixture was allowed to stand at room temperature for 15 minutes. In the meanwhile, 1 × 10⁶ of Sf9 cells were suspended in 2 ml of an ESF 921 medium and placed on a plastic dish for cell culture having a diameter of 50 mm. To this was added whole amount of the above-mentioned mixed solution of plasmid DNA, linear baculovirus DNA and lipofectin, the cells were cultured at 27°C for 3 days and 1 ml of a culture supernatant containing recombinant virus was collected. To the dish was newly added 1 ml of an ESF 921 medium and a further cultivation was carried out at 27°C for 3 days to give additional 1.5 ml of culture supernatant containing the recombinant virus.

Thereafter, each of the recombinant viruses containing a DNA encoding VPLF and a DNA encoding VPLFΔN was proliferated according to the following procedures.

A shaking culture of the Sf9 cells of 5 × 10⁵/ml in 50 ml of an ESF 921 medium was carried out at 27°C and 125 rpm using a 125-ml Erlenmeyer flask . At the stage when the cells were proliferated to an extent of 2 × 10⁶/ml, the recombinant virus was infected so as to make MOI (multiplicity of infection) 10 and cultivation was carried out for 3 days longer. The cultured medium was centrifuged for 10 minutes at 11,760 m/s² to remove the cells and a recombinant virus solution used for the expression of the protein was obtained.

Titer of the recombinant virus solution was measured by a method as mentioned below.

Sf9 cells (6 × 10⁵) were suspended in 4 ml of an ESF 921 medium, placed in a plastic dish for cell culture having a diameter of 50 mm and allowed to stand at room temperature for 1 hour whereupon the cells were adhered to the dish. The supernatant was removed, 400 ml of an ESF 921 medium and 100 ml of the above recombinant virus solution which was diluted with an ESF 9219 medium were added thereto, the mixture was allowed to stand at room temperature for 1 hour, the medium was removed and 5 ml of a medium [prepared by mixing 1 ml of sterilized 5% aqueous solution of AgarPlaque Plus Agarose with 4 ml of TMN-FH Insect Medium (manufactured by Pharmingen) followed by keeping at 42°C] containing 1% low melting point agarose [AgarPlaque Agarose; manufactured by Pharmingen] was poured into the dish. After it was allowed to stand at room temperature for 15 minutes, vinyl tape was wound on the dish for the prevention of drying, the dish was placed in a plastic container which was able to be tightly closed and cultivation was carried out at 27°C for 5 days. To the said dish was added 1 ml of PBS containing 0.01% of Neutral Red, cultivation was carried out for 1 day longer and number of the resulting plaques were counted. As a result, a recombinant virus solution of 0.5 to 2 × 10⁸/ml was prepared.

### (3) Expression of protein

High 5 cells (manufactured by Invitrogen) were subjected to a shaking culture at 27°C and 125 rpm at the density of 5 × 10⁵/ml in 100 ml of ESF 921 medium using a 250-ml Erlenmeyer flask. When the cells were proliferated to an extent of 3 to 4 × 10⁶/ml, they were subcultured at 3 × 10⁷ cells in a flask having a base area of 182 cm² to which 25 ml of ESF 921 medium were previously added. The cells were allowed to stand at room temperature for 1 hour to adhere, the medium was removed, a recombinant virus containing the DNA encoding VPLF or the DNA encoding VPLFΔN was added so as to make MOI 5, then an ESF 921 medium was further added to make 10 ml and the cells were infected for 1 hour at room temperature. To this were added 20 ml of ESF 921 medium followed by cultivation at 27°C for 3 days, and the aimed recombinant protein was expressed.

Heparin-Cellulofine resin was added to each of supernatants of cultured cells to which a recombinant virus containing a DNA encoding VPLF or a DNA encoding VPLFΔN was infected, reaction was carried out at 4°C for one night and the resin was recovered, washed with 20 mmol/l sodium phosphate (pH 7.2) and eluted with the same buffer containing 1 mol/l of NaCl. An eluate corresponding to 1 ml of the supernatant was used as a sample and subjected to Western blotting using anti-VPLF peptide antibody (KM 2676) obtained in Reference Example 13. As a result, a band for molecular weight of about 40 kDa was detected when the DNA encoding VPLF was infected while, when a recombinant virus containing DNA encoding VPLFΔN was infected, a band for molecular weight of about 20 kDa was detected.

### [Reference Example 7] Purification of VPLFΔN

Four ml of Heparin-Cellulofine resin (manufactured by Chisso) equilibrated with 50 nmmol/l sodium phosphate (pH 7.2) was added to 400 ml of the supernatantof cultured High 5 cells in which VPLFΔN was expressed and then gently stirred at 4°C for 12 hours whereupon the protein was adsorbed with the resin. The resin was filled in a column, washed with 10 ml of 50 mmol/l sodium phosphate (pH 7.2) and eluted with 10 ml of 50 mmol/l sodium phosphate (pH 7.2)/0.75 mol/l NaCl solution. After this was dialyzed against 20 mmol/l sodium phosphate (pH 7.2)/0.2 mol/l NaCl solution, centrifugation was carried out at 78,400 m/s² for 5 minutes and the supernatant was passed through 10 ml of SP Sepharose (manufactured by Pharmacia Biotech) equilibrated with 20 mmol/l sodium phosphate (pH 7.2)/0.2 mol/l NaCl solution. After this was washed with 40 ml of 20 mmol/l sodiumphosphatebuffer (pH 7.2)/0.2 mol/l NaCl solution, elution with 40 ml of a linear gradient of 0.2 to 1 mol/l NaCl was conducted. Further elution with 10 ml of 20 mmol/l sodium phosphate (pH 7.2)/l mol/l NaCl solution was carried out. The eluting condition conducted was 1 ml/minute, 2 ml/fraction. A fraction containing much VPLFΔN was recovered and 20 mmol/l sodium phosphate (pH 7.2)/4 mol/l NaCl solution was added so as to give 3 mol/l NaCl. Thereafter, it was passed through 1.57 ml of Butyl Sepharose (manufactured by Pharmacia Biotech) equilibrated with 20 mmol/l sodium phosphate (pH 7.2)/3 mol/l NaCl solution, washed with 10 ml of 20 mmol/l sodium phosphate (pH 7.2)/3 mol/l NaCl solution and eluted with 20 ml of a linear gradient of 3 to 0 mol/l NaCl. The eluting condition used was 0.2 ml/minute, 1 ml/fraction. The fraction containing VPLFΔN was recovered and concentrated using Centricon-10 (manufactured by Amicon).

The resulting concentrated solution was used as a sample, subjected to 5 to 20% polyacrylamide gel electrophoresis and analyzed by means of gel staining using Coomassie Brilliant Blue R250 whereupon the resulting purity was about 85%. The result of analysis of the N-terminal amino acid sequence of the purified protein was Asp-Pro-Ser-Pro-Phe-Val (SEQ ID NO: 35) and Ser-Pro-Phe-Val-Phe-Gly (SEQ ID NO: 36) and, therefore, it was apparent that the present purified protein was a mixture of a protein (SEQ ID NO: 32) where four amino acids (Asp-Pro-Ser-Pro: SEQ ID NO: 34) derived from mature melittin were added to the N-terminal of the 227th Phe to the 345th Gly of VPLF and a protein (SEQ ID NO: 33) where two amino acids (Ser-Pro) were added thereto.

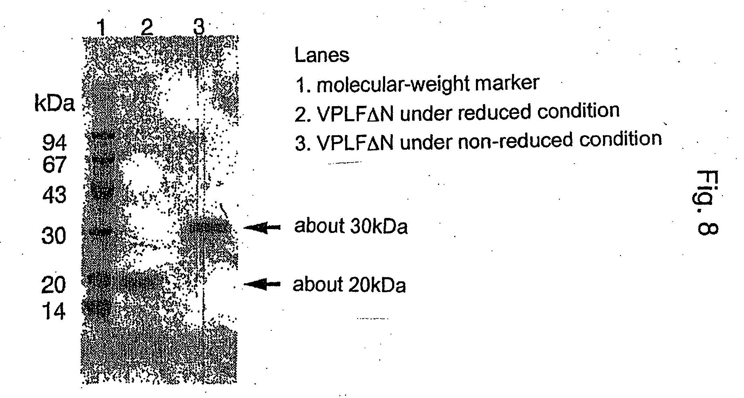

Thereafter, the purified VPLFΔN (2 µg) was used as a sample and subjected to an SDS-polyacrylamide gel electrophoresis under reducing condition and non-reducing condition to check the change of mobility of VPLFΔN. Detection was carried out by staining with Coomassie Brilliant Blue R250. As a result, mobility of a band showing a mobility corresponding to molecular weight of about 20 kDa under the reducing condition lowered under the non-reducing condition whereby a band was detected at the position corresponding to molecular weight of about 30 kDa (Fig. 8). Therefore, it was suggested that VPLFΔN formed a dimer via an S-S bond.

Then it was checked whether an N-type sugar chain was present or absent. VPLFΔN (2 µg) was treated at 100°C for 5 minutes in the presence of 0.5% SDS and 50 mmol/l β-mercaptoethanol, then Nonidet P-40 (manufactured by Nacalai Tesque) was added at 2.5% and reaction was carried out by addition of 0.3U of N-glycosidase F (manufactured by Takara Shuzo). After the reaction at 37°C for 20 minutes, SDS-polyacrylamide gel electrophoresis was carried out and VPLFΔN was detected by silver-staining. As a result, its molecular weight lowered by the treatment with N-glycosidase F and, therefore, it was noted that N-type sugar chain was added to VPLFΔN.

### [Reference Example 8] Expression of VPLF using animal cell as a host

### (1) Construction of a recombinant vector:

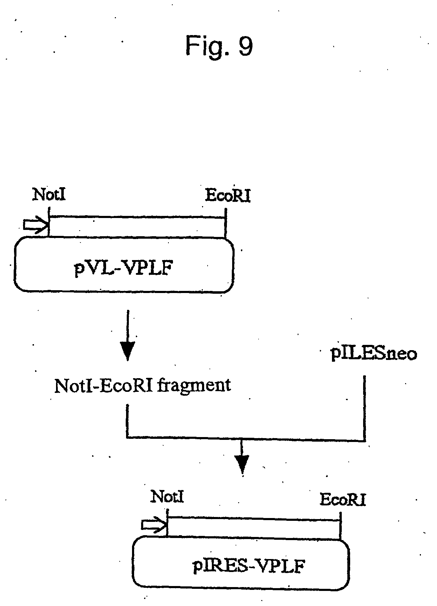

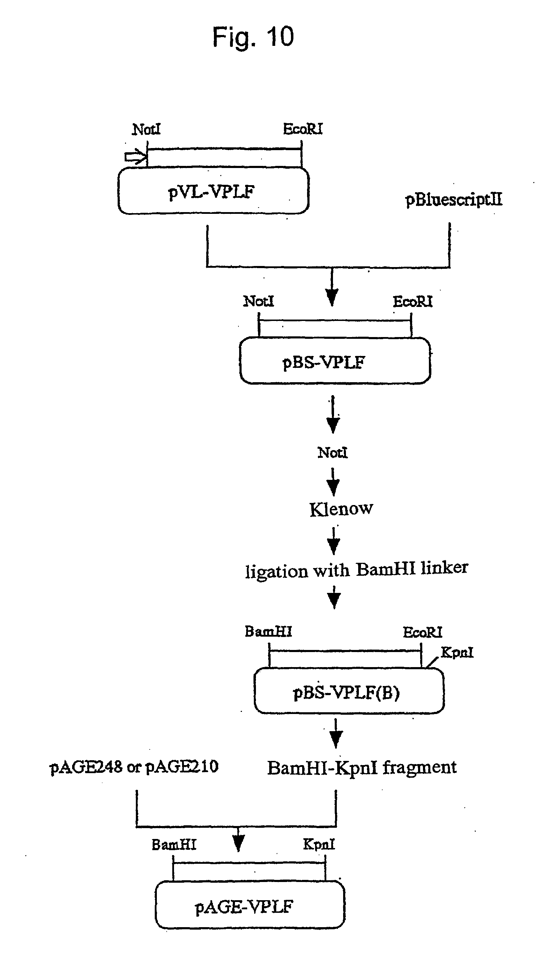

(i) pIRES-VPLF: NotI-EcoRI (1 kb) fragment of pVL-VPLF was inserted into a
   NotI-EcoRI site of pIRESneo (manufactured by Clontech) to prepare pIRES-VPLF (Fig. 9).
(ii) pAGE248-VPLF and pAGE210-VPLF: NotI-EcoRI (1 kb) fragment of pVL-VPLF was inserted into NotI-EcoRI of a vector pBluescript II to construct pBS-VPLF. The pBS-VPLF was cleaved with NotI and treated with Klenow and BamHI linker (5'-CGGATCCG-3') was inserted thereinto to construct pBS-VPLF (B). BamHI-KpnI (1 kb) of pBS-VPLF (B) was inserted into a BamHI-KpnI site of pAGE210 or pAGE248 [Sasaki, K. et al.: J. Biol. Chem., 269, 14730-14737 (1994)] to construct pAGE210-VPLF and pAGE248-VPLF (Fig. 10).

### (2) Transfection of recombinant vector into cell:

(i) Transfection into PC-9 cell:
   An RPMI 1640 medium (50 ml) (containing 0.2% sodium carbonate and 2 mmol/l L-glutamine) which contained 1 µg of plasmid pIRES-VPLF and 50 ml of an RPMI 1640 medium which contained 2 ml of LipofectAMINE™ 2000 (manufactured by Gibco BRL) were mixed and allowed to stand at room temperature for 20 minutes. PC-9 cells were suspended in the RPMI 1640 medium and 3 × 10⁵ cells were inoculated into 0.5 ml of the RPMI 1640 medium which was added to a 24-well plate. The above mixed solution was added to the resulting cell culture and cultured at 37°C for one day. Cultivation was carried out in an RPMI 1640 medium containing 0.3 mg/ml of G418 and 5% dFCS (manufactured by Gibco) to obtain a resistant cell (hereinafter, referred to as "PC-9/VPLF cell" ).
(ii) Transfection into CHO (cell line DG 44) cell:
   pAGE248-VPLF or pAGE210-VPLF (4 µg) was added to 200 ml of a cell suspension in a K-PBS solution (10.2 g KCl, 0.16 g Na 1.15 g Na₂HPO₄, 0.2 g KH₂PO₄ and 0.81 g MgCl₂.6H₂O) of 8 × 10⁶ cell/ml and the recombinant vector was transfected into the cells by an electroporation method (0.35 kV, 250 mF) using Gene Pulser 2 (manufactured by Bio-Rad). The cells transfected with the recombinant vector were cultured for one day in an EXCELL 302 medium (manufactured by Nichirei) containing 5% of dFCS (manufactured by Gibco) and hygromycin was added at 0.3 mg/ml to select a resistant cell. Further, methotrexate (MTX) was added at 100 nmol/l or 500 nmol/l to select a resistant cell (hereinafter, the resistant cells prepared by the transfection of pAGE248-VPLF and pAGE210-VPLF will be referred to as "DG44/pAGE248-VPLF cell" and "DG44/pAGE210-VPLF cell", respectively and the two kinds of cells will be referred to "DG44/VPLF cell" as a whole).

### (3) Confirmation of expression

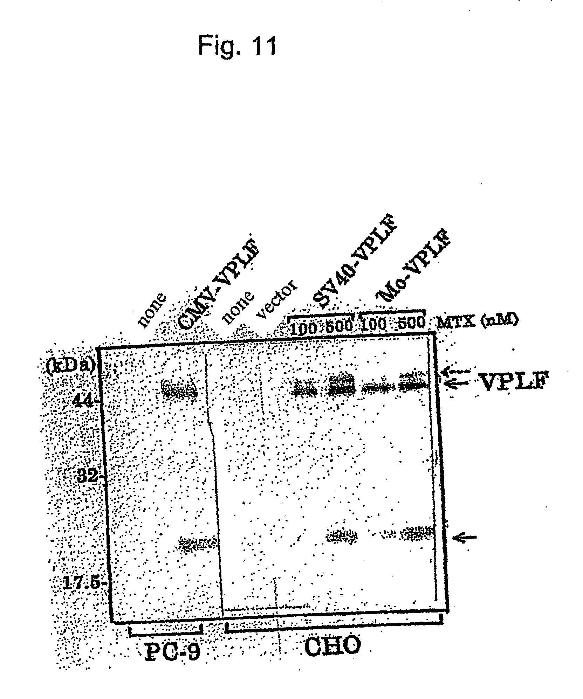

Each of the PC-9/VPLF cell prepared in the above (2) (i) and the DG44/VPLF cell prepared in the above (ii) was cultured in an RPMI 1640 medium (containing 0.2% of sodium carbonate, 2 mmol/l of L-glutamine, 10% of FCS and 0.3 mg/ml of G418) and an EXCELL 302 (containing 0.3 mg/ml of hygromycin and 100 nmol/l or 500 nmol/l of MTX) until it became confluent to give a culture supernatant. To each of the culture supernatant was added Heparin-Cellulofine resin (manufactured by Chisso), reaction was conducted at 4°C for one night and the resin was recovered, washed with 20 mmol/l sodium phosphate (pH 7.2) and eluted with the same buffer containing 1 mol/l of NaCl. The eluate corresponding to 1 ml of the supernatant was used as a sample for subjecting to Western blotting using an anti-VPLF peptide antibody (KM 2676) obtained in the following Reference Example 13. The result is shown in Fig. 11. In Fig. 11, a lane where the supernatant of PC-9/VPLF cell is electrophoresed is shown as CMV-VPLF, a lane where the supernatant of DG44/pAGE248-VPLF cell electrophoresed is shown as SV40-VPLF and a lane where the supernatant of DG44/pAGE210-VPLF cell is electrophoresed is shown as Mo-VPLF.

As a result of analysis by Western blotting, there were detected two bands of molecular weights of about 45 kDa and about 20 kDa from the culture supernatant of expressed animal cells. The band of about 20 kDa showed a mobility which was nearly the same as that of VPLFΔN expressed using insect cell as a host. Accordingly, it has been found that VPLF expresses in animal cells in a processed form.

### [Reference Example 9] Effect of VPLF on human undifferentiated hematopoietic cells

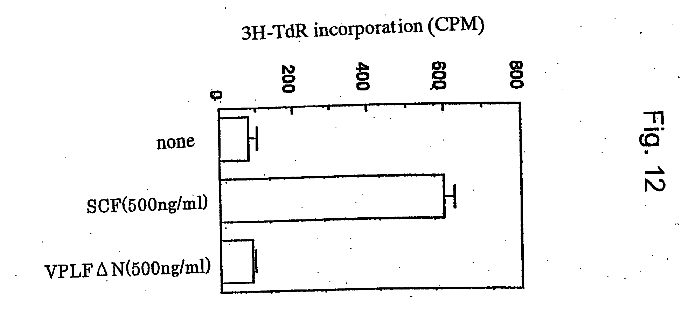

Effect of VPLFΔN obtained in Reference Example 7 to human undifferentiated hematopoietic cells was investigated as follows. CD34 positive human bone marrow cells (manufactured by BioWhittaker) were used as the human undifferentiated hematopoietic cells. The CD34 positive human bone marrow cells suspended at a cell density of 1 × 10⁵ cells/ml in an IMDM (manufactured by Stemcell Technologies) were inoculated on a 96-well plate for cell culture (manufactured by Sumitomo Bakelite) at the rate of 100 µl/well, then VPLFΔN or stem cell factor SCF (manufactured by Genzyme) was added at the final concentration 500 ng/ml and cultivation was carried out at 37°C for 48 hours under the condition of 5% CO₂. After 48 hours, 9.25 kBq of [6-³H]-thymidine (hereinafter, abbreviated as "³H-TdR") (manufactured by NEN) was added, cultivation was conducted for 12 hours more and the cells were recovered on a glass filter using a cell harvester. Radioactivity taken up into the recovered cells was measured using a radiation counter Matrix 96 (manufactured by Packard). The above experiments were repeated independently using CD34 positive human bone marrow cells of lot No. 9F0329 (19 years age; Asian male; CD34 positive rate: 96.4%) and that of lot No. 9F1809 (29 years age; Caucasian male; CD34 positive rate: 96.1%) from two volunteers and the same result was obtained in any of both cases. Result of the experiment using lot No. 9F0329 is shown in Fig. 12. Although there was observed a growth-promoting activity of SCF for human CD 34 positive bone marrow cells, the said growth-promting activity as the effect of VPLFΔN was not observed.

### [Reference Example 10] Effect of VPLF on smooth muscle cells

Biological activity of VPLFΔN obtained in Reference Example 7 for smooth muscle cells was confirmed as follows.

To a 96-well collagen-coated plate (manufactured by Iwaki) were added rat-derived smooth muscle cells (RSMC) [FEBS Letters, 425, 123 (1998)] suspended in an M-199 medium (manufactured by Gibco BRL) to which 10% fetal bovine serum (FBS), 100 units/ml of penicillin (manufactured by Gibco BRL) and 100 µg/ml of streptomycin (manufactured by Gibco BRL) were added at 3,000 cells/200 µl/well and cultivation was carried out for one day in a CO₂ incubator of 37°C. After the cultivation, the supernatant was removed, each 10 µl of Opti-MEM (manufactured by Gibco BRL) were added to each well and cultivation was carried out at 37°C for 3 hours. After the cultivation, each 100 µl/well of human VEGF165 (manufactured by R&D), human IL-5 (manufactured by R&D), human PDGF BB (manufactured by R&D) or VPLFΔN diluted with Opti-MEM were added to each well (final concentration being from 160 pg/ml to 500 ng/ml) and cultivation was carried out for 2 days in a CO₂ incubator of 37°C. After completion of the cultivation, each 10 µl of MTT reaction reagent [Cell proliferation Kit I; manufactured by Boehringer-Mannheim] to each well and cultivation was carried out for 4 hours in a CO₂ incubator of 37°C. After completion of the cultivation, each 100 µl of dissolving reagent were added to each well followed by dissolving at 37°C for one night in an incubator. After completion of dissolving, absorbance of each well at OD 590 nm was measured using OD 650 nm as an reference wavelength.

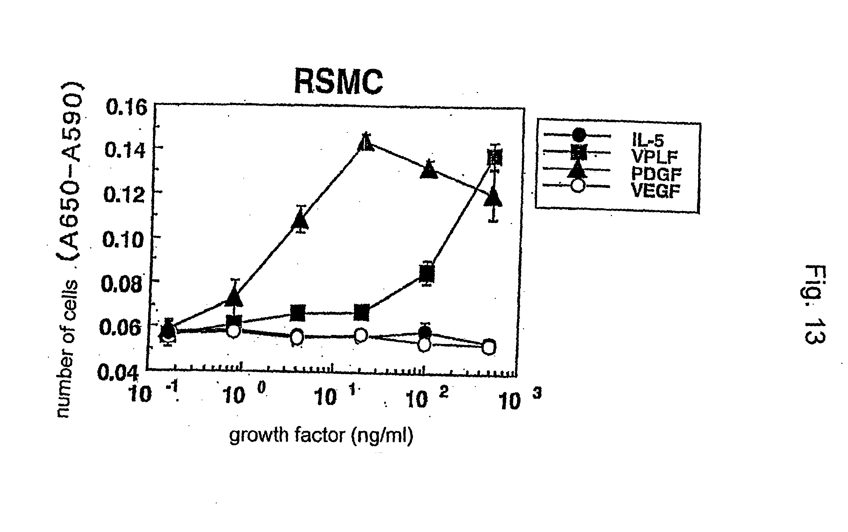

The result is shown in Fig. 13. Human VPLFΔN showed a growth-promoting activity for RSMC on a concentration-depending manner. Human PDGF BB which was a positive control showed a growth-promoting activity for RSMC at 50- to 100-fold lower concentration. On the contrary, human VEGF 165 and human IL-5 which was used as a control showed no growth-promoting activity for RSMC.

### [Reference Example 11] Effect of VPLF on endothelial cells

Biological activity of VPLFΔN obtained in Reference Example 7 was confirmed as follows.

To a 96-well collagen-coated microtiter plate (manufactured by Iwaki) were added human skin-derived microvascular endothelial cells HMVEC (manufactured by Kurabo) suspended in a E-BH medium (manufactured by Kurabo) to which 2% of fetal bovine serum (FBS), 10 ng/ml of human recombinant-type epithelial growth factor (rEGF), 1 µg/ml of hydrocortisone, 50 µg/ml of gentamicin and 50 ng/ml of amphotericin B were added at 3000 cells/100 µl/well. Thereafter, each 100 µl/well of human VEGF 165 (manufactured by R&D), human IL-5 (manufactured by R&D), human PDGF BB (manufactured by R&D) or VPLFΔN diluted with the above-mentioned medium were added (final concentration being from 10 pg/ml to 100 ng/ml) and cultivation was carried out for 5 days in a CO₂ incubator of 37°C. After completion of cultivation, each 20 µl of chromogenic reagent for cells (Cell Counting Kit; manufactured by Dojindo) were added to each well and incubation was carried out at 37°C for 1 to 2 hour(s). After completion of the incubation, absorbance of eachwell at OD 650 nmwas measured using OD 450 nm as a reference wavelength.

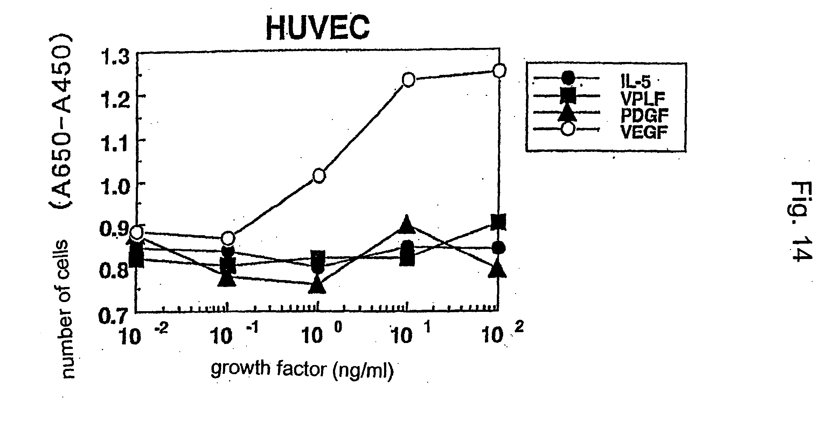

The result is shown in Fig. 14. Human VEGF 165 showed a growth-promoting activity for HMVEC on a concentration-depending manner but human VPLFΔN and human PDGF BB did not show a growth-promoting activity. Human IL-5 used as a control did not show a growth-promoting activity.

### [Reference Example 12] Preparation of antigen for the preparation of VPLF antibody

Protein sequence of VPLF was analyzed and compounds 1 to 4 (SEQ ID NOS: 28 to 31) were selected as partial sequences presumed to be appropriate as antigens from highly hydrophilic part, N-terminal, C-terminal and the part having turn structure or random coil structure in view of secondary structure.

In the following Reference Examples, physical and chemical properties of the compounds were measured by the following methods. Mass analysis was carried out by FAB-MS using JMS-HX 110A of JEOL. Amino acid analysis was carried out by a method of Cohen, S.A., et al. [Analytical Biochemistry, 222, 19 (11994)]. Hydrolysis was carried out in a hydrochloric acid vapor at 110°C for 20 hours and amino acid composition of the hydrolyzed product was analyzed using a Waters AccQ-Tag Amino Acid Analyzer (manufactured by Waters).

### (1) Synthesis of the compound 1 (SEQ ID NO: 28) (H-Cys-Thr-Gln-Ala-Glu-Ser-Asn-Leu-Ser-Ser-Lys-Phe-Gln-Phe-Ser-Ser-Asn-Lys-Glu-Gln-Asn-Gly-NH₂)

A carrier resin bound to 16.5 µmol of Fmoc-NH [Rink amide MBHA resin; manufactured by Novabiochem] (30 mg) was placed in a reactor of an automatic synthesizer (manufactured by Shimadzu), 858 µl of DMF were added, the mixture was stirred for 1 minute, the solution was discharged therefrom and the following operations were carried out according to the synthesis program of Shimadzu.
(a) A 30% piperidine-DMF solution (734 µl) was added, the mixture was stirred for 4 minutes, the said solution was discharged and such an operation was repeated once again.
(b) The carrier resin was washed with 500 µl of DMF for 1 minute, the said solution was discharged and such an operation was repeated for five times.
(c) Fmoc-Gly-OH (165 µmol), HBTU (165 µmol), HOBt monohydrate (165 µmol) and DIEA (330 µmol) were stirred for 3 minutes in DMF (858 µl), the resulting solution was added to the resin, the mixture was stirred for 30 minutes and the solution was discharged.
(d) The carrier resin was washed with 858 µl of DMF for one minute, the solution was discharged and such an operation was repeated for five times.

As such, Fmoc-Gly-NH was synthesized on the carrier.

Next, after the steps of (a) and (b), condensation reaction was carried out using Fmoc-Asn(Trt)-OH in the step of (c) and a washing step of (d) was carried out whereupon Fmoc-Asn(Trt)-Gly-NH was synthesized on the carrier.

Thereafter, the steps of (a) to (d) were repeated where Fmoc-Gln(Trt)-OH, Fmoc-Glu(OtBu)-OH, Fmoc-Lys(Boc)-OH, Fmoc-Asn(Trt)-OH, Fmoc-Ser(tBu)-OH, Fmoc-Ser(tBr)-OH, Fmoc-Phe-OH, Fmoc-Gln(Trt)-OH, Fmoc-Phe-OH, Fmoc-Lys(Boc)-OH, Fmoc-Ser(tBu)-OH, Fmoc-Ser(tBu)-OH, Fmoc-Leu-OH, Fmoc-Asn(Trt)-OH, Fmoc-Ser(tBu)-OH, Fmoc-Glu(OtBu)-OH, Fmoc-Ala-OH, Fmoc-Gln(Trt)-OH, Fmoc-Thr(tBu)-OH and Pmoc-Cys(Trt)-OH were successively used in the step (c). Thereafter, deprotecting and washing steps of (a) and (b) were carried out, successive washings with methanol and butyl ether were conducted and drying in vacuo was carried out for 12 hours to give a carrier resin to which side chain protected peptide was bound. In the case of condensation of Fmoc-Phe-OH however, a mixed solvent comprising 198 µl of NMP and 660 µl of DMF was used instead of 858 µl of DMF. To this was added 1 ml of a mixed solvent comprising TFA (90%), thioanisole (5%) and 1,2-ethanediol (5%), the mixture was allowed to stand at room temperature for 2 hours, the protective group for side chain was removed and, at the same time, peptide was excised from the resin. After filtering the resin, about 10 ml of ether were added to the resulting solution and the resulting precipitate was recovered by centrifugation and decantation to give 37.9 mg as a crude peptide. The crude product was dissolved in 1 ml of 90% acetic acid, 4 ml of DMF and 1 ml of trifluoroethanol and the resulting supernatant was purified by an HPLC using a reversed phase column (Capcell Pak C18 30 mm I. D. × 25 mm; manufactured by Shiseido). Elution was carried out by means of a linear concentration gradient method where a 90% aqueous solution of acetonitrile containing 0.1% of TFA was added to a 0.1% aqueous solution of TFA and detection was conducted at 220 nm to give a fraction containing the compound 1. Further, the precipitate upon dissolving the crude peptide was dissolved by adding 30 mg of dithiothreitol and 1 ml of 8 mol/l urea thereto, diluted with 3 ml of 2 mol/l acetic acid and purified by an HPLC using the same reversed phase column as mentioned above to give a fraction containing the compound 1. Those fractions were freeze-dried to give 8.6 mg of the compound 1.

Mass analysis [FABMS]: m/z = 2434.0 (M + H⁺)

Amino acid analysis: Asx 3.1 (3), Ser 4.6 (5), Glx 5.1 (5), Gly 1.1 (1), Thr 0.9 (1), Ala 1.0 (1), Cys 1.3 (1), Lys 2.0 (2), Leu 1.0 (1), Phe 2.0 (2)

### (2) Synthesis of compound 2 (SEQ ID NO: 29) (H-Cys-Ser-Ile-Arg-Glu-Glu-Leu-Lys-Arg-Thr-Asp-Thr-Ile-Phe-Trp-Pro-Gly-NH₂)

A carrier resin to which 16.5 µmol of Fmoc-NH were bound [Rink Amide MBHA resin; manufactured by Novabiochem] (30 mg) was used as a starting substance, Fmoc-Gly-OH, Fmoc-Pro-OH, Fmoc-Trp(Boc)-OH, Fmoc-Phe-OH, Fmoc-Ile-OH, Fmoc-Thr(tBu)-OH, Fmoc-Asp(OtBu)-OH, Fmoc-Thr(tBu)-OH, Fmoc-Arg(Pmc)-OH, Fmoc-Lys(Boc)-OH, Fmoc-Leu-OH, Fmoc-Glu(OtBu)-OH, Fmoc-Glu(OtBu)-OH, Fmoc-Arg(Pmc)-OH, Fmoc-Ile-OH, Fmoc-Ser(tBu) and Fmoc-Cys(Trt)-OH were successively condensed therewith in the same way as in the above (1) and removal of Fmoc group, washing and drying were carried out to give a carrier resin to which side chain-protected peptide was bound. To this was added 1 ml of a mixed solvent comprising TFA (82.5%), thioanisole (5%), water (5%), ethyl methyl sulfide (3%), 1,2-ethanedithiol (2.5%) and thiophenol (2%) containing 2-methylindole at the concentration of 5 mg/ml and the mixture was allowed to stand at room temperature for 6 hours whereupon the protective group at the side chain was removed and, at the same time, peptide was excised from the resin. In the same way as in the above (1), 41.4 mg of crude peptide were obtained, dissolved in aqueous solution of acetic acid and purified by an HPLC using a reversed phase column to give 7.1 mg of the compound 2.

Mass analysis [FABMS]: m/z = 2051.2 (M + H⁺);

Amino acid analysis: Asx 1.1 (1), Ser 1.0 (1), Glx 2.1 (2), Gly 1.3 (1), Arg 1.5 (2), Thr 1.9 (2), Pro 1.1 (1), Cys 1.2 (1), Lys 1.0 (1), Ile 1.8 (2), Leu 1.1 (1), Phe 0.9 (1) (Trp was not analyzed)

### (3) Synthesis of compound 3 (SEQ ID NO: 30) (Ac-Thr-Phe-Asp-Glu-Arg-Phe-Gly-Leu-Glu-Asp-Pro-Glu-Asp-Asp -Ile-Cys-Lys-NH₂)

A carrier resin to which 16.5 µmol of Fmoc-NH were bound [Rink Amide MBHA resin; manufactured by Novabiochem] (30 mg) was used as a starting substance, Fmoc-Lys(Boc)-OH, Fmoc-Cys(Trt)-OH, Fmoc-Ile-OH, Fmoc-Asp(OtBu)-OH, Fmoc-Asp(OtBu)-OH, Fmoc-Glu(OtBu)-OH, Fmoc-Pro-OH, Fmoc-Asp(OtBu)-OH, Fmoc-Glu(OtBu)-OH, Fmoc-Leu-OH, Fmoc-Gly-OH, Fmoc-Phe-OH, Fmoc-Arg(Pmc)-OH, Fmoc-Glu(OtBu)-OH, Fmoc-Asp(OtBu)-OH, Fmoc-Phe-OH and Fmoc-Thr(tBu)-OH were successively condensed therewith in the same way as in the above (1), Fmoc group was removed, 500 µl of DMF containing 31 µl of acetic anhydride were added and the mixture was stirred for 30 minutes. The solution was discharged and washing and drying were carried out to give a carrier resin to which side chain-protected peptide was bound. To this was added 1 ml of a mixed solvent comprising TFA (82.5%), thioanisole (5%), water (5%), ethyl methyl sulfide (3%), 1,2-ethanedithiol (2.5%) and thiophenol (2%) and the mixture was allowed to stand at room temperature for 8 hours whereupon the protective group at the side chain was excised and, at the same time, peptide was excised from the resin. In the same way as in the above (1), 40.7 mg of crude peptide were obtained, dissolved in aqueous solution of acetic acid and purified by an HPLC using a reversed phase column to give 18.3 mg of the compound 3.

Mass analysis [FABMS]: m/z = 2071.6 (M + H⁺);

Amino acid analysis: Asx 4.0 (4), Glx 3.1 (3), Gly 1.0 (1), Arg 0.9 (1), Thr 1.0 (1), Pro 1.0 (1), Cys 1.3 (1), Lys 1.0 (1), Ile 0.9 (1), Leu 1.0 (1), Phe 2.0 (2)

### (4) Synthesis of compound 4 (SEQ ID NO: 31) (H-Cys-Arg-Gly-Ser-Thr-Gly-Gly-OH)

A carrier resin to which 24.0 µmol of Fmoc-Gly were bound [Wang resin; manufactured by Novabiochem] (30 mg) was used as a starting substance, Fmoc-Gly-OH, Fmoc-Thr(tBu)-OH, Fmoc-Ser(tBu)-OH, Fmoc-Gly-OH, Fmoc-Arg(Pmc)-OH and Fmoc-Cys(Trt)-OH were successively condensed therewith in the same way as in the above (1), Fmoc group was removed and washing and drying were carried out to give a carrier resin to which side chain-protected peptide was bound. To this was added 1 ml of a mixed solvent comprising TFA (82.5%), thioanisole (5%), water (5%), ethyl methyl sulfide (3%), 1,2-ethanedithiol (2.5%) and thiophenol (2%) and the mixture was allowed to stand at room temperature for 8 hours whereupon the protective group at the side chain was excised and, at the same time, peptide was excised from the resin. In the same way as in the above (1), 21.1 mg of crude peptide were obtained, dissolved in a 0.1% aqueous solution of TFA and purified by an HPLC using a reversed phase column to give 3.6 mg of the compound 4.

Mass analysis [FABMS]: m/z = 637.2 (M + H⁺);

Amino acid analysis: Ser 0.9 (1), Gly 3.1 (3), Arg 0.9 (1), Thr 0.9 (1), Cys 1.1 (1)

### [Reference Example 13] Preparation of monoclonal antibody recognizing VPLF

### (1) Preparation of immunogen

For the purpose of enhancing the immunogenicity, the compounds 1 to 4 prepared in Reference Example 12 were converted to a conjugate with KLH (manufactured by Calbiochem) by the following method to prepare immunogens. Thus, KLH was dissolved in PBS to make 10 mg/ml, then 1/10 volume of 25 mg/ml MBS [N-(m-maleimidobenzoyloxy)succinimide; manufactured by Nacalai Tesque] was dropped thereinto and the mixture was reacted for 30 minutes with stirring. KLH-MB (2.5 mg) wherefrom free MBS was removed by gel filtration such as Sephadex G-25 column or the like which was previously equilibrated with PBS was mixed with 1 mg of peptide dissolved in a 0.1 mol/l sodium phosphate buffer (pH 7.0) and the reaction was carried out at room temperature for 3 hours with stirring. After the reaction, the product dialyzed against PBS was used as an immunogen.

### (2) Immunization of animals and preparation of antibody-producing cells

Each of the KLH conjugates (100 µg) of the compounds 1 to 4 prepared hereinabove (1) was administered to each 3 female SD rats of 5 weeks old together with 2 mg of aluminum hydroxide adjuvant (Antibodies - A Laboratory Manual, Cold Spring Harbor Laboratory, p. 99, 1988) and 1 × 10⁹ cells of pertussis vaccine (manufactured by Chiba Serum Institute). From 2 weeks after the administration, 100 µg of each KLH conjugate were administered once a week for four times in total. Blood was collected from heart of the rat, its serum antibody titer was checked by enzyme linked immunosorbent assay as shown below and spleen was excised from the rat showing a sufficient antibody titer after 3 days from the final immunization.

The spleen was finely cut in an MEM (minimum essential medium) (manufactured by Nissui Seiyaku), loosened by tweezers and centrifuged (at 2,450 m/s² for 5 minutes). To the resulting precipitate fraction was added a Tris-ammonium chloride buffer (pH 7.6) and a treatment was conducted for 1 to 2 minutes to remove erythrocytes. The resulting precipitate fraction (cell fraction) was washed with an MEM for three times and used for the cell fusion.

### (3) Enzyme linked immunosorbent assay (Binding ELISA)

With regard to an antigen for the assay, each of the compounds obtained in Reference Example 12 was conjugated with thyroglobulin (hereinafter, referred to as "THY") was used. The method of preparation was the same as the above (1) except that SMCC [4-(N-maleimidoethyl)-cyclohexane-1-carboxylic acid N-hydroxysuccinimido ester; manufactured by Sigma] was used instead of MBS as a Cross-linker. The conjugate prepared as mentioned above (10 µg/ml) was placed on a 96-well plate for EIA (manufactured by Greiner) at the rate of 50 µl/well and adsorbed by being allowed to stand at 4°C for one night. The plate was washed, a 1% bovine serum albumin (BSA)/PBS was added at the rate of 100 ml/well and allowed to stand at room temperature for 1 hour and the remaining active groups were blocked.

After being allowed to stand, the 1% BSA/PBS was discarded and 50 ml/well of immunized rat antiserum, cultured medium of monoclonal antibody or purified monoclonal antibody were added to the plate and allowed to stand for 2 hours. The plate was washed with 0.05% polyoxyethylene (20) sorbitan monolaurate [trade name: Span 20 (an equivalent product to Tween 20(trademark of ICI); manufactured by Wako Pure Chemical)]/PBS (hereinafter, referred to as "Tween-PBS") and 50 ml/well of peroxidase-labeled rabbit anti-rat immunoglobulin were added followed by being allowed to stand at room temperature for 1 hour. After the plate was washed with Tween-PBS, an ABTS (ammonium 2,2-azinobis(3-ethylbenzothiazole-6-sulfonate) substrate solution (1 mmol/l ABTS/0.1 mol/l citrate buffer (pH 4.2)) were added thereto to produce color and absorbance at OD 415 nm was measured using a plate reader [Emax; Molecular Devices].

### (4) Preparation of mouse myeloma cells

8-Azaguanine-resistant mouse myeloma cell line P3X63Ag8U.1 [P3-U1; purchased fromATCC] was cultured in a normal medium (RPMI 1640 medium to which 10% fetal bovine serum were added) and 2 × 10⁷ or more cells were secured for the cell fusion and used as a parent cell for the cell fusion.

### (5) Preparation of hybridomas

Rat spleen cells obtained in the above (2) and myeloma cells obtained in the above (4) were mixed to make the ratio 10:1 followed by subjecting to centrifugation (at 2,450 m/s² for 5 minutes). Cells of the precipitate fraction was well loosened, a mixed solution of 2 g of polyethylene glycol 1000 (PEG-1000), 2 ml of MEM and 0.7 ml of dimethyl sulfoxide was added thereto at 37°C with stirring, then 1 ml of MEM was added every 1 to 2 minute(s) for several times and MEM was added so as to make the total volume 50 ml.

The suspension was centrifuged (at 900 rpm for 5 minutes), cells of the precipitate fraction was gently loosened and the cells were suspended in 100 ml of HAT medium [a medium where HAT Media Supplement (manufactured by Boehringer-Mannheim) was added to RPMI 1640 medium to which 10% of fetal bovine serum were added] by means of sucking in and out using a measuring pipette. The suspension was placed on a 96-well culture plate at the rate of 200 ml/well and cultured at 37°C for 10 to 14 days in a 5% CO₂ incubator.

After cultivation, the supernatant was checked by the enzyme-linked immunosorbent assay mentioned in the above (3) so as to select the wells which reacted with the antigen peptide but did not react with the control peptide, cloning by a limiting dilution method was repeated twice from the cells contained therein and there was established an anti-human VPLF monoclonal antibody-producing hybridoma. As a result, there was obtained anti-human VPLF monoclonal antibody KM 2676 using the compound 2 as antigen.

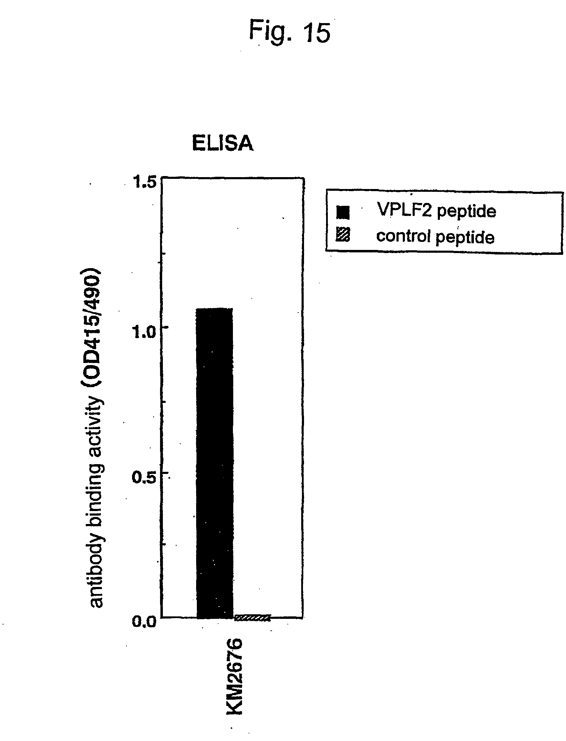

As shown in Fig. 15, the KM 2676 showed a specific reactivity to the compound 2. (In Fig. 15, the well coated with the compound 2 is shown as VPLF 2 peptide.)

Incidentally, the hybridoma KM 2676 which produces anti-human VPLF monoclonal antibody KM 2676 has been deposited as FERM BP-7137 on April 18, 2000 at the International Patent Organism Depositary, National Institute of Advanced Industrial Science and Technology (Central 6, 1-1, Higashi 1-chome, Tsukuba-shi, Ibaraki, Japan; post office code: 305-8566).

### (6) Purification of the monoclonal antibody

The hybridoma cell line obtained in the above (5) was intraperitoneally injected to pristane-treated female nude mice (BALB/c) of 8 weeks old at the dose of 5 to 20 × 10⁶ cells/mouse. After 10 to 21 days, ascites was collected (1 to 8 ml/mouse) from the mice where ascites was produced because of conversion of the hybridoma to ascites cancer.

The said ascites was centrifuged (at 11,760 m/s² for 5 minutes) to remove solid materials. Purified IgG monoclonal antibody was prepared by purification by a caprylic acid precipitation method (Antibodies - A Laboratory Manual, Cold Spring Harbor Laboratory, 1988). Subclass of the monoclonal antibody was decided to be IgG2b by an ELISA using a subclass typing kit.

### [Reference Example 14] Detection of human VPLF using the anti-human VPLF monoclonal antibody (Western blotting)

VPLFΔN prepared in Reference Example 7 was fractionated at 100 ng/lane by an SDS-PAGE (5 to 20% gradient gel; manufactured by Atoh) (Antibodies - A Laboratory Manual, Cold Spring Harbor Laboratory, 1988) and blotted to a PVDF membrane (manufactured by Millipore).

The membrane was blocked by 1% BSA/PBS and a non-diluted culture supernatant of the anti-human VPLF monoclonal antibody KM 2676 was added to the membrane followed by being allowed to stand at room temperature for 2 hours. The membrane was well washed with Tween-PBS and a peroxidase-labeled rabbit anti-rat immunoglobulin antibody (manufactured by Dako) diluted to an extent of 1000-fold was added thereto as a second antibody followed by being allowed to stand at room temperature for 1 hour.

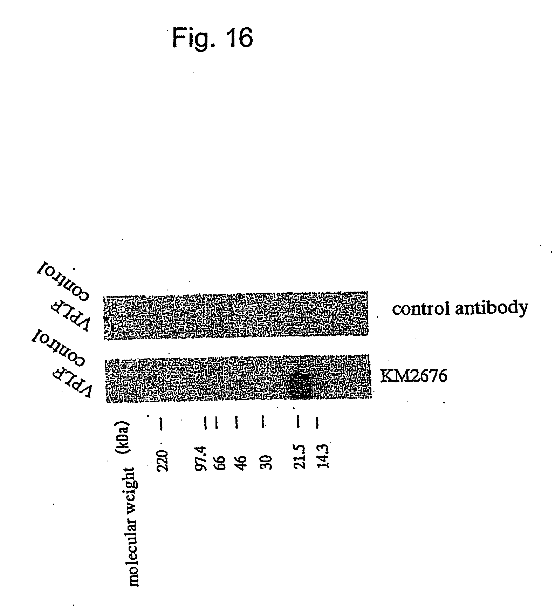

The membrane was well washed with Tween-PBS and the detection was carried out using ECL kit (manufactured by Amersham Pharmacia Biotech) (Fig. 16).

As shown in Fig. 16, the anti-human VPLF monoclonal antibody KM 2676 specifically reacted with the band near 20 kDa corresponding to the molecular weight of human VPLF (N-terminal deleted mutant).

### [Example 1] Preparation of monoclonal antibody which recognizes human VPLF and inhibits its biological activity

### (1) Preparation of immunogen

For the purpose of enhancing the immunogenicity, a conjugate of the VPLFΔN protein which was expressed in insect cells and obtained in Reference Example 7 (hereinafter, referred to as "VPLFΔN") with keyhole limpet hemocyanin (KLH; Calbiochem) was prepared by the following method and used as an immunogen. VPLFΔN (120 µg) was dissolved in 800 µl of a 0.1 mol/l CH₃COONH₄ (PH 7)-0.15 mol/l NaCl solution. KLH (30 µg) was added to the VPLF solution, then 5 µl of 1% glutaraldehyde were further added thereto and the mixture was stirred at room temperature for 5 hours. The resulting solution was dialyzed against PBS and used as an immunogen.

### (2) Immunization of animals and preparation of antigen-producing cells

The conjugate (30 µg) of VPLFΔN with KLH prepared in the above (1) was administered to each 3 female Balb/c mice of 6 weeks old together with 2 mg of an aluminum hydroxide adjuvant [Antibodies-A Laboratory Manual, Cold Spring Harbor Laboratory, p.99, 1988] and 1 × 10⁹ cells of pertussis vaccine (manufactured by Chiba Serum Institute). From two weeks after the administration, the KLH conjugate (30 µg) was administered once a week for 3 times in total. Blood was collected from vein of eyeground of the mice, its serum antibody titer was checked by the enzyme-linked immunosorbent assay as shown below and spleen was excised from the mice showing a sufficient antibody titer on the third day after the final immunization.

The spleen was finely cut in an MEM (minimum essential medium) (manufactured by Nissui Seiyaku), loosened by tweezers and centrifuged (at 2,450 m/s² for 5 minutes). Tris-ammonium chloride buffer (pH 7.6) was added to the precipitate fraction and a treatment was conducted for 1 to 2 minutes to remove the erythrocytes. The precipitate fraction (cell fraction) was washed with MEM for three times and used for a cell fusion.

### (3) Enzyme-linked immunosorbent aasay (biding ELISA)

With regard to an antigen for the assay, VPLFΔN prepared in Reference Example 2 was used. With regard to a control antigen protein, a heparin column bound protein of culture supernatant of High Five cells was used. The above antigen protein (2 µg/ml) was placed on a 96-well plate for EIA (Greiner) at the rate of 50 µl/well and allowed to stand at 4°C for one night to adsorb. The plate was washed, 100 ml/well of 1% BSA/PBS were added thereto and the mixture was allowed to stand at room temperature for 1 hour whereby the remaining active groups were blocked.

After being allowed to stand, the 1% BSA/PBS was discarded and 50 ml/well of immunized mouse antiserum, cultured medium of monoclonal antibody or purifiedmonoclonal antibody were added to the plate and allowed to stand for 2 hours. The plate was washed with Tween-PBS and 50 ml/well of the peroxidase-labeled rabbit anti-mouse immunoglobulin were added followed by being allowed to stand at room temperature for 1 hour. After the plate was washed with Tween-PBS, an ABTS substrate solution [ammonium 2,2-azinobis(3-ethylbenzothiazole-6-sulfonate), 1 mmol/l ABTS/0.1 mol/l citrate buffer (pH 4.2)] was added thereto so as to produce color and absorbance at OD 415 nm was measured using a plate reader (Emax; Molecular Devices).

### (4) Preparation of mouse myeloma cells

8-Azaguanine-resistant mouse myeloma cell line P3X63Ag8U.1 [P3-U1; purchased fromATCC] was cultured in a normal medium (RPMI 1640 medium to which 10% fetal bovine serum were added) and 2 × 10⁷ or more cells were secured for the cell fusion and used as a parent cell for the cell fusion.

### (5) Preparation of hybridoma

Mouse spleen cells obtained in the above (2) and myeloma cells obtained in the above (4) were mixed to make the ratio 10:1 followed by subjecting to centrifugation (at 2,450 m/s² for 5 minutes). Cells of the precipitate fraction was well loosened, a mixed solution of 2 g of polyethylene glycol 1000 (PEG-1000), 2 ml of MEM and 0.7 ml of dimethyl sulfoxide was added thereto at 37°C with stirring, then 1 ml of MEM was added every 1 to 2 minute(s) for several times and MEM was added so as to make the total volume 50 ml.

The suspension was centrifuged (at 900 rpm for 5 minutes), cells of the precipitate fraction was gently loosened and the cells were suspended in 100 ml of HAT medium [a medium where HAT Media Supplement (manufactured by Boehringer-Mannheim) was added to RPMI 1640 medium to which 10% of fetal bovine serum were added] by means of sucking in and out using a measuring pipette. The suspension was placed on a 96-well culture plate at the rate of 200 ml/well and cultured at 37°C for 10 to 14 days in a 5% CO₂ incubator.

After cultivation, the culture supernatant was checked by the enzyme-linked immunosorbent assay mentioned in the above (3) so as to select the wells which reacted with VPLFΔN but did not react with the control antigen protein, cloning by a limiting dilution method was repeated twice from the cells contained therein and there was established an anti-human VPLF monoclonal antibody-producing hybridoma. As a result, there were obtained hybridomas KM 2764 to 2772.

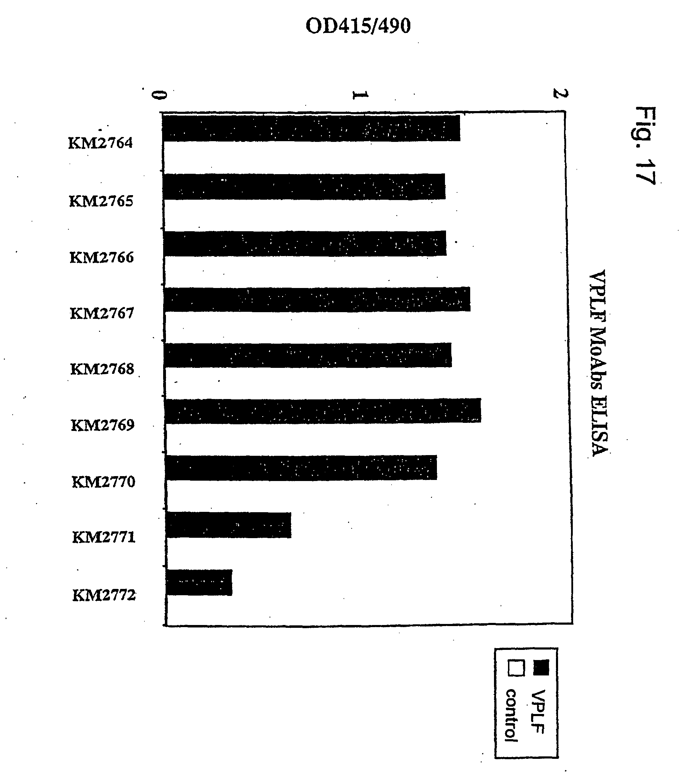

As shown in Fig. 17, they showed a specific reactivity to VPLFΔN.

Incidentally, the hybridoma cell lines KM 2764 and KM 2767 which produce anti-VPLF monoclonal antibodies KM 2764 and KM 2767 have been deposited as FERM BP-7293 and FERM BP-7294 on September 7,2000 at the International Patent Organism Depositary, National Institute of Advanced Industrial Science and Technology (Central 6, 1-1, Higashi 1-chome, Tsukuba-shi, Ibaraki, Japan.

### (6) Purification of monoclonal antibodies

The hybridoma cell lines obtained in the above (5) were intraperitoneally injected to pristane-treated female nude mice (BALB/c) of 8 weeks old at the dose of 5 to 20 × 10⁶ cells/mouse. After 10 to 21 days, ascites was collected (1 to 8 ml/mouse) from the mice where ascites was accumulated because of conversion of the hybridoma to ascites cancer.

The ascites was centrifuged (at 11,760 m/s² for 5 minutes) to remove solid materials. Purified IgG monoclonal antibody was prepared by purification according to a caprylic acid precipitation method [Antibodies - A Laboratory Manual, Cold Spring Harbor Laboratory, 1988]. Subclass of the monoclonal antibody was determined to be as shown in Table 1 by ELISA using a subclass typing kit.

**Table 1**

| Antibody Class of Anti-Human VPLF Monoclonal Antibodies | |
|---|---|
| KM Nos. | Subclass |
| KM 2764 | G1 |
| KM 2765 | G1 |
| KM 2766 | G1 |
| KM 2767 | G1 |
| KM 2768 | G1 |
| KM 2769 | G1 |
| KM 2770 | G1 |
| KM 2771 | M |
| KM 2772 | M |

### [Example 2] Investigation of reaction specificity of anti-human VPLF monoclonal antibody

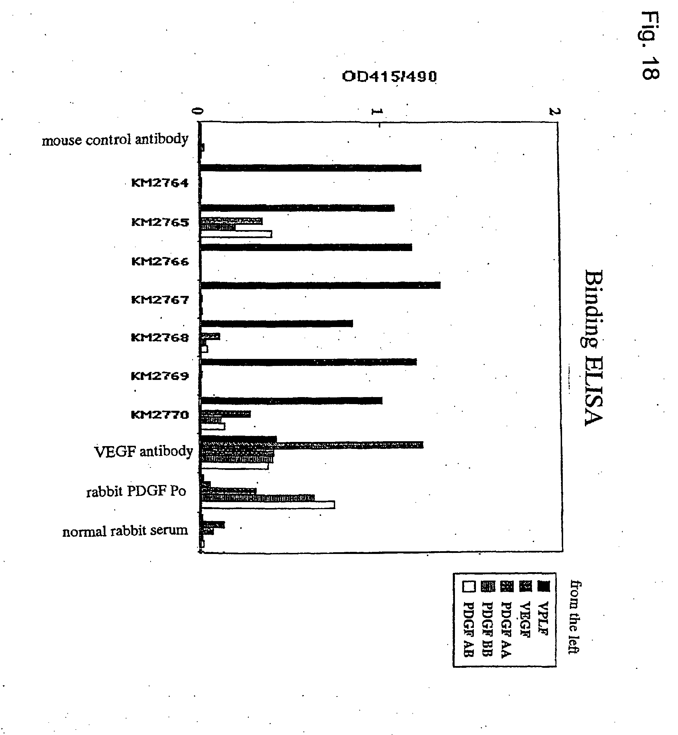

Reaction specificity of anti-VPLF monoclonal antibody was investigated by a binding ELISA as shown in Example 1(3). In addition to ΔN-VPLF used in Example 1(3), there were used VEGF expressed in insect cells, PDGF AA (manufactured by R&D), PDGF BB (manufactured by R&D) and PDGF AB (manufactured by R&D) as antigens. The result is shown in Fig. 18.

According to Fig. 18, any of KM 2764-2770 showed no cross reaction to VEGF while KM 2765, KM 2768 and KM 2770 showed a cross reaction to PDGF. KM 2764, KM 2766, KM 2767 and KM 2769 showed a specific reactivity to VPLF. Anti-VEGF monoclonal antibody reacted with VEGF and showed a cross reaction to VPLF and PDGF while rabbit anti-PDGF polyclonal antibody (manufactured by Genzyme) reacted with PDGF and slightly showed a cross reaction to VEGF.

### [Example 3] Inhibition of biological activity of human VPLF using the anti-human VPLF monoclonal antibody

It was checked whether the anti-VPLF monoclonal antibody had an inhibitory activity to the growth-promoting activity of VPLF for rat-derived smooth muscle cell RSMC mentioned in Reference Example 10.

RSMC suspended in M-199 medium (manufactured by Gibco BRL) added with 10% of fetal bovine serum (FBS), 100 units/ml of penicillin (manufactured by Gibco BRL) and 100 µg/ml of streptomycin (manufactured by Gibco BRL) was added to a 96-well collagen-coated plate (manufactured by Iwaki) to an extent of 3,000 cells/200 µl/well and cultured for one day in a CO₂ incubator of 37°C. After the cultivation, the medium was exchanged with Opti-MEM medium (manufactured by Gibco BRL) for two times and cultivation was carried out for one day longer. After removal of the medium, Opti-MEM-diluted anti-human VPLF monoclonal antibody KM 2764 to 2770 each (final concentration: 0.04 µg/ml to 1 µg/ml) or soluble PDGF receptor α or β (manufactured by R&D; final concentration: from 0.04 µg/ml to 5 µg/ml) was added to an extent of 50 µl/well and reacted at 37°C for 30 to 60 minutes. As a control antibody, KM 511 (anti-ND 28 monoclonal antibody) was similarly reacted therewith. Further, 50 µl/well of Opti-MEM-diluted VPLFΔN (final concentration: 300 ng/ml) or human PDGF BB (manufactured by R&D; final concentration: 50 ng/ml) were added to the well and cultivation was conducted for 2 days in a CO₂ incubator of 37°C. After completion of the cultivation, 10 µl of WST-1 reaction reagent (manufactured by Boehringer-Mannheim) were added to each well and incubated for 3 hours in a CO₂ incubator of 37°C. After completion of the incubation, absorbance at OD 450 nm was measured.

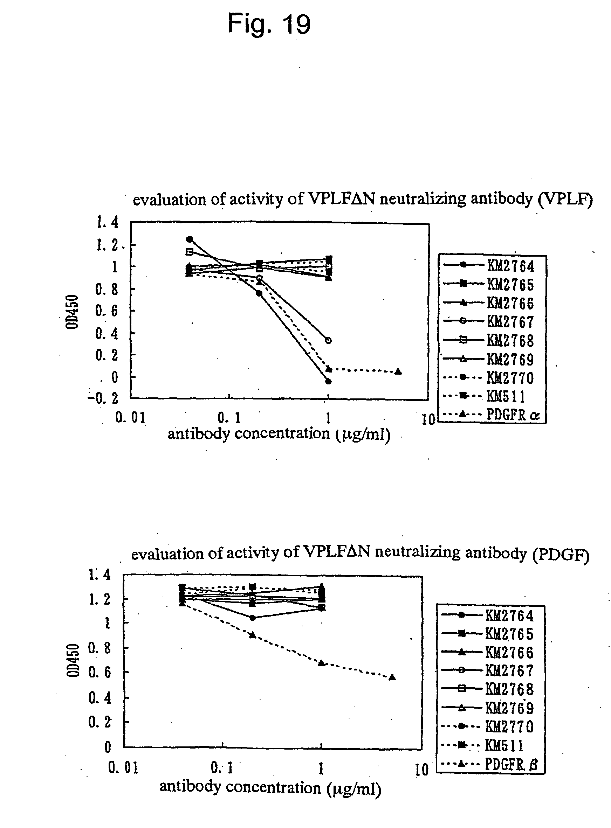

As shown in Fig. 19, the growth-promoting activity of VPLFΔN for RSMC was not inhibited at all in a control antibody while it was inhibited by KM 2764 and KM 2767 in an antibody concentration dependent manner . Further, KM 2764 and KM 2767 did not inhibit the growth-promoting activity for RSMC of PDGF BB and, therefore, those antibodies were found to have an inhibitory activity specific to VPLF.

### [Example 4] Quantitative determination of human VPLF using anti-human VPLF monoclonal antibody

Quantitative determination of human VPLF by sandwich ELISA was investigated.

Firstly, a biotin-labeled product of anti-VPLF monoclonal antibody KM 2764 was prepared. KM 2764 was dissolved in PBS at the concentration of 1 mg/ml, 1/4 volume of 0.5 mol/l carbonate buffer (pH 9.2) was added and then Sulfo-NHS-Lc-Biotin (manufactured by Pias) dissolved in dimethylformamide at the concentration of 1 mg/ml was added thereto with stirring in an amount of 1/4 volume of the antibody solution. Reaction was carried out at room temperature with stirring for 3 hours followed by dialyzing using PBS for one night to give biotin-labeled KM 2764.

The anti-human VPLF monoclonal antibody KM 2767 was placed to a 96-well plate for EIA at the concentration of 10 µg/ml in an amount of 50 µl/well and adsorbed by being allowed to stand at 4°C for one night. After washing the plate, 1% BSA/PBS was added in an amount of 100 ml/well and the mixture was allowed to stand at room temperature for 1 hour so that the remaining active groups were blocked.

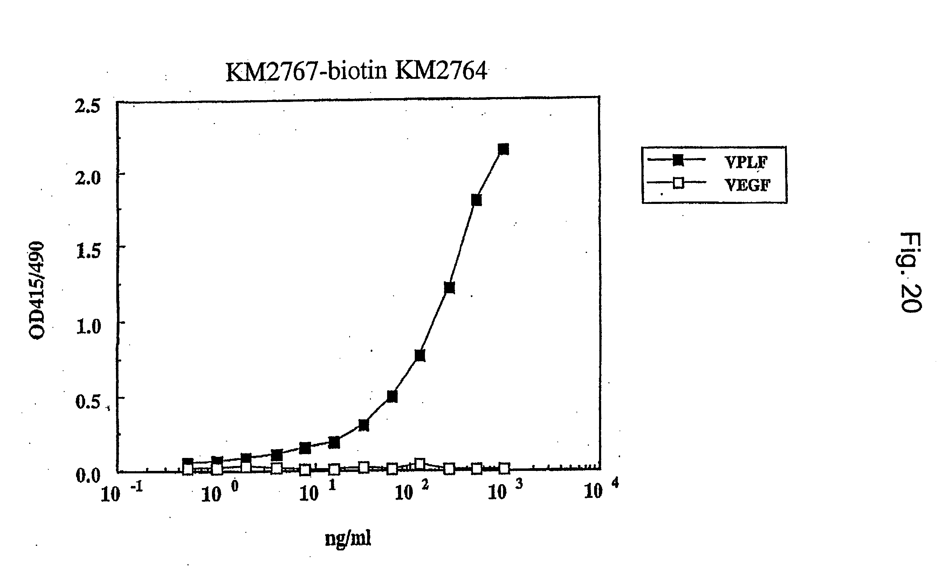

After being allowed to stand, the 1% BSA/PBS was discarded and 1% BSA/PBS-diluted VPLFΔN and insect cell expressed VEGF 165 (0.49 ng/ml to 1,000 ng/ml) were added to the plate at the concentration of 50 ml/well followed by being allowed to stand at 4°C for one night. The plate was washed with Tween-PBS and the above-prepared biotin-labeled KM 2764 was added at the concentration of 10 µg/ml to an extent of 50 ml/well followed by being allowed to stand at room temperature for 2 hours. The plate was washed with Tween-PBS and HRP-Avidin (manufactured by Vector) was added to an extent of 50 ml/well followed by being allowed to stand at room temperature for 1 hour. The plate was washed with Tween-PBS, colorized by addition of an ABTS substrate solution and absorbance at OD 415 nm was measured using a plate reader. As shown in Fig. 20, it was possible to specifically quantify the VPLF by means of a sandwich ELISA using KM 2767 and biotin-labeled KM 2764. Limit for the detection was about 10 ng/ml.

### Industrial Applicability

In accordance with the present invention, it is now possible to prepare a monoclonal antibody which specifically reacts with human VPLF and inhibits its activity and to utilize as a therapeutic agent or a diagnostic agent for the diseases in which VPLF is involved in such as diseases associated with abnormal stimulation of angiogenesis, eye diseases based on abnormal angiogenesis, arthritis based on abnormal angiogenesis, skin diseases associated with abnormal angiogenesis, diseases associated with abnormal stimulation of vascular permeability, diseases associated with abnormal differentiation and proliferation of smooth muscle cells, diseases associated with abnormal differentiation and proliferation of kidney mesangial cells, diseases associated with abnormal differentiation and proliferation of blood stem cells, diseases based on abnormality in osteoblasts, diseases based on abnormality in pancreatic β cells, ischemic diseases and diseases associated with the delay of wound healing.

### Free Text of Sequence Listing

SEQ ID NO: 3: an artificially synthesized oligo-cap linker sequence
SEQ ID NO: 4: an artificially synthesized oligo (dT) primer sequence
SEQ ID NOS: 5 to 27: an artificially synthesized primer sequence
SEQ ID NOS: 28 to 31: an artificially synthesized peptide sequence
SEQ ID NOS: 32 and 33: Artificially fused polypeptide sequences
SEQ ID NO: 34: A partial sequence contained in a commercially available plasmid pMbac (STRATAGENE)
SEQ ID NO: 35: N-terminus sequence of synthetic peptide of SEQ ID NO: 32
SEQ ID NO: 36: N-terminus sequence of synthetic peptide of SEQ ID NO: 33

## Claims

1. An antibody which specifically recognizes protein comprising an amino acid sequence represented by SEQ ID NO: 1 and inhibits a growth factor activity of the said protein.

2. The antibody according to claim 1, wherein it recognizes an epitope existing in the 227th amino acid to the 345th amino acid in SEQ ID NO: 1.

3. An antibody which specifically recognizes the protein comprising an amino acid sequence where one or more amino acid(s) is/are deleted, substituted or added in the amino acid sequence represented by SEQ ID NO: 1 and having a growth factor activity of the protein comprising the amino acid sequence represented by SEQ ID NO: 1, and which inhibits the growth factor activity of the said protein.

4. An antibody which specifically recognizes the protein comprising an amino acid sequence having homology of 60% or more to the amino acid sequence represented by SEQ ID NO: 1 and having a growth factor activity of the protein comprising the amino acid sequence represented by SEQ ID NO: 1 and, which inhibits the growth factor activity of the said protein.

5. An antibody which specifically recognizes the protein comprising a partial sequence of the amino acid sequence represented by SEQ ID NO: 1, containing eight cysteine residues conserved among the factors belonging to a VEGF/PDGF superfamily and having a growth factor activity of the protein comprising the amino acid sequence represented by SEQ ID NO: 1, and which inhibits the growth factor activity of the said protein.

6. An antibody which specifically recognizes the protein comprising an amino acid sequence where one or more amino acid(s) is/are deleted, substituted or added in a partial sequence of the amino acid sequence represented by SEQ ID NO: 1 and containing eight cysteine residues conserved among the factors belonging to a VEGF/PDGF superfamily, having a growth factor activity of the protein comprising the amino acid sequence represented by SEQ ID NO: 1, and which inhibits the growth factor activity of the said protein.

7. An antibody which specifically recognizes the protein comprising an amino acid sequence where the amino acids from N-terminal to at least the 226th amino acid are deleted in the amino acid sequence represented by SEQ ID NO: 1 and having a growth factor activity of the protein comprising the amino acid sequence represented by SEQ ID NO: 1, and which inhibits the growth factor activity of the said protein.

8. An antibody which specifically recognizes a protein comprising the amino acid sequence represented by SEQ ID NO: 32 and inhibits the growth factor activity of the said protein.

9. An antibody which specifically recognizes a protein comprising the amino acid sequence represented by SEQ ID NO: 33 and inhibits the growth factor activity of the said protein.

10. The antibody according to any one of claims 1 to 9, wherein the growth factor activity of the protein is a growth-promoting activity for smooth muscle cells.

11. The antibody according to claim 10, wherein the smooth muscle cells are derived from rat.

12. The antibody according to any one of claims 1 to 11, wherein the antibody is a monoclonal antibody.

13. The monoclonal antibody according to claim 12, wherein the monoclonal antibody is a mouse monoclonal antibody.

14. The antibody according to claim 12, wherein the monoclonal antibody is an IgG1 subclass.

15. The antibody according to claim 13, wherein the mouse monoclonal antibody is an IgG1 subclass.

16. A monoclonal antibody which is produced by hybridoma cell line KM 2764 (FERM BP-7293).

17. A monoclonal antibody which is produced by hybridoma cell line KM 2767 (FERM BP-7294).

18. An antibody fragment comprising a partial fragment of the monoclonal antibody according to claim 12.

19. An antibody derivative where an antibody according to any one of claims 1 to 17 or an antibody fragment according to claim 18 is bound to a radioactive isotope, protein or low-molecular-weight agent.

20. A hybridoma which produces the antibody according to any one of claims 1 to 17.

21. The hybridoma according to claim 20, wherein it is a hybridoma cell line KM 2764 (FERM BP-7293).

22. The hybridoma according to claim 20, wherein it is a hybridoma cell line KM 2767 (FERM BP-7294).

23. DNA encoding the antibody according to any one of claims 1 to 17, the antibody fragment according to claim 18 or the antibody derivative according to claim 19.

24. A recombinant vector containing the DNA according to claim 23.

25. A transformant which is prepared by introduction of the recombinant vector according to claim 24 into a host cell.

26. A process for producing antibody, antibody fragment or derivative, which comprises culturing the transformant in a medium, so as to produce and accumulate the antibody according to any one of claims 1 to 17, the antibody fragment according to claim 18 or the derivative according to claim 19 in a culture and recovering the said antibody, antibody fragment or derivative.

27. A pharmaceutical agent comprising the antibody according to any one of claims 1 to 17, the antibody fragment according to claim 18 or the antibody derivative according to claim 19.

28. A therapeutic agent comprising the antibody according to any one of claims 1 to 17, the antibody fragment according to claim 18 or the antibody derivative according to claim 19 for at least one disease selected from a group consisting of diseases associated with abnormal stimulation of angiogenesis, eye diseases based on abnormal angiogenesis, arthritis based on abnormal angiogenesis, skin diseases associated with abnormal angiogenesis, diseases associated with abnormal stimulation of vascular permeability, diseases associated with abnormal differentiation and proliferation of smooth muscle cells and diseases associated with abnormal differentiation and proliferation of kidney mesangial cells.

29. The therapeutic agent according to claim 28, wherein the disease associated with abnormal stimulation of angiogenesis is selected from solid tumor and tumor metastasis, the eye disease based on abnormal angiogenesis is selected from a group consisting of diabetic retinopathy, retinopathy of prematurity, age-related macular degeneration and neovascular glaucoma, the arthritis based on abnormal angiogenesis is rheumatoid arthritis, the skin disease associated with abnormal angiogenesis is psoriasis, the disease associated with abnormal stimulation of vascular permeability is selected from a group consisting of ascites cancer, cancer with pleural effusion, Crow-Fukase syndrome and ovarian hyperstimulation syndrome, the disease associated with abnormal differentiation and proliferation of smoothmuscle cells is arterosclerosis and the disease associated with abnormal differentiation and proliferation of kidney mesangial cells is glomerulonephritis.

30. A diagnostic agent comprising the antibody according to any one of claims 1 to 17, the antibody fragment according to claim 18 or the antibody derivative according to claim 19 for at least one disease selected from a group consisting of diseases associated with abnormal stimulation of angiogenesis, eye diseases based on abnormal angiogenesis, arthritis based on abnormal angiogenesis, skin diseases associated with abnormal angiogenesis, diseases associated with abnormal stimulation of vascular permeability, diseases associated with abnormal differentiation and proliferation of smooth muscle cells, diseases associated with abnormal differentiation and proliferation of kidney mesangial cells, diseases associated with abnormal differentiation and proliferation of blood stem cells, diseases based on abnormality in osteoblasts, diseases based on abnormality in pancreatic β cells, ischemic diseases and diseases associated with the delay of wound healing.

31. The diagnostic agent according to claim 30, wherein the disease associated with abnormal stimulation of angiogenesis is selected from solid tumor and tumor metastasis, the eye disease based on abnormal angiogenesis is selected from a group consisting of diabetic retinopathy, retinopathy of prematurity, age-related macular degeneration and neovascular glaucoma, the arthritis based on abnormal angiogenesis is rheumatoid arthritis, the skin disease associated with abnormal angiogenesis is psoriasis, the disease associated with abnormal stimulation of vascular permeability is selected from a group consisting of ascites cancer, cancer with pleural effusion, Crow-Fukase syndrome and ovarian hyperstimulation syndrome, the disease associated with abnormal differentiation and proliferation of smooth muscle cells is arterosclerosis, the disease associated with abnormal differentiation and proliferation of kidney mesangial cells is glomerulonephritis, the disease associated with abnormal differentiation and proliferation of blood stem cells is anemia, the disease based on abnormality in osteoblasts is osteoporosis, the disease based on abnormality in pancreatic β cells is diabetes mellitus, the ischemic disease is selected from a group consisting of cerebral infarction, acute myocardial infarction and peripheral artery occlusion and the disease associated with the delay of wound healing is selected from a group consisting of neurogenic ulcer of lower limb and diabetic ulcer of lower limb.

32. A method for an immunological quantitative determination of at least one protein selected from a group consisting of the following (a) to (j) which comprises using the antibody according to any one of claims 1 to 17, the antibody fragment according to claim 18 or the antibody derivative according to claim 19:
(a) a protein comprising the amino acid sequence represented by SEQ ID NO: 1;
(b) a protein comprising an amino acid sequence where one or more amino acid(s) is/are deleted, substituted or added in the amino acid sequence represented by SEQ ID NO: 1 and having the growth factor activity of the protein comprising the amino acid sequence represented by SEQ ID NO: 1;
(c) a protein comprising an amino acid sequence having 60% or more homology to the amino acid sequence represented by SEQ ID NO: 1 and having the growth factor activity of the protein comprising the amino acid sequence represented by SEQ ID NO: 1;
(d) a protein comprising a partial sequence of the amino acid sequence represented by SEQ ID NO: 1, containing eight cysteine residues conserved among the factors belonging to a VEGF/PDGF superfamily and having the growth factor activity of the protein comprising the amino acid sequence represented by SEQ ID NO: 1;
(e) a protein comprising a partial amino acid sequence where one or more amino acid(s) is/are deleted, substituted or added in the amino acid sequence represented by SEQ ID NO: 1, containing eight cysteine residues conserved among the factors belonging to a VEGF/PDGF superfamily and having the growth factor activity of the protein comprising the amino acid sequence represented by SEQ ID NO: 1;
(f) a protein comprising an amino acid sequencewhere amino acids of from N-terminal to at least the 226th amino acid are deleted from the amino acid sequence represented by SEQ ID NO: 1 and having the growth factor activity of the protein comprising the amino acid sequence represented by SEQ ID NO: 1;
(g) a protein comprising the amino acid sequence represented by SEQ ID NO: 32;
(h) a protein comprising the amino acid sequence represented by SEQ ID NO: 33;
(i) the protein according to any one of the above (a) to (h) where the growth factor activity of the protein is a growth-promoting activity for smooth muscle cells; and
(j) the protein according to the above (i) where the smooth muscle cells are derived from rat.

33. A method for the immunological detection of at least one protein selected from a group consisting of the following (a) to (j) which comprises using the antibody according to any one of claims 1 to 17, the antibody fragnment according to claim 18 or the antibody derivative according to claim 19:
(a) a protein comprising the amino acid sequence represented by SEQ ID NO: 1;
(b) a protein comprising an amino acid sequence where one or more amino acid(s) is/are deleted, substituted or added in the amino acid sequence represented by SEQ ID NO: 1 and having the growth factor activity of the protein comprising the amino acid sequence represented by SEQ ID NO: 1;
(c) a protein comprising an amino acid sequence having 60% or more homology to the amino acid sequence represented by SEQ ID NO: 1 and having the growth factor activity of the protein comprising the amino acid sequence represented by SEQ ID NO: 1;
(d) a protein comprising a partial sequence of the amino acid sequence represented by SEQ ID NO: 1, containing eight cysteine residues conserved among the factors belonging to a VEGF/PDGF superfamily and having the growth factor activity of the protein comprising the amino acid sequence represented by SEQ ID NO: 1;
(e) a protein comprising a partial amino acid sequence where one or more amino acid(s) is/are deleted, substituted or added in the amino acid sequence represented by SEQ ID NO: 1, containing eight cysteine residues conserved among the factors belonging to a VEGF/PDGF superfamily and having the growth factor activity of the protein comprising the amino acid sequence represented by SEQ ID NO: 1;
(f) a protein comprising an amino acid sequence where amino acids of from N-terminal to at least the 226th amino acid are deleted from the amino acid sequence represented by SEQ ID NO: 1 and having the growth factor activity of the protein comprising the amino acid sequence represented by SEQ ID NO: 1;
(g) a protein comprising the amino acid sequence represented by SEQ ID NO: 32;
(h) a protein comprising the amino acid sequence represented by SEQ ID NO: 33;
(i) the protein according to any one of the above (a) to (h) where the growth factor activity of the protein is a growth-promoting activity for smooth muscle cells; and
(j) the protein according to the above (i) where the smooth muscle cells are derived from rat.

34. A method for detecting at least one disease selected from a group consisting of diseases associated with abnormal stimulation of angiogenesis, eye diseases based on abnormal angiogenesis, arthritis based on abnormal angiogenesis, skin diseases associated with abnormal angiogenesis, diseases associated with abnormal stimulation of vascular permeability, diseases associated with abnormal differentiation and proliferation of smooth muscle cells, diseases associated with abnormal differentiation and proliferation of kidney mesangial cells, diseases associated with abnormal differentiation and proliferation of blood stem cells, diseases based on abnormality in osteoblasts, diseases based on abnormality in pancreatic β cells, ischemic diseases and diseases associated with the delay of wound healing, which comprises using the antibody according to any one of claims 1 to 17, the antibody fragment according to claim 18 or the antibody derivative according to claim 19.

35. The method according to claim 34, wherein the disease associated with abnormal stimulation of angiogenesis is selected from solid tumor and tumor metastasis, the eye disease based on abnormal angiogenesis is selected from a group consisting of diabetic retinopathy, retinopathy of prematurity, age-related macular degeneration and neovascular glaucoma, the arthritis based on abnormal angiogenesis is rheumatoid arthritis, the skin disease associated with abnormal angiogenesis is psoriasis, the disease associated with abnormal stimulation of vascular permeability is selected from a group consisting of ascites cancer, cancer with pleural effusion, Crow-Fukase syndrome and ovarian hyperstimulation syndrome, the disease associated with abnormal differentiation and proliferation of smooth muscle cells is arterosclerosis, the disease associated with abnormal differentiation and proliferation of kidney mesangial cells is glomerulonephritis, the disease associated with abnormal differentiation and proliferation of blood stem cells is anemia, the disease based on abnormality in osteoblasts is osteoporosis, the disease based on abnormality in pancreatic β cells is diabetes mellitus, the ischemic disease is selected from a group consisting of cerebral infarction, acute myocardial infarction and peripheral artery occlusion and the disease associated with the delay of wound healing is selected from a group consisting of neurogenic ulcer of lower limb and diabetic ulcer of lower limb.


SEQUENCE LISTING

<110> KYOWA HAKKO KOGYO CO., LTD.
HELIX RESEARCH INSTITUTE, INC.

<120> Antibody inhibiting VPLF activity

<130> PH-1425-PCT

<150> JP 2000-319985
<151> 2000-10-19

<160> 36

<170> PatentIn Ver. 2.0

<210> 1
<211> 345
<212> PRT
<213> Homo sapiens

<400> 1
Met Ser Leu Phe Gly Leu Leu Leu Leu Thr Ser Ala Leu Ala Gly Gln
1               5                   10                  15

Arg Gln Gly Thr Gln Ala Glu Ser Asn Leu Ser Ser Lys Phe Gln Phe
            20                  25                  30

Ser Ser Asn Lys Glu Gln Asn Gly Val Gln Asp Pro Gln His Glu Arg

        35                  40                  45

Ile Ile Thr Val Ser Thr Asn Gly Ser Ile His Ser Pro Arg Phe Pro
    50                  55                  60

His Thr Tyr Pro Arg Asn Thr Val Leu Val Trp Arg Leu Val Ala Val
65                  70                  75                  80

Glu Glu Asn Val Trp Ile Gln Leu Thr Phe Asp Glu Arg Phe Gly Leu
                85                  90                  95

Glu Asp Pro Glu Asp Asp Ile Cys Lys Tyr Asp Phe Val Glu Val Glu
            100                 105                 110

Glu Pro Ser Asp Gly Thr Ile Leu Gly Arg Trp Cys Gly Ser Gly Thr
        115                 120                 125

Val Pro Gly Lys Gln Ile Ser Lys Gly Asn Gln Ile Arg Ile Arg Phe
    130                 135                 140

Val Ser Asp Glu Tyr Phe Pro Ser Glu Pro Gly Phe Cys Ile His Tyr
145                 150                 155                 160

Asn Ile Val Met Pro Gln Phe Thr Glu Ala Val Ser Pro Ser Val Leu
                165                 170                 175

Pro Pro Ser Ala Leu Pro Leu Asp Leu Leu Asn Asn Ala Ile Thr Ala
            180                 185                 190

Phe Ser Thr Leu Glu Asp Leu Ile Arg Tyr Leu Glu Pro Glu Arg Trp
        195                 200                 205
Gln Leu Asp Leu Glu Asp Leu Tyr Arg Pro Thr Trp Gln Leu Leu Gly
    210                 215                 220
Lys Ala Phe Val Phe Gly Arg Lys Ser Arg Val Val Asp Leu Asn Leu
225                 230                 235                 240
Leu Thr Glu Glu Val Arg Leu Tyr Ser Cys Thr Pro Arg Asn Phe Ser
                245                 250                 255
Val Ser Ile Arg Glu Glu Leu Lys Arg Thr Asp Thr Ile Phe Trp Pro
            260                 265                 270
Gly Cys Leu Leu Val Lys Arg Cys Gly Gly Asn Cys Ala Cys Cys Leu
        275                 280                 285
His Asn Cys Asn Glu Cys Gln Cys Val Pro Ser Lys Val Thr Lys Lys
    290                 295                 300
Tyr His Glu Val Leu Gln Leu Arg Pro Lys Thr Gly Val Arg Gly Leu
305                 310                 315                 320
His Lys Ser Leu Thr Asp Val Ala Leu Glu His His Glu Glu Cys Asp
                325                 330                 335
Cys Val Cys Arg Gly Ser Thr Gly Gly
            340                 345

<210> 2

<211> 1328

<212> DNA

<213> Homo sapiens

<220>

<221> CDS

<222> (94)..(1128)

<400> 2

agtgcagcct tcccctggcg gtggtgaaag agactcggga gtcgctgctt ccaaagtgcc 60

cgccgtgagt gagctctcac cccagtcagc caa atg agc ctc ttc ggg ctt ctc 114
Met Ser Leu Phe Gly Leu Leu
1               5

ctg ctg aca tct gcc ctg gcc ggc cag aga cag ggg act cag gcg gaa 162
Leu Leu Thr Ser Ala Leu Ala Gly Gln Arg Gln Gly Thr Gln Ala Glu
        10                  15                  20

tcc aac ctg agt agt aaa ttc cag ttt tcc agc aac aag gaa cag aac 210
Ser Asn Leu Ser Ser Lys Phe Gln Phe Ser Ser Asn Lys Glu Gln Asn
    25                  30                  35

gga gta caa gat cct cag cat gag aga att att act gtg tct act aat 258
Gly Val Gln Asp Pro Gln His Glu Arg Ile Ile Thr Val Ser Thr Asn
40                  45                  50                  55

gga agt att cac agc cca agg ttt cct cat act tat cca aga aat acg 306
Gly Ser Ile His Ser Pro Arg Phe Pro His Thr Tyr Pro Arg Asn Thr
                60                  65                  70

gtc ttg gta tgg aga tta gta gca gta gag gaa aat gta tgg ata caa 354
Val Leu Val Trp Arg Leu Val Ala Val Glu Glu Asn Val Trp Ile Gln
            75                  80                  85

ctt acg ttt gat gaa aga ttt ggg ctt gaa gac cca gaa gat gac ata 402
Leu Thr Phe Asp Glu Arg Phe Gly Leu Glu Asp Pro Glu Asp Asp Ile
        90                  95                  100

tgc aag tat gat ttt gta gaa gtt gag gaa ccc agt gat gga act ata 450
Cys Lys Tyr Asp Phe Val Glu Val Glu Glu Pro Ser Asp Gly Thr Ile
    105                 110                 115

tta ggg cgc tgg tgt ggt tct ggt act gta cca gga aaa cag att tct 498
Leu Gly Arg Trp Cys Gly Ser Gly Thr Val Pro Gly Lys Gln Ile Ser
120                 125                 130                 135

aaa gga aat caa att agg ata aga ttt gta tct gat gaa tat ttt cct 546
Lys Gly Asn Gln Ile Arg Ile Arg Phe Val Ser Asp Glu Tyr Phe Pro
                140                 145                 150

tct gaa cca ggg ttc tgc atc cac tac aac att gtc atg cca caa ttc 594
Ser Glu Pro Gly Phe Cys Ile His Tyr Asn Ile Val Met Pro Gln Phe
            155                 160                 165

aca gaa gct gtg agt cct tca gtg cta ccc cct tca gct ttg cca ctg 642

Thr Glu Ala Val Ser Pro Ser Val Leu Pro Pro Ser Ala Leu Pro Leu
        170                 175                 180

gac ctg ctt aat aat gct ata act gcc ttt agt acc ttg gaa gac ctt 690
Asp Leu Leu Asn Asn Ala Ile Thr Ala Phe Ser Thr Leu Glu Asp Leu
    185                 190                 195

att cga tat ctt gaa cca gag aga tgg cag ttg gac tta gaa gat cta 738
Ile Arg Tyr Leu Glu Pro Glu Arg Trp Gln Leu Asp Leu Glu Asp Leu
200                 205                 210                 215

tat agg cca act tgg caa ctt ctt ggc aag gct ttt gtt ttt gga aga 786
Tyr Arg Pro Thr Trp Gln Leu Leu Gly Lys Ala Phe Val Phe Gly Arg
                220                 225                 230

aaa tcc aga gtg gtg gat ctg aac ctt cta aca gag gag gta aga tta 834
Lys Ser Arg Val Val Asp Leu Asn Leu Leu Thr Glu Glu Val Arg Leu
            235                 240                 245

tac agc tgc aca cct cgt aac ttc tca gtg tcc ata agg gaa gaa cta 882
Tyr Ser Cys Thr Pro Arg Asn Phe Ser Val Ser Ile Arg Glu Glu Leu
        250                 255                 260

aag aga acc gat acc att ttc tgg cca ggt tgt ctc ctg gtt aaa cgc 930
Lys Arg Thr Asp Thr Ile Phe Trp Pro Gly Cys Leu Leu Val Lys Arg
    265                 270                 275

tgt ggt ggg aac tgt gcc tgt tgt ctc cac aat tgc aat gaa tgt caa 978
Cys Gly Gly Asn Cys Ala Cys Cys Leu His Asn Cys Asn Glu Cys Gln

280                 285                 290                 295

tgt gtc cca agc aaa gtt act aaa aaa tac cac gag gtc ctt cag ttg     1026
Cys Val Pro Ser Lys Val Thr Lys Lys Tyr His Glu Val Leu Gln Leu
                300                 305                 310

aga cca aag acc ggt gtc agg gga ttg cac aaa tca ctc acc gac gtg     1074
Arg Pro Lys Thr Gly Val Arg Gly Leu His Lys Ser Leu Thr Asp Val
            315                 320                 325

gcc ctg gag cac cat gag gag tgt gac tgt gtg tgc aga ggg agc aca     1122
Ala Leu Glu His His Glu Glu Cys Asp Cys Val Cys Arg Gly Ser Thr
        330                 335                 340

gga gga tagccgcatc accaccagca gctcttgccc agagctgtgc agtgcagtgg     1178
Gly Gly
    345

ctgattctat tagagaacgt atgcgttatc tccatcctta atctcagttg tttgcttcaa 1238

ggacctttca tcttcaggat ttacagtgca ttctgaaaga ggagacatca aacagaatta 1298

ggagttgtgc aacagctctt ttgagaagga                                   1328

<210> 3
<211> 30
<212> RNA
<213> Artificial Sequence

<220>

<223> Description of Artificial Sequence:an artificially

synthesized oligo-cap linker sequence

<400> 3

agcaucgagu cggccuuguu ggccuacugg 30

<210> 4

<211> 42

<212> DNA

<213> Artificial Sequence

<220>

<223> Description of Artificial Sequence:an artificially

synthesized oligo(dT) primer sequence

<400> 4

gcggctgaag acggcctatg tggccttttt tttttttttt tt 42

<210> 5

<211> 21

<212> DNA

<213> Artificial Sequence

<220>

<223> Description of Artificial Sequence:an artificially

synthesized primer sequence

<400> 5

agcatcgagt cggccttgtt g                                                21

<210> 6
<211> 21
<212> DNA
<213> Artificial Sequence

<220>
<223> Description of Artificial Sequence:an artificially
      synthesized primer sequence

<400> 6
gcggctgaag acggcctatg t                                                21

<210> 7
<211> 30
<212> DNA
<213> Artificial Sequence

<220>
<223> Description of Artificial Sequence:an artificially
      synthesized primer sequence

<400> 7
tcttcgggct tctcctgctg acatctgccc                                       30

<210> 8
<211> 30
<212> DNA

<213> Artificial Sequence

<220>
<223> Description of Artificial Sequence:an artificially
      synthesized primer sequence

<400> 8
gcacacacag tcacactcct catggtgctc                                        30

<210> 9
<211> 30
<212> DNA
<213> Artificial Sequence

<220>
<223> Description of Artificial Sequence:an artificially
      synthesized primer sequence

<400> 9
tgggtgcatt ggagcctcgc cttgctgctc                                        30

<210> 10
<211> 30
<212> DNA
<213> Artificial Sequence

<220>
<223> Description of Artificial Sequence:an artificially
      synthesized primer sequence

<400> 10
ctgtaggaag ctcatctctc ctatgtgctg                                        30

<210> 11
<211> 30
<212> DNA
<213> Artificial Sequence

<220>
<223> Description of Artificial Sequence:an artificially
      synthesized primer sequence

<400> 11
cagcacatag gagagatgag cttcctacag                                        30

<210> 12
<211> 30
<212> DNA
<213> Artificial Sequence

<220>
<223> Description of Artificial Sequence:an artificially
      synthesized primer sequence

<400> 12
ccgagcagtc agctggggga gatgtccctg                                        30

<210> 13

<211> 30

<212> DNA

<213> Artificial Sequence

<220>

<223> Description of Artificial Sequence:an artificially

synthesized primer sequence

<400> 13

gccttcgagt ccggactcga cctctcggac 30

<210> 14

<211> 30

<212> DNA

<213> Artificial Sequence

<220>

<223> Description of Artificial Sequence:an artificially

synthesized primer sequence

<400> 14

cagggacatc tcccccagct gactgctcgg 30

<210> 15

<211> 30

<212> DNA

<213> Artificial Sequence

<220>

<223> Description of Artificial Sequence:an artificially
      synthesized primer sequence

<400> 15
cagggctcca gtaatgaaca tggaccagtg                                        30

<210> 16
<211> 30
<212> DNA
<213> Artificial Sequence

<220>
<223> Description of Artificial Sequence:an artificially
      synthesized primer sequence

<400> 16
ggcaacttta acaggcacta attcaggtac                                        30

<210> 17
<211> 30
<212> DNA
<213> Artificial Sequence

<220>
<223> Description of Artificial Sequence:an artificially
      synthesized primer sequence

<400> 17
ttcccttgct tcctgcagct cctggccggg                                        30

<210> 18

<211> 30

<212> DNA

<213> Artificial Sequence

<220>

<223> Description of Artificial Sequence:an artificially
      synthesized primer sequence

<400> 18
agagaacgtc agctccacgt aggagggccg                                        30

<210> 19

<211> 30

<212> DNA

<213> Artificial Sequence

<220>

<223> Description of Artificial Sequence:an artificially
      synthesized primer sequence

<400> 19
ctcgcccatg ttctggccga ggaagccgag                                        30

<210> 20

<211> 30

<212> DNA

<213> Artificial Sequence

<220>
<223> Description of Artificial Sequence:an artificially
      synthesized primer sequence

<400> 20
ggccaccttg acgctgcggt ggtggacgcg                                        30

<210> 21
<211> 30
<212> DNA
<213> Artificial Sequence

<220>
<223> Description of Artificial Sequence:an artificially
      synthesized primer sequence

<400> 21
ctctgctgct acctgcgtct ggtcagcgcc                                        30

<210> 22
<211> 30
<212> DNA
<213> Artificial Sequence

<220>
<223> Description of Artificial Sequence:an artificially
      synthesized primer sequence

<400> 22
tctcacctgg acaggtcgca gctgcacctg                                        30

<210> 23
<211> 24
<212> DNA
<213> Artificial Sequence

<220>
<223> Description of Artificial Sequence:an artificially
      synthesized primer sequence

<400> 23
gatatcgccg cgctcgtcgt cgac                                              24

<210> 24
<211> 24
<212> DNA
<213> Artificial Sequence

<220>
<223> Description of Artificial Sequence:an artificially
      synthesized primer sequence

<400> 24
caggaaggaa ggctggaaga gtgc                                              24

<210> 25
<211> 27

<212> DNA
<213> Artificial Sequence

<220>
<223> Description of Artificial Sequence:an artificially
      synthesized primer sequence

<400> 25
accagagaga tggcagttgg acttaga                                          27

<210> 26
<211> 30
<212> DNA
<213> Artificial Sequence

<220>
<223> Description of Artificial Sequence:an artificially
      synthesized primer sequence

<400> 26
gagctgctgg tggaattctt actatcctcc                                       30

<210> 27
<211> 30
<212> DNA
<213> Artificial Sequence

<220>
<223> Description of Artificial Sequence:an artificially

synthesized primer sequence

<400> 27
acttcttggc aaaatatttg tttttggaag                                        30

<210> 28
<211> 22
<212> PRT
<213> Artificial Sequence

<220>
<223> Description of Artificial Sequence:Synthetic peptide

<220>
<221> MOD RES
<222> (22)
<223> AMIDATION

<400> 28
Cys Thr Gln Ala Glu Ser Asn Leu Ser Ser Lys Phe Gln Phe Ser Ser
  1               5                  10                  15
Asn Lys Glu Gln Asn Gly
             20

<210> 29
<211> 17
<212> PRT
<213> Artificial Sequence

<220>
<223> Description of Artificial Sequence:Synthetic peptide

<220>
<221> MOD RES
<222> (17)
<223> AMIDATION

<400> 29
Cys Ser Ile Arg Glu Glu Leu Lys Arg Thr Asp Thr Ile Phe Trp Pro
1               5                   10                  15
Gly

<210> 30
<211> 17
<212> PRT
<213> Artificial Sequence

<220>
<223> Description of Artificial Sequence:Synthetic peptide

<220>
<221> MOD RES
<222> (1)
<223> ACETYLATION

<220>
<221> MOD RES
<222> (17)

<223> AMIDATION

<400> 30
Thr Phe Asp Glu Arg Phe Gly Leu Glu Asp Pro Glu Asp Asp Ile Cys
1               5                   10                  15
Lys

<210> 31
<211> 7
<212> PRT
<213> Artificial Sequence

<220>
<223> Description of Artificial Sequence:Synthetic peptide

<400> 31
Cys Arg Gly Ser Thr Gly Gly
1               5

<210> 32
<211> 123
<212> PRT
<213> Artificial Sequence

<220>
<223> Description of Artificial Sequence:Synthetic peptide

<400> 32
Asp Pro Ser Pro Phe Val Phe Gly Arg Lys Ser Arg Val Val Asp Leu

1                   5                   10                  15

Asn Leu Leu Thr Glu Glu Val Arg Leu Tyr Ser Cys Thr Pro Arg Asn

                20                  25                  30

Phe Ser Val Ser Ile Arg Glu Glu Leu Lys Arg Thr Asp Thr Ile Phe

            35                  40                  45

Trp Pro Gly Cys Leu Leu Val Lys Arg Cys Gly Gly Asn Cys Ala Cys

        50                  55                  60

Cys Leu His Asn Cys Asn Glu Cys Gln Cys Val Pro Ser Lys Val Thr

65                  70                  75                  80

Lys Lys Tyr His Glu Val Leu Gln Leu Arg Pro Lys Thr Gly Val Arg

                85                  90                  95

Gly Leu His Lys Ser Leu Thr Asp Val Ala Leu Glu His His Glu Glu

            100                 105                 110

Cys Asp Cys Val Cys Arg Gly Ser Thr Gly Gly

        115                 120

<210> 33

<211> 121

<212> PRT

<213> Artificial Sequence

<220>

<223> Description of Artificial Sequence:Synthetic peptide

<400> 33

Ser Pro Phe Val Phe Gly Arg Lys Ser Arg Val Val Asp Leu Asn Leu

1                   5                   10                  15

Leu Thr Glu Glu Val Arg Leu Tyr Ser Cys Thr Pro Arg Asn Phe Ser

                20                  25                  30

Val Ser Ile Arg Glu Glu Leu Lys Arg Thr Asp Thr Ile Phe Trp Pro
        35                  40                  45
Gly Cys Leu Leu Val Lys Arg Cys Gly Gly Asn Cys Ala Cys Cys Leu
    50                  55                  60
His Asn Cys Asn Glu Cys Gln Cys Val Pro Ser Lys Val Thr Lys Lys
65                  70                  75                  80
Tyr His Glu Val Leu Gln Leu Arg Pro Lys Thr Gly Val Arg Gly Leu
                85                  90                  95
His Lys Ser Leu Thr Asp Val Ala Leu Glu His His Glu Glu Cys Asp
            100                 105                 110
Cys Val Cys Arg Gly Ser Thr Gly Gly
        115                 120

<210> 34
<211> 4
<212> PRT
<213> Unknown

<220>
<223> Description of Unknown Sequence:A partial sequence contained in
a commercially available plasmid pMbac (STRATAGENE)

<400> 34
Asp Pro Ser Pro
1

<210> 35
<211> 6
<212> PRT

<213> Artificial Sequence

<220>
<223> Description of Artificial Sequence:N-terminus sequence of
      synthetic peptide of SEQ ID NO: 32

<400> 35
Asp Pro Ser Pro Phe Val
 1               5

<210> 36
<211> 6
<212> PRT
<213> Artificial Sequence

<220>
<223> Description of Artificial Sequence:N-terminus sequence of
      synthetic peptide of SEQ ID NO: 33

<400> 36
Ser Pro Phe Val Phe Gly
 1               5